# EUROPEAN PATENT SPECIFICATION

(11) **EP 1 966 379 B1**
(45) Date of publication and mention of the grant of the patent: **28.04.2010**
(21) Application number: 06850425.7
(22) Date of filing: 22.12.2006
(51) Int. Cl.: C12N 15/11

(54) **COMPOSITIONS AND METHODS FOR REGULATING COMPLEMENT SYSTEM**
ZUSAMMENSETZUNGEN UND VERFAHREN ZUR REGULIERUNG EINES KOMPLEMENTSYSTEMS
COMPOSITIONS ET PROCÉDÉS POUR RÉGULER LE SYSTÈME DU COMPLÉMENT

(30) Priority: 22.12.2005 US 753041 P
(43) Date of publication of application: 10.09.2008
(73) Proprietor: OPKO Ophthalmics, LLC, Miami, Florida 33137 (US)
(72) Inventor: REICH, Samuel Jotham, Miami Beach, FL 33140 (US); DEJNEKA, Nadine, Wynnewood, Pennsylvania 19096 (US)
(74) Representative: Ouzman, Beverley Nicola Claire
(86) International application number: PCT/US2006/062568
(87) International publication number: WO 2007/089375

(56) References cited:
- WO-A-03/066805
- WO-A-2004/009769
- BRANTL S: "Antisense-RNA regulation and RNA interference" BIOCHIMICA ET BIOPHYSICA ACTA . GENE STRUCTURE AND EXPRESSION, ELSEVIER, AMSTERDAM, NL, vol. 1575, no. 1-3, 3 May 2002 (2002-05-03), pages 15-25, XP004356720 ISSN: 0167-4781
- ZHENG XIUFEN ET AL: "Protection of renal ischemia injury using combination gene silencing of complement 3 and caspase 3 genes" TRANSPLANTATION (HAGERSTOWN), vol. 82, no. 12, December 2006 (2006-12), pages 1781-1786, XP002449720 ISSN: 0041-1337

## Description

### F. BACKGROUND

The complement system includes some 30 proteins that circulate in the extracellular fluid capable of launching a non-antigen specific immune response against invading bacterial and viral pathogens. Complement system proteins are circulated in an inactive form and become activated when a molecular pattern on the surface of an invading microorganism is recognized by early components of the system. The activated early components of the complement system recruit other complement proteins to the surface of the invader forming activated complexes that act as proteinases proteolytically cleaving other members of the complement system and activating them. What follows is a cascade of proteolytic activation, recruitment, and co-binding of members of the complement system resulting in the formation of the membrane attack complex (MAC), a pore in the cell wall of the invader resulting in cytolysis.

The complement system is activated by three different pathways: the classical pathway, the alternative pathway, and the lectin pathway. Each pathway culminates in the formation of the MAC but is initiated by a different stimulus.

The classical pathway is initiated when antibody (multiple IgG molecules or a single IgM molecule) binds to the surface of the pathogen. Binding allows the Fc component of the antibody to interact with the C1q subunit of factor C1 leading to the activation of subunit Clr. Clr, a proteolytic enzyme, cleaves subunit Cls and activates its protease function. Cls cleaves factor C4 to generate C4a and C4b. C4a remains biologically active at the reaction site mediating inflammation. C4b can either become an inactive by-product, bond covalently with an IgG molecule, or covalently attach to the cell surface of the antibody-bound microbe. In the presence of mg²⁺, cell surface bound C4b recruits C2 to the cell surface where it is cleaved by Cls to generate C2b and C2a. C2b diffuses from the cell surface. C2a forms a complex with C4b to form C3 convertase (C4b2a), a C3 specific protease.

C3 convertase cleaves C3 to generate C3a and C3b fragments. C3a is a potent anaphylatoxin that diffuses away from the cell surface and acts as a chemoattractant for leukocytes. Like C4b, C3b can become an inactive by-product, but alternatively, up to 10% of C3b can remain covalently bound to the plasma membrane of the pathogen. While bound to the plasma membrane C3b can serve as an opsonin or can combine with C3 convertase (C4b2a) to form C5 convertase (C4b2a3b). C5 convertase binds to and cleaves C5 generating C5a and C5b. C5a diffuses away from the cell surface and acts as a potent anaphylatoxin and chemoattractant that mediates inflammation. C5b remains bound to the cell wall of the pathogen and initiates the assembly of the MAC.

The MAC is formed from the late components of the complement system. C5b bound to the cell wall of the pathogen recruits C6 to form a C5bC6 complex that, in turn, recruits C7 to form a C5b67 complex that inserts into the cell membrane of the pathogen. C8 then binds to C5b67 forming a C5b678 complex and this complex initiates C9 polymerization resulting in the insertion of C9 into the lipid bilayer of the pathogen and the formation of the MAC. The MAC creates a pore in the cell membrane of the pathogen resulting in an influx of small molecules, ions and water into the cell ultimately leading to osmotic cell lysis.

Activation of the lectin pathway is based on carbohydrate recognition. Mannose binding lectin (MBL) and ficolins, which structurally resemble Clq, bind to specific carbohydrates on the pathogen cell surface and associate with MBL-associated serine proteases (MASPs), which resemble Clr and Cls. MASP-2, like Cls, cleaves complement components C4 and C2 initiating the formation C3 convertase. MASP-1 stimulates the alternative pathway by cleaving C3 directly. Cleavage of C3 results in the formation of C5 convertase and eventually MAC formation as described above.

The alternative pathway is activated in the absence of antibody binding by low-grade cleavage of C3 in plasma. A C3b produced in the plasma covalently binds to hydroxyl groups on cell-surface carbohydrates and proteins. C3b bound to the cell surface of the pathogen recruits Factor B to the cell surface. Factor D, which is mainly produced by adipose cells, cleaves bound Factor B to generate Ba and Bb. Ba is released into the circulation. Bb binds C3b to form the alternative pathway C3 convertase, C3bBb. Properdin stabilizes C3 convertase and allows the complex to continue to cleave C3. A proportion of the C3b produced binds C3bBb forming the alternative pathway C5 convertase, C3bBb3b. Like the other C5 convertase, alternative pathway C5 convertase cleaves C5 initiating MAC assembly.

Receptor binding mediates the activation of several complement system components. iC3b, a fragment generated by Factor 1-mediated cleavage of C3b, and C4b bind complement receptor type 1 (CR1, CD35) with high affinity. CR1 is an integral membrane protein found on erythrocytes, macrophages, monocytes, polymorphonuclear leukocytes, B cells and follicular dendritic cells that promotes phagocytosis of C3b-and C4b-coated particles and mediates the clearance of immune complexes from the circulation. Complement receptor type 2 (CR2, C3d receptor, CD21), a membrane glycoprotein found on B lymphocytes, follicular dendritic cells and epithelial cells, specifically binds the cleavage product of Factor 1 mediated C3b cleavage. Complement receptor type 3 (CR3, Macl, CD11bCD18) is found on macrophages, monocytes, polymorphonuclear leukocytes and dendritic cells and is thought to stimulate the phagocytosis of iC3b-coated microorganisms and particles by binding iC3b. Complement receptor type 4 (CR4, p150,95, CD11cCD18) binds iC3b as well as C3dg (another cleavage product of C3b) and promotes phagocytosis. Its cellular distribution is similar to that of CR3. Finally, C5a receptor (C5aR) and the C3a/4a receptor are found on endothelial cells, mast cells and phagocytes and bind C5a and C3a or C4a, respectively, to promote the anaphylatoxic and chemotactic activities of these protein fragments.

The complement system is tightly regulated to avoid erroneous activation or immune attack on host cells. The classical pathway is regulated by at least 6 different proteins. C1 esterase inhibitor (C1 INH) combines with and inactivates Clr and Cls as well as MASP-1 and MASP-2 proteases of the lectin pathway to inhibit the cleavage of C2 and C4 and the formation of C3 convertase. Most of the C1 found in blood is bound to C1 INH preventing spontaneous activation; however C1 is released from C1 INH when bound to an antigen-antibody complex activating the classical pathway. Another regulator, Factor I of the classical pathway, proteolitically cleaves C4b and C4b binding protein (C4bBP) inactivates Factor 1 using CR1 or membrane cofactor protein (MCP, CD46) as cofactors. In addition to serving as cofactors for Factor 1, C4bBP and CR1 bind to C4b and competitively inhibit the binding of C2a, which inhibits the production of classical pathway C3 convertase by promoting its dissociation. Decay accelerating factor (DAF) a transmembrane glycoprotein found on peripheral blood cells, endothelium, and some mucosal epithelial cells, promotes the dissociation of classical pathway C3 convertase and binds C4b to prevent it from binding C2.

The alternative pathway is also tightly regulated. Factor H competes with Factor B and Bb for the binding of C3b inhibiting production of alternative pathway C3 convertase. Factor B binding is favored on surfaces with high sialic acid content, and since most bacterial cells have low amounts of surface sialic acid compared to mammalian cells, Factor B binds bacterial cells readily activating the complement system. In contrast, Factor H binds polyanionic molecules like glycosaminoglycans or sulphated polysaccharides such as heparin that are located on the surface of mammalian cells. Factor H, therefore, binds mammalian cells protecting them from the complement system. Additionally, MCP and CR1 increase the affinity of surface bound C3b for Factor H and inactivates alternative pathway C3 convertase by promoting the dissociation of Bb. Factor I has a similar effect on alternative pathway C3 convertase using Factor H, CR1 and MCP as cofactors.

Complement regulation also occurs at the MAC. C5b67 complex insertion into the lipid membrane is inhibited by vitronectin (S-protein) binding in the serum, and membrane inhibitor of reactive lysis (CD 59, MRL) and homologous restriction factor (HRF) are membrane bound proteins found on erythrocytes, lymphocytes, monocytes, neutrophils, and platelets that prevent C7 and C8 from binding to C5b6 and interfere with C9 binding to C8, respectively. Finally, clusterin (SP-40/40), another serum protein, modulates MAC formation by preventing C9 assembly on C5b-8 and C5b-9 and by preventing bound C5b67 from attaching to the membrane.

Complement deficiencies also promote disease by increasing susceptibility to bacterial and yeast infections. Patients with defects in antibody production or phagocytic function, or have defective classical pathway complement proteins are at high risk for developing *Haemophilus influenzae* and *Streptococcus pneumoniae* infections. Similarly, patients with inherited deficiencies in components of the MAC are susceptible to neisserial disease, especially *Neisseria meningitides,* and reduced levels of MBL have been linked to recurrent pyogenic (pus forming) infections and a failure to thrive in young children. Interestingly, adults with a similar opsonin deficiency are healthy suggesting that the MBL pathway plays a critical role during the period when passively acquired maternal antibodies are lost and the mature immune system is being developed.

Many microorganisms have taken advantage of the complement system to avoid host mediated immune response and promote their virulence. Several pathogens bind C4bBP and/or Factor H. Epstein-Barr virus envelope glycoprotein gp350/220 binds CR2, and human immunodeficiency virus (HIV) and pathogenic mycobacteria bind C3b and use C3 receptors to gain entry into the cell. Other bacteria express proteins that inhibit activation of complement, and still others have developed thick capsules that form physical barriers against MAC formation.

Viruses avoid complement by incorporating complement regulatory proteins into their envelope (*i.e*., HIV), producing proteins that structurally mimic complement regulator proteins, or produce proteins that do not possess structural homology to complement recognition proteins but possess similar functional properties. Vaccinia virus is of specific interest because it secretes vaccinia complement control protein (VCP), a complement inhibitor whose amino acid sequence resembles host C4bBP (38%), MCP (35%) and DAF (31%). While, VCP is most structurally similar to C4bBP, its functional profile is most similar to CR 1. VCP blocks complement activation at several stages of the pathway by binding to C4b or C3b. It is also able to bind heparin which may confer the ability of VCP to bind to endothelial cells and block the attachment of small chemo-attractant cytokines which regulate the localization and migration of leukocytes into the tissues. Based on these properties, VCP has a strong potential as a therapeutic agent for diseases involving aberrant complement activation. VCP blocks complement activation by β-amyloid protein making it a a potentially useful treatment of Alzheimer's disease and representing a promising treatment for hyperacute rejection following xenotransplantation. Studies have shown that VCP can prolong survival of xenotransplanted organs *in vivo* by blocking complement activation. VCP may also be useful for the treatment of multiple organ dysfunction as well as brain and spinal cord injury.

Deficiencies in any single protein component can lead to abnormal complement activation, resulting in limited MAC formation and a lack of complement-mediated response. Additionally, aberrant activation can result in human disease, and members of the complement cascade have been implicated in the development of a diverse group of diseases for example, systemic lupus erythematosus (SLE), sepsis, immune complex disease, inflammation, pulmonary and hepatic fibrosis, asthma, atherosclerosis, diabetes and Alzheimer's disease. Abnormal stimuli, such as the presence of persistent microoraganisms or autoimmune humoral responses to self antigens, can trigger the abnormal complement system activation. Thus, a major goal of research has been to identify effective agents to target the specific complement components and inactivate the pathway, and the design of novel therapeutics for complement-mediated disease.

The enzymatic cleavage of C5 initiates MAC assembly. A small molecule C5aR antagonist (AcF-OPdChaWR) has produced favorable effects in multiple *in vivo* models including significant anti-inflammatory activity in a rodent model of sepsis and prevention of progressive impairment of renal function and reduced infiltration of neutrophils and macrophages in a murine lupus nephritis model. Furthermore, AcF-OPdChaWR significantly reduced neutrophil extravasation in a traumatic brain injury model, and intravenous administration of small molecule C5aR inhibitors significantly reduced liver collagen levels and fibrosis in a murine hepatic fibrogenesis model.

Another C5 inhibitor, the sodium salt of K-76 monocarboxylic acid (K-76COONa) prevents C5b generation and facilitates its decay. Oral administration of this compound to diabetic rats resulted in a reduction in proteinuria and mesangial expansion in glomeruli.

C5 has also been targeted using antibodies. Pexelizumab, a monoclonal antibody, significantly reduced mortality following acute myocardial infarction (MI) when administered as adjunctive therapy with primary percutaneous coronary intervention and reduced mortality or MI in patients after coronary artery bypass graft surgery, in the presence or absence of valve surgery. Pexelizumab has completed a Phase III clinical trial (PRIMO-CABG). While the primary endpoint of the study was not achieved, there was an overall decrease in post-operative patient mortality and morbidity. Eculizumab, another monoclonal antibody, has been tested in patients with rheumatoid arthritis, idiopathic membranous nephropathy, SLE, dermatomyositis and paroxysmal nocturnal hemoglobinuria, and evidence of clinical improvement in these conditions has been observed.

C3 inhibition has also been the focus of research. *N*²-[2,2-diphenylethoxy)acetyl]-L-arginine (SB 290157), a low molecular weight nonpeptide antagonist of C3aR, decreased paw edema in a rodent arthritis model and inhibited neutrophil recruitment in a guinea pig airway neutrophilia model. Compstatin, a cyclic 13 residue peptide, which is species-specific for primate C3, binds C3 and inhibits complement activation in serum. Furthermore, the peptide has been shown to extend the life of a porcine-to-human xenograft perfused with human blood.

Researchers have explored the possibility of targeting Factors B and D with monoclonal antibodies to specifically inhibit the alternative pathway, mAb1379 is directed against an epitope of mouse factor B and inhibits the alternative pathway in serum of mice, rats, humans, monkeys, pigs and horses. In a murine anti-phospholipid syndrome model, an autoimmune disease characterized by recurrent fetal loss, vascular thrombosis, and thrombocytopenia in the presence of anti-phospholipid antibodies, intraperitoneal administration of the antibody provided protection from complement activation and fetal loss, suggesting a therapeutic potential for the inhibition of Factor B. 166-32 is a monoclonal antibody directed against human Factor D that successfully inhibited the activation of complement, leukocytes and platelets in a cardiopulmonary bypass model. Alternative antibodies directed against Factor D have also been effective *in vitro* and *in vivo.*

The use of native and modified complement regulatory proteins is also being explored as potential therapeutics. C1 INH has been used clinically for more than 25 years for the treatment of hereditary angioedema, an autosomal dominant condition that is caused by C I INH deficiency, and has shown promising results in a number of other disease models including sepsis, brain and myocardial ischemia-rcperfusion injury, hyperacute transplant rejection, traumatic shock and the vascular leak syndromes associated with thermal injury. IL-2 therapy, and cardiopulmonary bypass. The cardio-protective effects of CI INH have been demonstrated in human clinical trials for myocardial ischemia-reperfusion injury; however, they are dose dependent, and excess C I INH has proven fatal to newborns during cardiopulmonary bypass.

TP10 is a soluble form of CR1 (sCR1) that has been tested in a clinical trial for cardiopulmonary bypass. While early results showed TP10 was well tolerated and beneficial, Phase II results failed to meet their primary endpoint. Nevertheless, further subgroup analysis of data from male patients in a high-risk population undergoing open-heart procedures revealed a significant decrease in infarct size and mortality, thus additional trials are planned. TP20 an alternative glycosylated form of TP10 has also been evaluated *in vivo* and in *vitro.*

APT070 (Mirococcpt) is a drug derived from a bacterially expressed region of CR1 combined with a membrane-targeting peptide that has been efficacious in animal models of vascular shock, rheumatoid arthritis, renal transplantation and Guillain-Barre syndrome. It has been administered systemically to healthy patients and locally to patients with rheumatoid arthritis and undergoing renal transplantation. A membrane-targeting strategy similar to that of APT070 has also been applied to CD59 and DAF.

The ability to target a complement inhibitor to a site of injury is particularly appealing as this should enhance efficacy and avoid systemic inhibition of complement. One possible strategy for targeting inhibitors was effectively demonstrated in a puromycin-induced nephrosis rat model with the use of a single chain antibody fragment. An antibody fragment specific for rat glomerular and proximal tubular epithelial cells was linked to Crry. a C3 convertase regulator found in rodents, and CD59. Intraperitoneal injection of either modified inhibitor resulted in protection against tubulointerstitial injury and renal dysfunction.

Studies have demonstrated that the presence of macular drusen is a strong risk factor for the development of both atrophic and neovascular AMD. Drusen causes a lateral stretching of the RPE monolayer and physical displacement of the RPE from its immediate vascular supply, the choriocapillaris. This displacement creates a physical barrier that may impede normal metabolite and waste diffusion between the choriocapillaris and the retina. It is likely that wastes may be concentrated near the RPE and that the diffusion of oxygen, glucose, and other nutritive or regulatory serum-associated molecules required to maintain the health of the retina and RPE are inhibited. It has also been suggested that drusen perturb photoreceptor cell function by placing pressure on rods and cones and/or by distorting photoreceptor cell alignment.

Recent studies have linked complement proteins to drusen in the eye. The presence of complement factors in drusen provides evidence that complement may play a role in AMD. Therefore, the ability to regulate complement activity may be beneficial in treating AMD.

### G. SUMMARY OF INVENTION

Embodiments of the invention presented herein are generally directed to isolated short interfering RNA (siRNA) having a sense RNA strand substantially identical to a complement pathway protein mRNA or complement system regulatory protein mRNA. The siRNA includes a sense RNA strand having a nucleotide sequence substantially identical to a target sequence of from about 19 to about 25 contiguous nucleotides of human C3 protein mRNA and an antisense RNA strand having a sequence substantially complementary to the sense RNA strand wherein the sense RNA strand and the antisense RNA strand form an RNA duplex. The sense RNA strand has a nucleotide sequence which comprises the sequence of one of SEQ ID Nos. 43, 45, 48, 49, 51, 53 or 56.

In some embodiments, the sense RNA strand may be one RNA molecule, and the antisense RNA strand may be one RNA molecule, and in other embodiments, the sense and antisense RNA strands of the siRNA may be covalently linked. In certain embodiments, the sense and antisense RNA strands may be stabilized against nuclease degradation, and the siRNA may further include non-nucleotide material.

The sense RNA strand may include a first 3' overhang and/or the antisense RNA strand may include a second 3' overhang in some embodiments, and in certain embodiments, the first and/or the second 3' overhang comprises from 1 to about 6 nucleotides. In particular embodiments, the first and/or the second 3' overhangs may be about 2 nucleotides, and in preferred embodiments, the first and/or the second 3' overhangs may be a dinucleotide of dithymidine (TT) or diuridine (UU).

Other embodiments of the invention include a pharmaceutical composition including an effective amount of an isolated siRNA according to the invention and a pharmaceutically acceptable carrier.

In some embodiments, the pharmaceutical composition may include a delivery reagent, and the delivery reagent may be lipofectin, lipofectamine, cellfectin, polycations, or liposomes, and combinations thereof. In particular embodiments, the delivery agent may be a liposome. The liposome of some embodiments may further include a targeting ligand that targets the liposome to cells at or near the site of a drusen, and in certain embodiments, the ligand may be an antibody. The liposome of other embodiments may be modified with an opsonization-inhibition moiety, and in particular embodiments, the opsonization-inhibiting moiety may include PEG, PPG, or derivatives thereof.

The sense RNA strand has a sequence corresponding to a sequence selected from SEQ ID NO: 43, SEQ ID NO: 45, SEQ ID NO: 48, SEQ ID NO: 49, SEQ ID NO: 51, SEQ ID NO: 53, or SEQ ID NO: 56, and in embodiments, the sense RNA strand may have a first 3' overhang and/or the antisense RNA strand may have a second 3' overhang. In some embodiments, the first and/or the second 3' overhang may include from 1 to about 6 nucleotides, and in others, the first and/or the second 3' overhang may be about 2 nucleotides. In particular embodiments, the first and/or the second 3' overhangs may be a dinucleotide of dithymidine (TT) or diuridine (UU).

The siRNA of the invention can be used for inhibiting expression of human complement system or complement system regulator mRNA, an effective amount of an isolated siRNA according to the invention being for administration to a subject in need thereof. Human C3 protein RNA is degraded wherein degrading the human C3 protein mRNA inhibits expression of human C3 protein mRNA and inhibits initiation and/or proliferation of a complement system response.

In embodiments, the subject in need thereof may be a human being, and in certain embodiments, the effective amount of the siRNA may be from about 1 nM to about 100 nM.

In some embodiments, the siRNA may be expressed from a recombinant plasmid, and in others, the siRNA is expressed from a recombinant virus which may be an adenoviral vector, an adeno-associated viral vector, a lentiviral vector, a retroviral vector, or a herpes virus vector. In particular embodiments, the recombinant viral vector comprises an adeno-associated viral vector. In still other embodiments, the recombinant virus may express surface proteins from other viruses such as vesicular stomatitis virus, rabies virus, Ebola virus, or Mokola virus.

The isolated siRNA of embodiments may be administered enterally by methods including oral, rectal, and intranasal administration. In other embodiments, the siRNA may be administered parenterally, by methods including intravascular administration, peri- and intra-tissue injection, subcutaneous injection or deposition, or subcutaneous infusion administration, and direct administration at or near a site of drusen formation. In particular embodiments, intravascular administration may include intravenous bolus injection, intravenous infusion, intra-arterial bolus injection, intra-arterial infusion and catheter instillation into a vasculature, and in other embodiments, peri- and intra-tissue injection may include intra-retinal injection, subretinal injection and intra-vitreal. In certain embodiments, the siRNA may be administered at or near the site of drusen formation by a method including administration by catheter, retinal pellet, suppository, an implant comprising a porous material, an implant comprising a non-porous material, or an implant comprising a gelatinous material, and in particular embodiments, the siRNA may be administered by eye drops or intraorbital injection.

Further embodiments of the invention include the siRNA of the invention for treating a disease in a subject.
Human C3 protein RNA is degraded wherein degrading the human C3 protein mRNA inhibits expression of human C3 protein mRNA and inhibits initiation and/or proliferation of a complement system response.

In various embodiments, the disease may be selected from macular degeneration-related disorder, age-related macular disorder, North Carolina macular dystrophy, Sorsby's fundus dystrophy, Stargardt's disease, pattern dystrophy, Best disease, dominant drusen, diabetic retinopathy and malattia leventinese, and macular degeneration-related disorder may include retinal detachment, chorioretinal degenerations, retinal degenerations, photoreceptor degenerations, RPE degenerations, mucopolysaccharidoses, rodcone dystrophies, cone-rod dystrophies, and cone degenerations. In particular embodiments, the disease may be age-related macular degeneration or diabetic retinopathy.

In certain embodiments, the siRNA of the invention can be used to detect the expression level of a human complement system mRNA or complement system regulatory mRNA with urine, blood plasma, serum, whole blood, or eye fluid from the subject.

The siRNA of the invention can further be used for treating an ocular disorder associated with drusen including ocularly administering to at least one eye of a subject an effective amount of the isolated siRNA. Human C3 protein RNA is degraded wherein degrading the human C3 protein mRNA inhibits expression of human C3 protein mRNA and inhibits initiation and/or proliferation of a complement system response

In various embodiments, the ocular disorder may be macular degeneration-related disorder, age-related macular disorder, North Carolina macular dystrophy, Sorsby's fundus dystrophy, Stargardt's disease, pattern dystrophy, Best disease, dominant drusen, diabetic retinopathy and malattia leventinese, and in particular embodiments, the disease may be age-related macular degeneration or diabetic retinopathy.

The ocular administration of embodiments may be topical, intra-retinal injection, subretinal injection, intravitreal injection, and intraorbital injection, and in some embodiments, the ocular administration is selected from eye drops or intraorbital injection.

The sense RNA strand has a sequence corresponding to a sequence selected from SEQ ID NO: 43, SEQ ID NO: 45, SEQ ID NO: 48, SEQ ID NO: 49, SEQ ID NO: 51, SEQ ID NO: 53, and SEQ ID NO: 56, and in particular embodiments, the siRNA may have a first 3' overhang and/or a second 3' overhang selected from dithymidine (TT) or diuridine (UU).

### H. BRIEF DESCRIPTION OF DRAWINGS

For a fuller understanding of the nature and advantages of the present invention, reference should be had to the following detailed description taken in connection with the accompanying drawings, in which:

Figure 1 illustrates human complement C3 expression in human cells treated with cytokines.

Figure 2 illustrates a complement C3 siRNA screen in ARPE19 cells.

Figure 3 illustrates the dose response of complement C3 siRNAs in ARPE19 cells.

Figure 4 illustrates the cytotoxicity of C3 siRNAs in ARPE19 cells.

### 1. DETAILED DESCRIPTION

Before the present compositions and methods are described, it is to be understood that this invention is not limited to the particular processes, compositions, or methodologies described, as these may vary. It is also to be understood that the terminology used in the description is for the purpose of describing the particular versions or embodiments only, and is not intended to limit the scope of the present invention which will be limited only by the appended claims. Unless defined otherwise, all technical and scientific terms used herein have the same meanings as commonly understood by one of ordinary skill in the art Although any methods and materials similar or equivalent to those described herein can be used in the practice or testing of embodiments of the present invention, the preferred methods, devices, and materials are now described. All publications mentioned herein are incorporated by reference in their entirety. Nothing herein is to be construed as an admission that the invention is not entitled to antedate such disclosure by virtue of prior invention.

It must also be noted that as used herein and in the appended claims, the singular forms "a", "an", and "the include plural reference sunless the context clearly dictates otherwise. Thus, for example, reference to an "eye" is a reference to one or more eyes and equivalents thereof known to those skilled in the art, and so forth.

As used herein, the term "about" means plus or minus 10% of the numerical value of the number with which it is being used. Therefore, about 50% means in the range of 45%-55%.

As used herein, a "subject" may include any human or non-human animal or mammal. Preferably, the subject is a human being.

"Administering" when used in conjunction with a therapeutic means to administer a therapeutic directly into or onto a target tissue or to administer a therapeutic to a patient whereby the therapeutic positively impacts the tissue to which it is targeted. Thus, as used herein, the term "administering", when used in conjunction with complement regulatory agent, can include, but is not limited to, providing a complement regulatory agent into or onto the target tissue; providing a complement regulatory agent systemically to a patient by, *e.g*., intravenous injection whereby the therapeutic reaches the target tissue; providing a complement regulatory agent in the form of the encoding sequence thereof to the target tissue (*e.g*., by so-called gene-therapy techniques). "Administering" a composition may be accomplished by injection, topical administration, or by either method in combination with other known techniques.

The term "improves" is used to convey that the present invention changes either the appearance, form, characteristic and/or the physical attributes of the tissue to which it is being provided, applied or administered. The change in form may be demonstrated by any of the following alone or in combination: decrease in drusen size; improved vision; decreases RPE vascularization; improvement in wet and/or dry AMD related symptoms; delayed drusen formation; and delayed onset of wet and/or dry AMD.

As used herein, the term "therapeutic" means an agent utilized to treat, combat, ameliorate, prevent or improve an unwanted condition or disease of a patient. In part, embodiments of the present invention are directed to improve the functionality of the eye as it applies to damage caused by drusen formation and AMD.

A "therapeutically effective amount" or "effective amount" of a composition is a predetermined amount calculated to achieve the desired effect, *i.e.,* to effectively inhibit, prevent or decrease drusen formation, treat wet and/or dry AMD or its symptoms. A therapeutically effective amount of a complement regulatory agent of this invention is typically an amount such that when it is administered in a physiologically tolerable excipient composition, it is sufficient to achieve an effective local concentration in the tissue. Effective amounts of compounds of the present invention can be measured by improvements in sight, decrease in size and number of drusen, or delayed drusen formation and delayed onset of AMD or symptoms of AMD.

Generally speaking, the term "tissue" refers to any aggregation of similarly specialized cells which are united in the performance of a particular function. As used herein, "tissue", unless otherwise indicated, refers to tissue which include, but are not limited to, the macula of the eye. For example, the macula comprises the choroid tissue, Bruch's membrane, retinal pigment epithelium (RPE), and photoreceptor cells (rod and cone cells).

As used herein, the term "agonist" is an agent that enhances or up regulates (e.g., potentiates or supplements) the production or activity of a gene product. An agonist can also be a compound which increases the interaction of a gene product, molecule or cell with another gene product, molecule or cell, for example, the agent may substitute a gene product with another homologous or heterologous gene product, or the agent may substitute a gene product with its receptor. In certain embodiments, an agonist may be a compound which enhances or increases binding or activation of a transcription factor to an upstream region of a gene thereby activating the gene. Any agent that activates gene expression, for example, by increasing RNA or protein synthesis or decreasing RNA or protein turnover, or activating a gene products activity may be an agonist whether the agent acts directly on the gene or gene product or acts indirectly, for example, by acting upstream in the gene regulation pathway. Examples of agonists of embodiments may be RNAs, peptides, antibodies and small molecules, or a combination thereof.

The term "antagonist", as used here, is an agent that down regulates, suppresses, or inhibits the production or activity of a gene or gene product. For example, an antagonist can be an agent that inhibits or decreases the interaction between a gene product, molecule or cell and another gene product, molecule or cell. In certain embodiments, an antagonist may be a compound that inhibits or decreases binding or activation of a transcription factor to an upstream region of a gene thereby blocking activation of the gene. Any agent that inhibits gene expression or gene product activity may be an antagonist whether the agent acts directly on the gene or gene product or acts indirectly, for example, by acting upstream in the gene regulation pathway. An antagonist can also be a compound that down regulates expression of a gene or which reduces the amount of gene product present, for example, by decreasing RNA or protein synthesis or increasing RNA or protein turnover. In various embodiments, an antagonist may be RNAs, peptides, antibodies, and small molecules, or a combination thereof.

The term "antibody" or "immunoglobulin" is used to include intact antibodies and binding fragments thereof. Typically, fragments compete with the intact antibody from which they were derived for specific binding to an antigen fragment including separate heavy chains, light chains Fab, Fab'. F(ab')2, Fabc, and Fv. Antibody fragments may be produced by any method known in the art, for example, by recombinant DNA techniques, or by enzymatic or chemical separation of intact immunoglobulins. The term "antibody" also includes one or more immunoglobulin chain that are chemically conjugated to, or expressed as, fusion proteins with other proteins. The term "antibody" also includes specific or bispecific antibody, and a specific or bifunctional antibody is an artificial hybrid antibody having two different heavy/tight chain pairs and two different binding sites. Bispecific antibodies can be produced by a variety of methods including, but not limited to, fusion of hybridomas or linking of Fab fragments. See, e. g., Songsivilai & Lachmann, Clin. Exp. Immunol. 79: 315321 (1990); Kostelny et al., J. Immunol.148, 1547-1553 (1992).

The term "antisense molecules" include antisense or sense oligonucleotides comprising a single-stranded nucleic acid sequence (either RNA or DNA) capable of binding to target mRNA (sense) or DNA (antisense) sequences for a specific protein (e. g., a complement pathway molecule). The ability to derive an antisense or a sense oligonucleotide, based upon a cDNA sequence encoding a given protein is described in, e.g., Stein and Cohen (Cancer Res. 48: 2639, 1988) and van der Krol et al. (BioTechniques 6: 958, 1988).

As used herein, a nucleic acid sequence "substantially identical" to a target sequence contained within the target mRNA is a nucleic acid sequence which is identical to the target sequence, or which differs from being identical to the target sequence by one or more nucleotides. Sense strands of the invention which comprise nucleic acid sequences substantially identical to a target sequence are characterized in that siRNA comprising such sense strands induce RNAi-mediated degradation of mRNA containing the target sequence. For example, an siRNA of the invention can comprise a sense strand comprise nucleic acid sequences which differ from a target sequence by one, two or three or more nucleotides, as long as RNAi-mediated degradation of the target mRNA is induced by the siRNA.

The complement system is constantly active at low levels in the normal eye and is closely regulated by intraocular complement regulatory proteins. Vitreous and aqueous humor from normal human eyes contain soluble forms of MCP, DAF and CD59. MCP is thought to interact with β1 integrin and may be involved in the adhesion of the retinal pigment epithelium (RPE) to its basement membrane and Bruch's membrane. Vitronectin is produced by photoreceptors, and the mean retinal RNA is nearly 50% of that measured in liver. C3 and C5 message has been identified in RPE, and C3, C5, and C9 transcripts have been detected in neural retina and/or choroid. Levels of C5 mRNA in cultured RPE cells is nearly equivalent to that found in fiver-derived cells, and almost 60% of that of human liver, suggesting that the RPE is probably the main source of C5.

Complement components and regulators have also been localized to drusen. Drusen are focal deposits of extracellular material that are localized between the basal lamina of the RPE and the inner collagenous layer of Bruch's membrane. Their presence in the eye is a significant risk factor and indicator for both dry and wet forms of age-retated macular degeneration (AMD). Drusen are composed of lipid, carbohydrate, protein and cellular components that are most likely of RPE origin. Immunohistochemical analysis of drusen revealed the presence of vitronectin, clusterin, IgG, MAC, C5, MCP and CR1. In addition, robust labeling was observed with antibodies raised against C3/C3d and the C3 activation fragments (C3b, iC3b, C3d).

Similarly, tissue surrounding Drusen also exhibited immunoreactivity for complement factors. C5, IgG, vitronectin, clusterin, components of MAC and MCP have been localized to RPE cells that are in close proximity of drusen. Furthermore, intense choroidal immunostaining was observed with antibodies against C3; however, little labeling was found in the rest of the choroid. Additionally, unknown structures that are generally deposited at sites of complement activation also showed strong immunoreactivity for proteolytic fragments of C3. It has been suggested that these structures are residual debris from degenerating RPE cells that have undergone cytolysis.

This hypothesis is further supported by animal studies. Mice deficient in monocyte chemoattractant protein-1 (Ccl-2, MCP-1) or its cognate C-C chemokine receptor-2 (Ccr-2) develop lipofuscin in the RPE, drusen beneath the RPE, photoreceptor atrophy and choroidal neovascularization (CNV). In addition, complement and IgG deposits are found in the RPE and choroid, suggesting immunological components are responsible for the AMD phenotype.

The role of complement was examined in a mouse model of laser-induced CNV. Laser-photocoagulation in C57BL6 mice resulted in C3 and MAC deposition at the site of drusen formation. In contrast, laser photocoagulation did not induce CNV in C3 deficient mice or produce MAC deposition. Systemic administration, of anti-C6 polyclonal antibodies significantly reduced the development ofCNV and deposition of MAC at the site of the laser spots. Furthermore, when C57BL6 mice were systemically depleted of complement with cobra venom factor (CVF) and treated with laser, no C3 or MAC immunoreactivity was observed in the laser spots and the development of CNV was significantly reduced. CVF is a structural analog of C3 that functionally resembles C3b and binds Factor B to form CVFBb, a C3 and C5 convertase. CVF is resistant to inactivation by Factors H and L Consequently, C3 and C5 are continuously hydrolyzed until the complement system is completely depleted. These findings suggest that activation of complement is necessary for the development of laser-induced CNV in mice and may also be necessary for the development of CPSV in AMD.

Indeed, it has been confirmed that a mutation (Y402H) in Factor H is significantly associated with the development of AMD. This mutation is located in the heparin and C-reactive protein binding sites of Factor H, and the association is prominent in cases of both early AMD and CNV. This mutation may alter the behavior of the protein so that it can no longer regulate the complement pathway. For example, a change in its binding capabilities may prevent it from properly interacting with microbes or complement fragment C3b, and this dysfunction may eventually result in local tissue damage. In addition to the Y402H mutation, at least 7 other Factor H variants have been linked with AMD.

Staining of tissue from patients with AMD revealed intense and specific immunoreactivity for Factor H in drusen, in the subRPE space and around the choroidal capillaries and was most prominent in the macula. The distribution of Factor H in macula correlated with the distribution of MAC. In certain instances sub-structural elements within the drusen also reacted with antibodies directed against C3 fragments. Locations outside the macula had less Factor H and C5b-9 immunoreactivity, and very little or no C5b-9 immunoreactivity was observed in RPE or choroid from patients under the age of 50 and without AMD. Analysis of mRNA from freshly isolated RPE and the RPE/choroid complex, but not neural retina, derived from donor eyes with and without AMD, revealed that Factor H and its truncated isoform are abundantly expressed in RPE, with levels approaching that of liver.

The recent Factor H finding provides insights into the roles of some established risk factors for AMD. For example, it has been reported that there is a strong association between *Chlamydia pneumoniae* infection and AMD. This pathogen has been identified in neovascular tissue that was surgically removed from eyes with AMD and has a high affinity for vascular tissue, where it establishes infection. In a scenario where one does not have the ability to regulate complement, such a pathogen may stimulate a chronic inflammation that ultimately leads to tissue damage.

Another risk for AMD is smoking. Cigarette smoke can activate the alternative pathway by modifying C3 *in vitro.* Thus, in the absence of functional Factor H, cigarette smoke can lead to uncontrolled complement activation. Interestingly, there is another disease caused by a deficiency in Factor H that has an AMD-like phenotype. Membranoproliferative glomerulonephritis type II (MPGNII), which can also be caused by a point mutation in Factor H, results in severe glomerulonephritis and early onset drusen formation. The drusen are structurally and compositionally indistinguishable from those found in AMD.

Aspects of the present invention include compositions for treating or preventing disease by regulating activation of the complement pathway. In some embodiments, the disease is an ocular disease, and in particular embodiments, the disease is macular degeneration related disorders including, for example, age-related macular disorder (AMD), North Carolina macular dystrophy, Sorsby's fundus dystrophy, Stargardt's disease, pattern dystrophy, Best disease, dominant drusen, diabetic retinopathy and malattia leventinese. Other diseases or disorders include retinal detachment, chorioretinal degenerations, retinal degenerations, photoreceptor degenerations, RPE degenerations, mucopolysaccharidoses, rod-cone dystrophies, cone-rod dystrophies, and cone degenerations.

In one embodiment, the siRNA of the present invention is suitable for treating or preventing the development of a macular degeneration in a subject suffering from or at risk of developing a macular degeneration-related disorder. An effective amount of the siRNA can be administered. In certain embodiments the subject may have a macular degeneration-related disorder, or the subject may be at risk of developing a macular degeneration-related disorder. In some embodiments, the subject may be free of complement-related diseases other than macular degeneration related disorders.

In one embodiment, the siRNA of the invention is a therapeutic agent which may modulate the activity of a complement pathway associated molecule by modulating the level of the complement pathway associated molecule or an up stream regulator of the complement pathway associated molecule, and in certain embodiments, the therapeutic agent may modulate the protein level of the complement pathway associated molecule. Non-limiting examples, of complement pathway associated molecule that may be modulated using methods of embodiments include anaphylatoxin C3a, anaphylatoxin C5a, C6, clusterin, haptoglobin, Ig kappa chain, Ig lambda chain, or Ig gamma chain. In other embodiments, the therapeutic agent may modulate an enzymatic activity of a complement protein or a complement pathway associated molecule. For example, the therapeutic agent may modulate the catalysis or conversion of C3 into C3a and C3b, conversion of C5 into C5a and C5b, or cleavage of Factor B into Ba and Bb.

In some embodiments, the level of the complement pathway associated molecule modulated using the therapeutic agent, is detected and the level of complement pathway associated molecule may be determined before during or after treatment with the therapeutic agent. The level of complement pathway associated molecule may be detected in any way known in the art, for example, the level may be determined from bodily fluids such as urine, blood plasma, serum, whole blood, or eye fluid from the subject.

Therapeutic agents of various embodiments may include any substance, molecule, element, compound, entity, or a combination thereof, including, but not limited to, protein, peptide, oligopeptide, small organic molecule, polysaccharide, and polynucleotide such as siRNA and the like. The agent may be a natural product, synthetic compound, or a chemical compound, or a combination of two or more substances.

In preferred embodiments, the isolated siRNA of the invention is for administration to a patient in need thereof. For example, siRNA of the invention is for administration to a patient exhibiting symptoms of diseases involving improper complement activation and may be used to inhibit complement activation. In particular, complement proteins that may be affected include, but are not limited to, classical, alternative, and lectin complement pathway proteins such as, for example, C1, C2, C3, C4, C5, C6, C7, C8, C9, Factor B, Factor D, Factor H, Factor I and proteins that modulate MAC formation on the cell surface such as vitronectin, heparin, clusterin, C4bBP, MCP and DAF are targeted. In preferred embodiments, C3, C5, and vitronectin may be targeted, preferably C3.

The invention is directed to an isolated siRNA having a sense RNA strand including a sequence substantially identical to a human C3 protein mRNA and which comprises the nucleotide sequence of one of SEQ ID Nos. 43, 45, 48, 49, 51, 53 or 56 and an antisense RNA strand substantially complementary to the sense RNA strand.

Without wishing to be bound by theory, siRNA of the invention may cause the RNAi-mediated degradation of targeted mRNAs, thereby reducing or eliminating production of the targeted complement protein. For example, siRNA-mediated degradation of C3 mRNA may inhibit MAC formation and complement system activity because C3 is necessary for the formation of C3C5 convertase and the subsequent activation of C5 which is required for initiating MAC formation. Similarly, inactivation of vitronectin may prohibit the formation of MAC on the surface on the RPE preventing or reversing drusen formation.

As used herein, "target mRNA" means the messenger RNA that a specific siRNA molecule has been designed to eliminate. The target mRNA is a human C3 protein

As used herein, a gene or mRNA which is "cognate" to human gene that is a gene or mRNA from another mammalian species which is homologous the human gene. For example, the cognate C5 human mRNA may be the C5 mRNA from a mouse.

As used herein, "isolated" means altered or removed from the natural state through human intervention. For example, an siRNA naturally present in a living animal is not "isolated," but a synthetic siRNA, or an siRNA partially or completely separated from the coexisting materials of its natural state is "isolated." An isolated siRNA can exist in substantially purified form, or can exist in a non-native environment such as, for example, a cell into which the siRNA has been delivered.

In embodiments, the invention may provide for isolated siRNA comprising short double-stranded RNA from about 19 to about 25 nucleotides in length, that are targeted to a particular mRNA. The siRNA includes a sense RNA strand and a substantially complementary antisense RNA strand annealed together by standard Watson-Crick base-pairing interactions (hereinafter "base-paired"). As described in more detail below, the sense strand includes a nucleic acid sequence that is identical or nearly identical to a sequence contained within the target mRNA.

Embodiments of the invention are, therefore, directed to an isolated siRNA having a sense RNA strand including a sequence substantially identical to 19 to 25 contiguous nucleotides of a human C3 mRNA including the sequence of at least one of SEQ ID Nos. 43, 45, 48, 49, 51, 53 or 56 and an antisense RNA strand substantially complementary to the sense RNA strand.

The sense and antisense strands of the present siRNA may consist of two separate complementary, single-stranded RNA molecules or include a single molecule in which two complementary portions are base-paired and are covalently linked by a single-stranded "hairpin" area. Without wishing to be bound by any theory, the hairpin of the latter siRNA molecule may be cleaved intracellularly by the "Dicer" protein (or its equivalent) to form an siRNA of two individual base-paired RNA molecules.

The siRNA of the invention can comprise partially purified RNA, substantially pure RNA, synthetic RNA, or recombinantly produced RNA, as well as altered RNA that differs from naturally-occurring RNA by the addition, deletion, substitution and/or alteration of one or more nucleotides. Such alterations can include addition of non-nucleotide material, such as to one or more end of the siRNA or to one or more internal nucleotides of the siRNA. Such alterations may include, but are not limited to, modifications that make the siRNA resistant to nuclease digestion.

One or both strands of the siRNA of the invention may also have a 3' overhang. As used herein, a "3' overhang" refers to at least one unpaired nucleotide, including ribonucleotides, deoxynucleotides, or combinations thereof extending from the 3'-end of an RNA strand. For example, in one embodiment, siRNA directed to complement mRNA may include at least one 3' overhang of from 1 to about 6 nucleotides or from 1 to about 5 nucleotides in length. In some embodiments, the overhang may be from 1 to about 4 nucleotides in length, and in at least one embodiment, the overhang may be from about 2 to about 4 nucleotides in length. In still other embodiments, both strands of the siRNA molecule have a 3' overhang. The length of which may be the same or different for each strand. In yet other embodiments, the 3' overhang is present on both strands of the siRNA and may be 2 nucleotides in length. For example, each strand of the siRNA of the invention can comprise 3' overhangs of dithymidine ("TT") or diuridine ("uu").

Embodiments of the invention are, therefore, directed to an isolated siRNA having a sense RNA strand including a sequence substantially identical to 19 to 25 contiguous nucleotides of a human complement system mRNA or complement system regulatory protein mRNA and an antisense RNA strand substantially complementary to the sense RNA strand wherein the sense RNA strand and/or the antisense RNA strand have 3' overhangs. In preferred embodiments, the sense RNA strand may be substantially identical to a 19 to 25 contiguous nucleotides of human C3 mRNA and the sense RNA strand and/or the antisense RNA strand having 3' overhangs. In particularly preferred embodiments, the siRNA may have a sense RNA strand substantially identical to human C3 mRNA and the sense RNA strand and/or the antisense RNA strand may have 3' overhangs of 2 nucleotides with the nucleotides each being deoxythymidine or deoxyuridine.

In order to enhance the stability of the present siRNA, the 3' overhangs can be stabilized against degradation. In one embodiment, the overhangs are stabilized by including purine nucleotides, such as adenosine or guanosine nucleotides. Alternatively, substitution of pyrimidine nucleotides by modified analogues, for example, substitution of uridine nucleotides in the 3' overhangs with 2'-deoxythymidine, is tolerated and does not affect the efficiency of RNAi degradation. In particular, the absence of a 2' hydroxyl in the 2'-deoxythymidine significantly enhances the nuclease resistance of the 3' overhang in tissue culture medium.

In certain embodiments, the siRNA of the invention may include the sequence AA(N19)TT or NA(N21), where N is any nucleotide. These siRNA may include approximately 30-70% G/C, and preferably comprise approximately 50% G/C. The sequence of the sense siRNA strand corresponds to (N19)TT or N21 (i.e., positions 3 to 23), respectively. In the latter case, the 3' end of the sense siRNA is converted to TT. The rationale for this sequence conversion is to generate a symmetric duplex with respect to the sequence composition of the sense and antisense strand 3' overhangs. The antisense RNA strand is then synthesized as the complement to positions 1 to 21 of the sense strand.

Because position 1 of the 23-nt sense strand in these embodiments may not be recognized in a sequence-specific manner by the antisense strand, the 3'-most nucleotide residue of the antisense strand can be chosen deliberately. However, the penultimate nucleotide of the antisense strand (complementary to position 2 of the 23-nt sense strand in either embodiment) may generally be complementary to the targeted sequence.

In another embodiment, the siRNA of the invention may include the sequence NAR(N17)YNN, where R is a purine (*e.g*., A or G) and Y is a pyrimidine (*e.g*., C or U/T). The respective 21-nt sense and antisense RNA strands of this embodiment, therefore, may generally begin with a purine nucleotide. Such siRNA can be expressed from pol III expression vectors without a change in targeting site, as expression of RNAs from pol III promoters is only believed to be efficient when the first transcribed nucleotide is a purine.

The siRNA of the invention can be targeted to any stretch of approximately 19-25 contiguous nucleotides in any of the target mRNA sequences (the "target sequence"). Techniques for selecting target sequences for siRNA are given, for example, in Tuschl T et al., "The siRNA User Guide," revised May 6, 2004, the entire disclosure of which is herein incorporated by reference. The siRNA User Guide" is available on the world wide web at a website maintained by Dr. Thomas Tuschl, Department of Cellular Biochemistry, AG 105, Max-Planck-Institute for Biophysical Chemistry, 37077 Gottingen, Germany, and can be found by accessing the website of the Max Planck Institute and searching with the keyword "siRNA." Thus, the sense strand of the present siRNA comprises a nucleotide sequence identical to any contiguous stretch of about 19 to about 25 nucleotides in the target mRNA.

Generally, a target sequence on the target mRNA can be selected from a given cDNA sequence corresponding to the target mRNA, preferably beginning 50 to 100 nt downstream (*i.e*., in the 3' direction) from the start codon. The target sequence can, however, be located in the 5' or 3' untranslated regions, or in the region nearby the start codon. For Example, target sequences SEQ ID NOS: 1 to 26 and 57 in Table 1, which arc within 100 nt of the 5'-end of the human C3 cDNA SEQ ID NO. 4 and correspond with target sequences SEQ ID NO. 27 to 41 and corresponding siRNA sequences SEQ ID NOS. 42 to 56.

**Table 1**

| ID NO | Source | ID NO | Source | ID NO | Source |
|---|---|---|---|---|---|
| 1 | Human C1 r | 20 | Human Vitronectin | 39 | C3 Target 13 |
| 2 | Human C1 s | 21 | Human Clusterin (Retinal) | 40 | C3 Target 14 |
| 3 | Human C2 | 22 | Human Clusterin-1 | 41 | C3 Target 15 |
| 4 | Human C3 | 23 | Human Clusterin-2 | 42 | C3 siRNA 1 |
| 5 | Human C4a | 24 | Human C4bBP (alpha) | 43 | C3 siRNA 2 |
| 6 | Human C4b | 25 | Human MCP | 44 | C3 siRNA 3 |
| 7 | Human C5 | 26 | Human DAF | 45 | C3 siRNA 4 |
| 8 | Human C6 | 27 | C3 Target 1 | 46 | C3 siRNA 5 |
| 9 | Human C7 | 28 | C3 Target 2 | 47 | C3 siRNA 6 |
| 10 | Human C8 (alpha) | 29 | C3 Target 3 | 48 | C3 siRNA 7 |
| 11 | Human C8 (beta) | 30 | C3 Target 4 | 49 | C3 siRNA 8 |
| 12 | Human C8 (gamma) | 31 | C3 Target 5 | 50 | C3 siRNA 9 |
| 13 | Human C9 | 32 | C3 Target 6 | 51 | C3 siRNA 10 |
| 14 | Human Factor B | 33 | C3 Target 7 | 52 | C3 siRNA 11 |
| 15 | Human Factor D | 34 | C3 Target 8 | 53 | C3 siRNA 12 |
| 16 | Human Factor H-2 | 35 | C3 Target 9 | 54 | C3 siRNA 13 |
| 17 | Human Factor H-4 | 36 | C3 Target 10 | 55 | C3 siRNA 14 |
| 18 | Human Factor H-5 | 37 | C3 Target 11 | 56 | C3 siRNA 15 |
| 19 | Human Factor 1 | 38 | C3 Target 12 | 57 | Human C4bBP (beta) |

Splice variants of human complement system proteins have been identified for factors C2 and MCP, and an exonic splicing enhancer has been idenitified siRNA directed to these and other as yet unidentified splice variants to complement system proteins are encompassed by the present invention.

Alternatively spliced variants of human complement system proteins may be identified as known in the art, for example, by using a technique called "RNAse protection". RNAse protection involves transcription of a gene sequence into synthetic RNA, which is hybridized to RNA derived from other cells; for example, cells from tissue at or near the site of complement activation. The hybridized RNA is then incubated with enzymes that recognize RNA:RNA hybrid mismatches. Smaller than expected fragments indicate the presence of alternatively spliced mRNAs. In embodiments, putative alternatively spliced mRNAs may be cloned and sequenced by methods well known to those skilled in the art and used to develop siRNA.

Alternatively spliced complement pathway proteins may be identified using RT-PCR. In RT-PCR, mRNA from the diseased tissue is converted into cDNA using methods well-known to those of ordinary skill in the art, for example, by using the enzyme reverse transcriptase. The entire coding sequence of the cDNA may then be amplified via PCR using a forward primer located in the 3' untranslated region, and a reverse primer located in the 5' untranslated region. The amplified products can be analyzed for alternative splice forms, for example, by comparing the size of the amplified products with the size of the expected product from normally spliced mRNA, for example, by agarose gel electrophoresis. Any change in the size of the amplified product can indicate alternative splicing. Alternatively spliced variants may then be sequenced and used to develop siRNA.

mRNA produced from mutant genes can also be identified through the techniques described above for identifying alternative splice forms. As used herein, "mutant" complement pathway proteins are produced from a gene having a sequence that differs in sequence from the complement pathway protein sequences set forth herein for example in SEQ ID NOS 1-26 and 57 at one or more base pair. Thus, allelic forms of there genes, and the mRNA produced form them, are considered "mutants" for purpose of this invention.

Databases containing nucleotide sequences related to a given disease gene can be used to identify alternatively spliced or mutant mRNAs. For example, useful databases may include GenBank, Embase, and the Cancer Genome Anatomy Project (CGAP) database. The CGAP database, for example, contains expressed sequence tags (ESTs) from various types of human cancers. An mRNA or gene sequence from including but not limited to classical, alternative, and lectin complement pathway proteins, C1, C2, C3, C4, C5, C6, C7, C8, C9, Factor B, Factor D, Factor H, Factor 1 and proteins that modulate MAC formation on the cell surface such as vitronectin, clusterin, C4bBP, MCP and DAF genes can be used to query such a database to determine whether ESTs representing alternatively spliced mRNAs have been found for a these genes.

The siRNA of the invention can be obtained using a number of techniques known to those of skill in the art, including, but not limited to, chemical synthesis or recombinantly produced using methods. For example, siRNA may be produced using the Drosophila in vitro system described in U.S. published application 2002/0086356 of Tuschl et al., the entire disclosure of which is herein incorporated by reference or siRNA may be chemically synthesized using appropriately protested ribonucleoside phosphoramidites and a conventional DNA/RNA synthesizer. In embodiments, siRNA may be synthesized as two separate, complementary RNA molecules that are combined following synthesis, or a single RNA molecule with two complementary regions may be synthesized that is capable of binding to itself to form a "hairpin" structure. Synthetic RNA molecules or synthesis reagents commercially available are also encompassed by embodiments of the invention.

Alternatively, siRNA can be expressed from recombinant circular or linear DNA plasmids using a suitable promoter. Suitable promoters for expressing siRNA of the invention from a plasmid are well known in the art and may include, for example, the U6 or H1 RNA pol III promoter sequences and the cytomegalovirus promoter. Selection of other suitable promoters is within the skill in the art. The recombinant plasmids of the invention may also include inducible or regulatable promoters for expression of the siRNA in a particular tissue or in a particular intracellular environment.

siRNA expressed from recombinant plasmids may be isolated from cultured cell expression systems using standard techniques or expressed intracellularly in the diseased tissue, for example, in or near the eye, in or near the ocular cavity, or at or near area of drusen formation *in vivo.* The use of recombinant plasmids to deliver siRNA of the invention to cells *in vivo* is discussed in more detail below. In various embodiments, a single siRNA can be expressed from a recombinant plasmid as either as two separate, complementary RNA molecules, or as a single RNA molecule with two complementary regions.

Selection of plasmids suitable for expressing siRNA of the invention, methods for inserting nucleic acid sequences for expressing the siRNA into the plasmid and methods of delivering the recombinant plasmid to the cells of interest vary in embodiments and may be selected from any plasmid known in the art or subsequently developed. For example, See Tuschl, T. (2002), Nat. Biotechnol, 20: 446-448; Brummelkamp T R et al. (2002), Science 296: 550-553; Miyagishi M et al. (2002), Nat. Biotechnol. 20: 497-500; Paddison P J et al. (2002), Genes Dev. 16: 948-958; Lee N S et al. (2002), Nat. Biotechnol. 20: 500-505; and Paul C P et al. (2002), Nat. Biotechnol. 20: 505-508, the entire disclosures of which are herein incorporated by reference.

As used herein, "in operable connection with a polyT termination sequence" means that the nucleic acid sequences encoding the sense or antisense strands are immediately adjacent to the polyT termination signal in the 5' direction. During transcription of the sense or antisense sequences from the plasmid, the polyT termination signals act to terminate transcription.

As used herein, "under the control" of a promoter means that the nucleic acid sequences encoding the sense or antisense strands are located 3' of the promoter, so that the promoter can initiate transcription of the sense or antisense coding sequences.

Recombinant viral vectors of embodiments may include sequences encoding the siRNA of the invention, any suitable promoter for expressing the siRNA sequences, and termination signals known in the art, for example, a polyT termination signal. Selection of other suitable promoters is within the skill in the art, and suitable promoters may include, for example, the U6 or H1 RNA pol III promoter sequences or the cytomegalovirus promoter. In some embodiments, recombinant viral vectors may include inducible or regulatable promoters for expression of the siRNA in a particular tissue, in a particular intracellular environment, or in response to a particular intracellular or extracellular signal. siRNA of the invention can be expressed from a recombinant viral vector either as two separate, complementary RNA molecules, or as a single RNA molecule with two complementary regions.

Selection of recombinant viral vectors suitable for use in the invention, methods for inserting nucleic acid sequences for expressing the siRNA into the vector, and methods of delivering the viral vector to the cells of interest are within the skill in the art. See, for example, Domburg R (1995), Gene Therap. 2: 301-310; Eglitis M A (1988), Biotechniques 6: 608-614; Miller A D (1990), Hum Gene Therap. 1: 5-14; and Anderson W F (1998), Nature 392: 25-30, the entire disclosures of which are herein incorporated by reference.

Any viral vector capable of accepting the coding sequences for the siRNA molecule(s) to be expressed can be used, for example vectors derived from adenovirus (AV); adeno-associated virus (AAV); retroviruses (*e.g*. lentiviruses (LV), Rhabdoviruses, murine leukemia virus); herpes virus, and the like. The tropism of the viral vectors can also be modified by pseudotyping the vectors with envelope proteins or other surface antigens from other viruses. For example, an AAV vector of the invention can be pseudotyped with surface proteins from vesicular stomatitis virus (VSV), rabies, Ebola, Mokola, and the like. In various embodiments, viral vectors arc those derived from AV and AAV. In a particular embodiment, the siRNA of the invention is expressed as two separate, complementary single-stranded RNA molecules from a recombinant AAV vector comprising, for example, either the U6 or HIRNA promoters, or the cytomegalovirus (CMV) promoter.

Methods for expressing siRNA, constructing recombinant viral vectors, and delivering viral vectors into target cells are well known in the art. For example, a suitable AV vector for expressing the siRNA, a method for constructing the recombinant AV vector, and a method for delivering the vector into target cells are described in Xia H ct al. (2002), Nat. Biotech. 20: 1006-1010 the entire disclosure of which is herein incorporated by reference, and suitable AAV vectors for expressing the siRNA of the invention, methods for constructing the recombinant AV vector, and methods for delivering the vectors into target cells are described in Samulski R et al. (1987), J. Virol. 61: 3096-3101; Fisher K J ct al. (1996). J. Virol, 70: 520-532; Samulski R et al. (1989), J. Virol. 63: 3822-3826; U.S. Pat. No. 5,252,479; U.S. Pat. No. 5,139,941; International Patent Application No. WO 94/13788; and International Patent Application No. WO 93/24641.

In some embodiments, the ability of an siRNA containing a given target sequence to cause RNAi-mediated degradation of the target mRNA may be evaluated using standard techniques for measuring the levels of RNA or protein in cells. For example, siRNA of the invention can be delivered to cultured cells, and the levels of target mRNA can be measured by Northern blot or dot blotting techniques, or by quantitative RT-PCR. Alternatively, the levels of complement pathway proteins or proteins that modulate in the cultured cells can be measured by ELISA or Western blot.

In other embodiments, RNAi-mediated degradation of target mRNA by an siRNA containing a given target sequence can also be evaluated with animal models. For example, the effectiveness of a specific siRNA for treating AMD may be evaluated using CNV mouse models, and areas of macular injury in a CNV mouse can be measured before and after administration of a siRNA. A reduction in the complement activation in these models upon administration of the siRNA indicates the down-regulation of the target mRNA or improvement in the macular injury.

As discussed above, the siRNA of the invention target and cause RNAi-mediated degradation of complement pathway mRNA or modulators of complement system activation mRNA, or alternative splice forms, mutants or cognates thereof. Degradation of the target mRNA by siRNA of the invention may reduce the production of a functional gene product from complement pathway proteins, or proteins that modulate complement system activation on the cell surface. Thus, the invention enables inhibition of expression of complement pathway proteins, or proteins that modulate complement system activation on the cell surface in a subject, wherein the siRNA of the invention is for administration in an effective amount to a subject such that the target mRNA is degraded. As the products of the complement pathway, or modulators of the complement system genes are required for initiating complement system activation, the invention also provides siRNA for inhibiting complement system activation in a subject by the RNAi-mediated degradation of the target mRNA using siRNA of the invention.

Inhibition of complement system activation can be evaluated, for example, by directly measuring the progress of AMD related drusen formation in a subject, for example, drusen can be observed by ophthalmoscopy or by observing the number of complement proteins in the drusen before and after treatment with the siRNA of the invention. An inhibition of drusen formation is indicated if the number of complement proteins in the drusen stays the same, is reduced, or is retarded. Techniques for observing and measuring the number of complement proteins in tissue samples from a subject are within the skill in the art as discussed above, for example, RNAi-mediated degradation of target mRNA can be detected by measuring levels of the target mRNA or protein in the cells of a subject using standard techniques for isolating and quantifying mRNA or protein as described above. Inhibition of complement system activation can also be inferred through observing a change or reversal in a pathogenic condition associated with uncontrolled complement system activation. For example, in AMD, a slowing, halting or reversal of vision loss indicates an inhibition of drusen formation in the choroid.

It is understood that the siRNA can degrade the target mRNA (and thus inhibit complement system activation) in substoichiometric amounts. Without wishing to be bound by any theory, it is believed that the siRNA of the invention causes degradation of the target mRNA in a catalytic manner. Thus, compared to standard anti-complement system therapies, significantly less siRNA needs to be delivered at or near the site of uncontrolled complement system activation to have a therapeutic effect.

In embodiments, isolated siRNA is administered to a subject in an effective amount. One skilled in the art can readily determine an effective amount of the siRNA of the invention to be administered to a given subject, by taking into account factors such as the size and weight of the subject; the extent of the complement system activation or disease penetration; the age, health and sex of the subject; the route of administration; and whether the administration is regional or systemic. Generally, an effective amount of the siRNA of the invention comprises an intercellular concentration at or near of the site uncontrolled complement system activation of from about 1 nanomolar (nM) to about 100 nM, from about 2 nM to about 50 nM, or from about 2.5 nM to about 10 nM. In particular embodiments, from about 0.01 mg to about 10 mg of an siRNA may be an effective amount. However, in certain embodiments, greater or lesser amounts of siRNA may be administered.

In some embodiments, SiRNAs of the invention may be used to inhibit complement system activation formation which is non-pathogenic such as complement system activation which results from normal processes in the subject. Examples of non-pathogenic complement system activation include non-antigen specific immune response against bacteria and viruses. Thus, the invention provides SiRNA for inhibiting non-pathogenic complement system activation for controlling immune response in relation to organ transplantation, xenotransplantation, and MI. In other embodiments, SiRNA of the invention may be used to inhibit complement activation associated with a disease with pathogenicity associated with inappropriate or uncontrolled complement system activation such as, for example, uncontrolled complement system activation linked to autoimmune diseases. Other uncontrolled complement system diseases include diabetic retinopathy, age-related macular degeneration (AMD), systemic lupus erythematosus (SLE), sepsis, immune complex disease, inflammation, pulmonary and hepatic fibrosis, asthma, atherosclerosis, diabetes and Alzheimer's disease. These diseases are generally characterized by the destruction of normal tissue by uncontrolled complement activation. For example, in AMD, the choroid is invaded and destroyed by drusen in the RPE inhibiting the sight of the patient, and drusen-driven destruction of the choroid in AMD eventually leads to partial or full blindness. Therfore, in certain embodiments, siRNA may be used to inhibit drusen formation in AMD.

For treating complement system related diseases, siRNA of the invention can be administered to a subject in combination with a pharmaceutical agent which is different from the present siRNA. Alternatively, the siRNA of the invention can be administered to a subject in combination with another therapeutic method designed to treat the complement system related disease. For example, the siRNA of the invention can be administered in combination with therapeutic methods currently employed for treating autoimmune diseases (*e.g*., Cl INH).

In embodiments, siRNA of the invention can be administered to the subject either as naked siRNA, in conjunction with a delivery reagent, or as a recombinant plasmid or viral vector which expresses the siRNA. Suitable delivery reagents for administration in conjunction with the present siRNA include the Mirus Transit TKO lipophilic reagent; lipofectin; lipofectamine; cellfectin; or polycations (*e.g*., polylysine), or liposomes. In particular embodiments, the delivery reagent is a liposome.

Liposomes can aid in the delivery of the siRNA to a particular tissue, such as retinal or diseased tissue and can increase the blood half-life of the siRNA. Liposomes suitable for use in the invention may be formed using standard vesicle-forming lipids, which may include neutral or negatively charged phospholipids and a sterol, such as cholesterol. The selection of lipids may generally be guided by consideration of factors such as the desired liposome size and half-life of the liposomes in the blood stream. A variety of methods are known for preparing liposomes, for example as described in Szoka et al. (1980), Ann. Rev. Biophys. Bioeng. 9: 467; and U.S. Pat. Nos. 4,235,871, 4,501,728, 4,837,028, and 5,019,369, the entire disclosures of which are herein incorporated by reference.

In some embodiments, liposomes encapsulating the siRNA of the invention may further include a ligand molecule that can target the liposome to a particular cell or tissue at or near the site of complement system activation. Ligands may include proteins that bind to receptors prevalent in tumor or vascular endothelial cells such as, for example, antibodies that bind to tumor antigens or endothelial cell surface antigens.

In other embodiments, liposomes encapsulating siRNA of the invention may be modified to avoid clearance by the mononuclear macrophage and reticuloendothelial systems. For example, in embodiments, the liposome may include opsonization-inhibition moieties bound attached to the surface of the liposome. Opsonization-inhibiting moieties for use in preparing the liposomes are typically large hydrophilic polymers that are bound to the liposome membrane. As used herein, an opsonization inhibiting moiety is "bound" to a liposome membrane when it is chemically or physically attached to the membrane, for example, by the intercalation of a lipid-soluble anchor into the membrane itself, or by binding directly to active groups of membrane lipids. These opsonization-inhibiting hydrophilic polymers form a protective surface layer which significantly decreases the uptake of the liposomes by the macrophage-monocyte system ("MMS") and reticuloendothelial system ("RES") as described in U.S. Pat. No. 4,920,016, the entire disclosure of which is herein incorporated by reference. Opsonization inhibiting moieties suitable for modifying liposomes are preferably vvater-soluble polymers with a molecular weight from about 500 to about 40,000 daltons, and more preferably from about 2,000 to about 20,000 daltons. Such polymers include polyethylene glycol (PEG) or polypropylene glycol (PPG) derivatives; *e.g*., methoxy PEG or PPG, and PEG or PPG stearate; synthetic polymers such as polyacrylamide or poly N-vinyl pyrrolidone; linear, branched, or dendrimeric polyamidoamines; polyacrylic acids; polyalcohols, e.g., polyvinylalcohol and polyxylitol to which carboxylic or amino groups are chemically linked, as well as gangliosides, such as ganglioside GM₁. Copolymers of PEG, methoxy PEG, or methoxy PPG, or derivatives thereof, are also suitable. In addition, the opsonization inhibiting polymer can be a block copolymer of PEG and either a polyamino acid, polysaccharide, polyamidoamine, polyethyleneamine, or polynucleotide. The opsonization inhibiting polymers can also be natural polysaccharides containing amino acids or carboxylic acids, e.g., galacturonic acid, glucuronic acid, mannuronic acid, hyaluronic acid, pectic acid, neuraminic acid, alginic acid, carrageenan; laminated polysaccharides or oligasaccharides (linear or branched); or carboxylated polysaccharides or oligosaccharides, *e.g*., reacted with derivatives of carbonic acids with resultant linking of carboxylic groups. In particular embodiments, the opsonization-inhibiting moiety is a PEG, PPG, or derivatives thereof. Liposomes modified with PEG or PEG-derivatives are sometimes called "PEGylated liposomes."

The opsonization inhibiting moiety can be bound to the liposome membrane by any one of numerous well-known techniques. For example, an N-hydroxysuccinimide ester of PEG can be bound to a phosphatidyl-ethanolamine lipid-soluble anchor, and then bound to a membrane. Similarly, a dextran polymer can be derivatized with a stearylamine lipid-soluble anchor via reductive amination using Na(CN)BH₃ and a solvent mixture such as tetrahydrofuran and water in a 30:12 ratio at 60°C.

Liposomes modified with opsonization-inhibition moieties remain in the circulation much longer than unmodified liposomes. For this reason, such liposomes arc sometimes called "stealth" liposomes. Stealth liposomes are known to accumulate in tissues fed by porous or "leaky" microvasculature. Thus, target tissue characterized by such microvasculature defects, for example solid tumors, will efficiently accumulate these liposomes; see Gabizon, et al. (1988), P.N.A.S., USA, 18: 6949-53. In addition, the reduced uptake by the RES lowers the toxicity of stealth liposomes by preventing significant accumulation in the liver and spleen. Thus, liposomes of the invention that are modified with opsonization-inhibition moieties can deliver the present siRNA to tumor cells.

In one embodiment, a liposome of the invention can comprise both opsonization-inhibition moieties and a ligand.

In still other embodiments, the siRNA of the invention can be administered to the subject by any means suitable for mechanically delivering the siRNA to the cells of the tissue at or near the area of uncontrolled complement system activation. For example, the siRNA can be administered by gene gun, or electroporation.

siRNA and pharmaceutical compositions of the invention may be delivered by any route known in the art. For example, suitable enteral administration routes may include eye drops, oral, rectal, or intranasal delivery. Suitable parenteral administration routes may include intravascular administration (e.g., intravenous bolus injection, intravenous infusion, intra-arterial bolus injection, intra-arterial infusion and catheter instillation into the vasculature); peri- and intra-tissue injection (*e.g*., intra-retinal injection, subretinal or intra-vitreal injection); subcutaneous injection or deposition including subcutaneous infusion (such as by osmotic pumps); direct application to the area at or near the site of uncontrolled complement system activation, for example by a catheter or other placement device (*e.g*., a retinal pellet or a suppository or an implant comprising a porous, non-porous, or gelatinous material); and inhalation. In certain embodiments, injections or infusions of siRNA of the invention may be given at or near the site of complement system activation.

In various embodiments, siRNA of the invention can be administered in a single dose or in multiple doses. One skilled in the art can also readily determine an appropriate dosage regimen for administering the siRNA of the invention to a given subject. For example, the siRNA can be administered to the subject once, for example as a single injection or deposition at or near the site of uncontrolled complement system activation. Alternatively, the siRNA can be administered once twice daily to a subject for a period of from about three to about twenty-eight days, or from about seven to about ten days. For example, in some embodiments, the siRNA may be injected at or near the site of uncontrolled complement system activation once a day for seven days. Where a dosage regimen comprises multiple administrations, it is understood that the effective amount of siRNA administered to the subject can comprise the total amount of siRNA administered over the entire dosage regimen.

The siRNA of the invention are preferably formulated as pharmaceutical compositions prior to administering to a subject, according to techniques known in the art. Pharmaceutical compositions of the present invention are characterized as being at least sterile and pyrogen-free. As used herein, "pharmaceutical formulations" include formulations for human and veterinary use. Methods for preparing pharmaceutical compositions of the invention are within the skill in the art, for example as described in Remington's Pharmaceutical Science, 17th ed., Mack Publishing Company, Easton, Pa. (1985), the entire disclosure of which is herein incorporated by reference.

The present pharmaceutical formulations comprise an siRNA of the invention (e.g., 0.1 to 90% by weight), of a physiologically acceptable salt thereof, mixed with a physiologically acceptable carrier medium such as, for example, water, buffered water, normal saline, 0.4% saline, 0.3% glycine, hyaluronic acid and the like.

Pharmaceutical compositions of the invention can also include conventional pharmaceutical cxcipients and/or additives. Suitable pharmaceutical excipients include stabilizers, antioxidants, osmolality adjusting agents, buffers, and pH adjusting agents. Suitable additives include physiologically biocompatible buffers (e.g., tromethamine hydrochloride), additions of chelants (such as, for example, DTPA or DTPA-bisamide) or calcium chelate complexes (as for example calcium DTPA, CaNaDTPA-bisamide), or, optionally, additions of calcium or sodium salts (for example, calcium chloride, calcium ascorbate, calcium gluconate or calcium lactate). Pharmaceutical compositions of the invention can be packaged for use in liquid form, or can be lyophilized.

For solid compositions, conventional nontoxic solid carriers can be used; for example, pharmaceutical grades of mannitol, lactose, starch, magnesium stearate, sodium saccharin, talcum, cellulose, glucose, sucrose, magnesium carbonate, and the like. For example, a solid pharmaceutical composition for oral administration can comprise any of the carriers and excipients listed above and for example about 10-95%, in a further example 25%-75%, of one or more siRNA of the invention. A pharmaceutical composition for aerosol (inhalational) administration can comprise 0.01-20% by weight, in a further example 1%-10% by weight, of one or more siRNA of the invention encapsulated in a liposome as described above, and propellant. A carrier can also be included as desired; *e.g*., lecithin for intranasal delivery.

The invention will now be illustrated with the following non-limiting examples.

### EXAMPLES

### MATERIALS AND METHODS

### Cell Culture

293 cells (human embryonic kidney cells), ARPE19 cells (human retinal epithelial cells), and A549 cells (human lung carcinoma cells) were obtained from ATCC (Manassas, VA) and cultured in Dulbecco's modified eagle medium (DMEM, Gibco, Carlsbad, CA) DMEM/F12 medium (Gibco) or F12-K medium (ATCC) respectively, with 10% fetal bovine serum (FBS; JRH Biosciences, Lenexa, KS) and an antibiotic-antimycotic reagent (Gibco).

### siRNAs

Fifteen siRNAs targeting human complement C3 were synthesized by Integrated DNA Technologies (Coralville, IA). The siRNA target sequences are listed in Table 2.

**Table 2: C3 siRNA Targeting Sequences**

| siRNA Name | Targeting Sequence 5'-3' |
|---|---|
| SEQ. ID. NO. 42 | AAT CCG AGC CGT TCT CTA CAA |
| SEQ. ID. NO. 43 | AAC AAG CTC TGC CGT GAT GAA |
| SEQ. ID. NO. 44 | AAT GGA CAA AGT CGG CAA GTA |
| SEQ. ID. NO. 45 | AAC TAC ATG AAC CTA CAG AGA |
| SEQ. ID. NO. 46 | AAA AAG CGG CCA GTC AGA AGA |
| SEQ. ID. NO. 47 | AAT GAT TGG TGG ATT ACG GAA |
| SEQ. ID. NO. 48 | AAG TCC TCG TTG TCC GTT CCA |
| SEQ. ID. NO. 49 | AAA TGA TTG GTG GAT TAC GGA |
| SEQ. ID. NO. 50 | AAT TAC CGG CAG AAC CAA GAG |
| SEQ. ID. NO. 51 | AAA TGG AAT CTC TAC GAA GCT |
| SEQ. ID. NO. 52 | AAT GAA CAG AGA TAC TAC GGT |
| SEQ. ID. NO. 53 | AAG CCT TGG CTC AAT ACC AAA |
| SEQ. ID. NO. 54 | AAG CGC ATT CCG ATT GAG GAT |
| SEQ. ID. NO. 55 | AAT GGA ATC TCT ACG AAG CTC |
| SEQ. ID. NO. 56 | AAC CTC ATC GCC ATC GAC TCC |

An additional siRNA, targeting enhanced green fluorescent protein (EGFP), served as a negative control (Dharmacon, Lafayette, CO). The sense strand sequence of the siRNA was 5'- GGC UAC GUC CAG GAG CGC AdTdT 3' and the antisense strand sequence was 5'- U GCG CUC CUG GAC GUA GCCdTdT -3'. Cytokine Treatments and Western Blot Analysis

293 cells, ARPE19 cells and A549 cells were cultured in 24 well plates at 37°C with 5% CO₂ overnight. The next day, cells were treated with the following cytokines (R & D Systems, Minneapolis, MN): interleukin 1β (IL-1β; 100ng/mL), interleukin 6 (IL-6; 250ng/mL) and interferon γ (IFN-γ; 500ng/mL).

Two days post-treatment, cell supernatants were collected from each well, and a human complement C3 Western blot was performed. Briefly, supernatants (25 µLs) were electrophoresed on a NuPAGE Novex 4-12% Bis-Tris gel (Invitrogen, Carlsbad, CA). The protein was transferred to nitrocellulose membrane and C3 was detected with a chicken anti-human complement C3 primary antibody (100 ng/mL) (Chemicon, Temecula, CA), an alkaline phosphatase conjugated rabbit anti-chicken IgY secondary antibody (1:10,000, Chemicon) and alkaline phosphatase detection reagent (Novagen, Madison, WI).

### Human Complement siRNA Screening and Dose Response Assay

ARPE19 cells were cultured in 24 well plates at 37°C with 5% CO₂ overnight. The next day, transfections were performed when cells were about 70% confluent. Cells were transfected with 5 nM, 25 nM, 125 nM or 625 nM siRNA mixed in a calcium phosphate (CaPi) reagent. Controls included transfection reagent lacking siRNA and nonspecific siRNA (EGFP1 siRNA). Briefly, siRNA was added to 20 µL of 250 mM CaCl₂ solution. The siRNA/CaCl₂ mixture was added drop-wise to 20 µL of 2X Hanks Balanced Salt Solution (HBS), while mixing by vortex. The siRNA/CaCl₂/HBS complex was added directly to the medium (300 µL/well). After 4 hours of incubation at 37°C, the medium was removed, and the cells were further incubated with 10% DMSO-containing serum-free medium (300 µL/well) at room temperature for 1-2 minutes. This medium was then removed, and the cells were fed again with 200 µL/well growth medium.

Complement C3 stimulation was performed four hours after transfection with IL-1β at a final concentration of 100 ng/mL. 48 hours post-transfection, the supernatant was collected from each well, and samples were analyzed via Western blotting.

### Cytotoxicity Assay

After the supernatant was removed from cells, a cytotoxicity assay was performed. Complete growth medium containing 10% alamarBlue™ (Biosource, Camarillo, CA) was added to each well, and the cells were incubated at 37°C with 5% CO₂ for 3 hours. Cytotoxicity was determined spectrophotometrically at 570 nm using an AD340 plate reader (Beckman Coulter). The alamarBlue™ Assay incorporates an oxidation-reduction (REDOX) indicator that responds to the chemical reduction of growth medium resulting from cell growth. In the presence of growing cells it changes from the oxidized (non-fluorescent, blue color) form to the reduced (fluorescent, red color) form.

### EXAMPLE 1

### Cytokines Upregulate C3 Expression in Cells

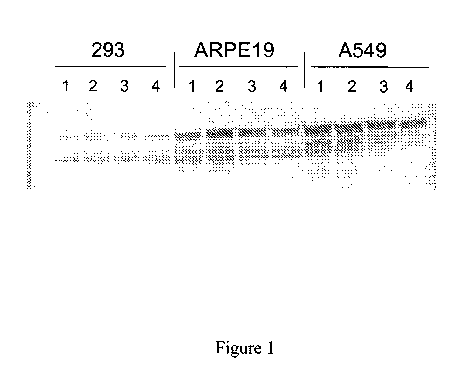

Human complement C3 expression was tested in cells following 2 days of cytokine treatment (Refer to Figure 1 for Example 1). The cytokines used include 100 ng/mL IL-1β (2), 250 ng/ml IL-6 (3), or 500 ng/mL IFN-γ (4). Levels of C3 generated by the cells treated with the cytokines were compared to levels of C3 in untreated cells (I). There was no obvious change in complement C3 expression in 293 and A549 cells treated with any cytokine or in ARPE19 cells treated with 500ng/mL IFN-γ (4). However, C3 expression was significantly up-regulated in ARPE19 cells treated with 100ng/mL IL-1β (2) or 250ng/ml IL-6(3).

### EXAMPLE 2 Human Complement C3 siRNAs Suppress IL-1β -induced Upregulation of Complement C3 Protein in ARPE19 Cells

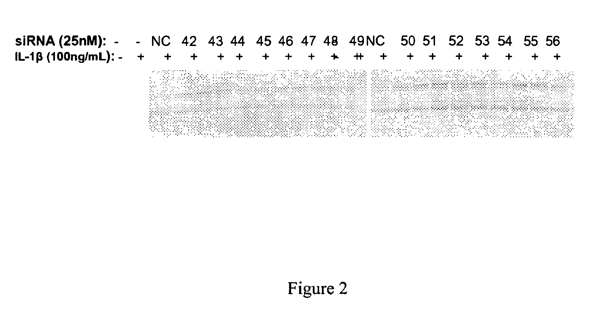

Complement C3 protein expression was upregulated by treating ARPE19 cells with 100 ng/mL of IL-1β treatment. Cells so treated were transfected with 25 nM complement C3 siRNAs or with a negative control siRNA (NC) and C3 protein levels were evaluated by Western blot analysis. As illustrated in Figure 2, cytokine-induced increase of complement C3 protein levels were significantly reduced in cells transfected with human complement C3 siRNAs SEQ. ID. NOS. 43, 45, 48, 49, 51, 53, and 56. Transfections with the negative control siRNA or mock transfections without siRNA had no effect on complement C3 levels.

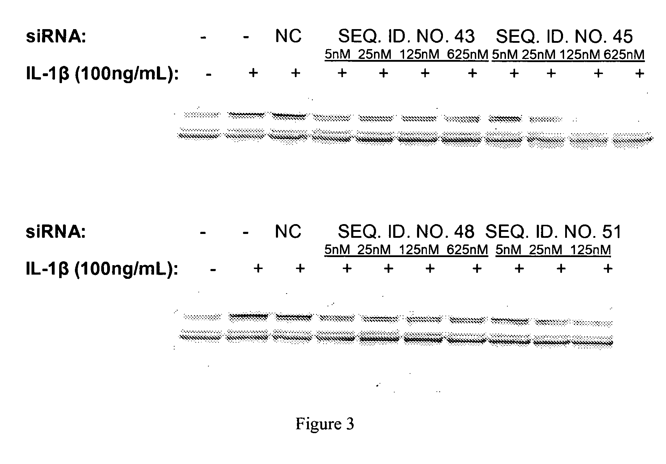

Dose response studies were performed on complement C3 siRNAs (Figure 3). ARPE19 cells treated with 100 ng/ml IL-1β were transfected with 5nM, 25nM, 125nM or 625nM complement C3 siRNAs SEQ. ID. NOS. 43, 45, 48, and 51 or a negative control siRNA (NC). C3 protein was then detected by Western blot analysis. Complement C3 siRNAs SEQ. ID. NOS. 45 and 51 showed the most significant dose response.

### EXAMPLE 3

### Cytotoxicity Assay

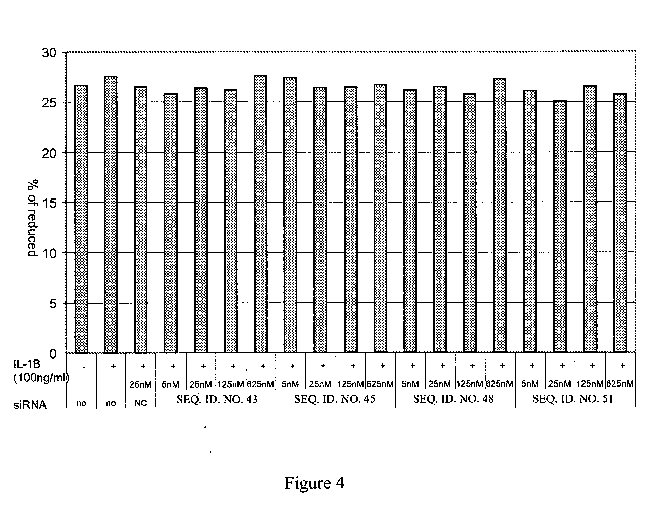

siRNAs were tested for cytotoxicity (Figure 4). ARPE19 cells treated with 100 ng/mL IL-1β were transfected with 5nM, 25nM, 125nM or 625nM complement C3 siRNAs or with negative control siRNA (NC). A cytotoxicity assay was performed 48 hours after transfection and IL-1β treatment using alamarBlue™. The siRNAs (5nM-625nM) were not cytotoxic to the ARPE19 cells.

## Claims

1. An isolated siRNA comprising:
a sense RNA strand having a nucleotide sequence identical to or which differs by a single nucleotide from a target sequence of from about 19 to about 25 contiguous nucleotides of human C3 protein mRNA, and wherein said nucleotide sequence comprises the sequence of one of SEQ ID Nos. 43, 45, 48, 49, 51, 53 or 56; and
an antisense RNA strand having a sequence substantially complementary to the sense RNA strand;
wherein the sense RNA strand and the antisense RNA strand form an RNA duplex.

2. The siRNA of claim 1, wherein the sense RNA strand comprises one RNA molecule, and the antisense RNA strand comprises one RNA molecule.

3. The siRNA of claim 1, wherein the sense and antisense RNA strands of the siRNA are covalently linked.

4. The siRNA of claim 1, wherein the siRNA further comprises non-nucleotide material.

5. The siRNA of claim 1, wherein the sense and antisense RNA strands are stabilized against nuclease degradation.

6. The siRNA of claim 1, wherein the sense RNA strand comprises a first 3' overhang and/or the antisense RNA strand comprises a second 3' overhang.

7. The siRNA of claim 6, wherein the first and/or the second 3' overhang comprises from 1 to about 6 nucleotides.

8. The siRNA of claim 6, wherein the first and/or the second 3' overhang comprises about 2 nucleotides.

9. The siRNA of claim 6, wherein the dinucleotide comprising the first and/or the second 3' overhangs is dithymidine (TT) or diuridine (UU).

10. The siRNA of claim 1, wherein the sense RNA strand has a sequence corresponding to a sequence selected from SEQ ID NO: 43, SEQ ID NO: 45, SEQ ID NO: 48, SEQ ID NO: 49, SEQ ID NO: 51, SEQ ID NO: 53, and SEQ ID NO: 56.

11. A pharmaceutical composition comprising an siRNA of any one of claims 1 to 10, and
a pharmaceutically acceptable carrier.

12. The pharmaceutical composition of claim 11, further comprising a delivery reagent.

13. The pharmaceutical composition of claim 11, wherein the delivery agent is selected from lipofectin, lipofectamine, cellfectin, polycations, liposomes, and combinations thereof.

14. The pharmaceutical composition of any one of claims 11 to 13
wherein said siRNA is naked siRNA.

15. The pharmaceutical composition of claim 13, wherein the liposome further comprises a targeting ligand that targets the liposome to cells at or near the site of a drusen.

16. The pharmaceutical composition of claim 15, wherein the ligand comprises an antibody.

17. The pharmaceutical composition of claim 13, wherein the liposome is modified with an opsonization-inhibition moiety.

18. The pharmaceutical composition of claim 16, wherein the opsonization-inhibiting moiety comprises a PEG, PPG, or derivatives thereof.

19. The siRNA of any one of claims 1 to 10 for inhibiting expression of human complement system or complement system regulator mRNA in a subject in need thereof.

20. The siRNA of claim 19, wherein the subject in need thereof is a human being.

21. The siRNA of claim 19, wherein the effective amount of the siRNA is from about 1 nM to about 100 nM.

22. The siRNA of claim 19, wherein the siRNA is expressed from a recombinant plasmid or a recombinant virus.

23. The siRNA of claim 22, wherein the recombinant virus comprises an adenoviral vector, an adeno-associated viral vector, a lentiviral vector, a retroviral vector, or a herpes virus vector.

24. The siRNA of claim 22, wherein the recombinant virus expresses surface proteins from a virus selected from vesicular stomatitis virus, rabies virus, Ebola virus, or Mokola virus.

25. The siRNA of claim 19, wherein the siRNA is administered enterally or parenterally.

26. The siRNA of claim 19, wherein the siRNA is administered by eye drops or intraorbital injection.

27. The siRNA of any one of claims 1 to 10 for treatment of a disease in a subject.

28. The siRNA of claim 27, wherein the disease is selected from macular degeneration-related disorder, age-related macular disorder, North Carolina macular dystrophy, Sorsby's fundus dystrophy, Stargardt's disease, pattern dystrophy, Best disease, dominant drusen, diabetic retinopathy and malattia leventinese.

29. The siRNA of claim 28, wherein macular degeneration-related disorder is selected from retinal detachment, chorioretinal degenerations, retinal degenerations, photoreceptor degenerations, RPE degenerations, mucopolysaccharidoses, rodcone dystrophies, cone-rod dystrophies, and cone degenerations.

30. The siRNA of claim 27, wherein the disease is age-related macular degeneration.

31. The siRNA of claim 27, wherein the disease is diabetic retinopathy.

32. The siRNA of claim 27, wherein the expression level of the human complement system or complement system regulatory mRNA is detected in urine, blood plasma, serum, whole blood, or eye fluid from the subject.

33. The siRNA of any one of claims 27 to 32, wherein the disease is an ocular disorder associated with drusen.

34. The siRNA of claim 32, wherein the treatment comprises ocular administration selected from topical, intra-retinal injection, subretinal injection, intravitreal injection, and intraorbital injection or eye drops.

35. The siRNA of any one of claims 1, 19 or 27, wherein said siRNA is naked siRNA.

## Patentansprüche

1. Eine isolierte siRNA, die Folgendes beinhaltet:
einen RNA-Sinnstrang, der eine Nukleotidsequenz aufweist, die mit einer Zielsequenz von ungefähr 19 bis ungefähr 25 zusammenhängenden Nukleotiden der menschlichen C3-Protein-mRNA identisch ist oder sich um ein Einzelnukleotid davon unterscheidet, und wobei die Nukleotidsequenz die Sequenz aus einer der SEQ ID NO. 43, 45, 48, 49, 51, 53 oder 56 beinhaltet, und einen RNA-Gegensinnstrang, der eine Sequenz aufweist, die im Wesentlichen komplementär zu dem RNA-Sinnstrang ist;
wobei der RNA-Sinnstrang und der RNA-Gegensinnstrang einen RNA-Duplex bilden.

2. siRNA gemäß Anspruch 1, wobei der RNA-Sinnstrang ein RNA-Molekül beinhaltet und der RNA-Gegensinnstrang ein RNA-Molekül beinhaltet.

3. siRNA gemäß Anspruch 1, wobei der RNA-Sinnstrang und der RNA-Gegensinnstrang der siRNA kovalent gebunden sind.

4. siRNA gemäß Anspruch 1, wobei die siRNA ferner Nicht-Nukleotid-Material beinhaltet.

5. siRNA gemäß Anspruch 1, wobei der RNA-Sinnstrang und der RNA-Gegensinnstrang gegenüber Nukleaseabbau stabilisiert sind.

6. siRNA gemäß Anspruch 1, wobei der RNA-Sinnstrang einen ersten 3'-Überhang beinhaltet und/oder der RNA-Gegensinnstrang einen zweiten 3'-Überhang beinhaltet.

7. siRNA gemäß Anspruch 6, wobei der erste und/oder der zweite 3'-Überhang von 1 bis ungefähr 6 Nukleotide beinhaltet.

8. siRNA gemäß Anspruch 6, wobei der erste und/oder der zweite 3'-Überhang ungefähr 2 Nukleotide beinhaltet.

9. siRNA gemäß Anspruch 6, wobei das Dinukleotid, das den ersten und/oder den zweiten 3'-Überhang beinhaltet, Dithymidin (TT) oder Diuridin (UU) ist.

10. siRNA gemäß Anspruch 1, wobei der RNA-Sinnstrang eine Sequenz aufweist, die einer Sequenz, ausgewählt aus SEQ ID NO: 43, SEQ ID NO: 45, SEQ ID NO: 48, SEQ ID NO: 49, SEQ ID NO: 51, SEQ ID NO: 53 und SEQ ID NO: 56, entspricht.

11. Eine pharmazeutische Zusammensetzung, die eine siRNA gemäß einem der Ansprüche 1 bis 10 und
einen pharmazeutisch zulässigen Träger beinhaltet.

12. Pharmazeutische Zusammensetzung gemäß Anspruch 11, die ferner ein Lieferungsreagens beinhaltet.

13. Pharmazeutische Zusammensetzung gemäß Anspruch 11, wobei das Lieferungsreagens aus Lipofectin, Lipofectamin, Cellfectin, Polykationen, Liposomen und Kombinationen daraus ausgewählt ist.

14. Pharmazeutische Zusammensetzung gemäß einem der Ansprüche 11 bis 13, wobei die siRNA nackte siRNA ist.

15. Pharmazeutische Zusammensetzung gemäß Anspruch 13, wobei das Liposom ferner einen leitenden Liganden beinhaltet, der das Liposom zu Zellen an oder in die Nähe der Stelle von Drusen leitet.

16. Pharmazeutische Zusammensetzung gemäß Anspruch 15, wobei der Ligand einen Antikörper beinhaltet.

17. Pharmazeutische Zusammensetzung gemäß Anspruch 13, wobei das Liposom mit einem Opsonisierungs-Inhibitionsteil modifiziert ist.

18. Pharmazeutische Zusammensetzung gemäß Anspruch 16, wobei der Opsonisierungs-Inhibitionsteil ein PEG, PPG oder Derivate davon beinhaltet.

19. siRNA gemäß einem der Ansprüche 1 bis 10 zum Inhibieren der Expression eines menschlichen Komplementsystems oder einer Komplementsystem-Regulator-mRNA bei einem Subjekt mit Bedarf daran.

20. siRNA gemäß Anspruch 19, wobei das Subjekt mit Bedarf daran ein Mensch ist.

21. siRNA gemäß Anspruch 19, wobei die wirksame Menge der siRNA bei ungefähr 1 nM bis ungefähr 100 nM liegt.

22. siRNA gemäß Anspruch 19, wobei die siRNA aus einem rekombinanten Plasmid oder einem rekombinanten Virus exprimiert wird.

23. siRNA gemäß Anspruch 22, wobei das rekombinante Virus einen adenoviralen Vektor, einen adeno-assoziierten viralen Vektor, einen lentiviralen Vektor, einen retroviralen Vektor oder einen Herpesvirusvektor beinhaltet.

24. siRNA gemäß Anspruch 22, wobei das rekombinante Virus Oberflächenproteine aus einem Virus, ausgewählt aus dem Vesicular-Stomatitis-Virus, Tollwutvirus, Ebolavirus oder Mokolavirus, exprimiert.

25. siRNA gemäß Anspruch 19, wobei die siRNA enteral oder parenteral verabreicht wird.

26. siRNA gemäß Anspruch 19, wobei die siRNA über Augentropfen oder intraorbitale Injektion verabreicht wird.

27. siRNA gemäß einem der Ansprüche 1 bis 10 zur Behandlung einer Krankheit bei einem Subjekt.

28. siRNA gemäß Anspruch 27, wobei die Krankheit aus durch Makuladegeneration bedingten Störungen, altersbedingter Makulastörung, North Carolina Makuladystrophie, Sorsby Fundusdystrophie, Stargardt-Syndrom, Musterdystrophie, Best-Krankheit, dominanten Drusen, diabetischer Retinopathie und Malattia Leventinese ausgewählt ist.

29. siRNA gemäß Anspruch 28, wobei die durch Makuladegeneration bedingte Störung aus Netzhautablösung, chorioretinalen Degenerationen, retinalen Degenerationen, Degenerationen der Sehzelle, RPE-Degenerationen, Mucopolysaccharidosen, Stäbchen-Zapfen-Dystrophien, Zapfen-Stäbchen-Dystrophien und Zapfendegenerationen ausgewählt ist.

30. siRNA gemäß Anspruch 27, wobei die Krankheit altersbedingte Makuladegeneration ist.

31. siRNA gemäß Anspruch 27, wobei die Krankheit diabetische Retinopathie ist.

32. siRNA gemäß Anspruch 27, wobei der Grad an Expression des menschlichen Komplementsystems oder der Regulator-mRNA des Komplementsystems in Urin, Blutplasma, Serum, Vollblut oder Augenfluid von dem Subjekt erfasst wird.

33. siRNA gemäß einem der Ansprüche 27 bis 33, wobei die Krankheit eine mit Drusen assoziierte okulare Störung ist.

34. siRNA gemäß Anspruch 32, wobei die Behandlung okulare Verabreichung, ausgewählt aus topischer, intraretinaler Injektion, subretinaler Injektion, intravitrealer Injektion und intraorbitaler Injektion oder Augentropfen, beinhaltet.

35. siRNA gemäß einem der Ansprüche 1, 19 oder 27, wobei die siRNA nackte siRNA ist.

## Revendications

1. Un ARNsi isolé comprenant :
un brin d'ARN sens ayant une séquence de nucléotides identique à ou qui diffère d'un nucléotide unique d'une séquence cible allant d'environ 19 à environ 25 nucléotides contigus d'ARNm de protéine C3 humaine, et où ladite séquence de nucléotides comprend une séquence parmi les séquences SEQ ID N° 43, 45, 48, 49, 51, 53 ou 56 ; et un brin d'ARN antisens ayant une séquence substantiellement complémentaire au brin d'ARN sens ;
où le brin d'ARN sens et le brin d'ARN antisens forment un duplex d'ARN.

2. L'ARNsi de la revendication 1, où le brin d'ARN sens comprend une molécule d'ARN, et le brin d'ARN antisens comprend une molécule d'ARN.

3. L'ARNsi de la revendication 1, où les brins d'ARN sens et antisens de l'ARNsi sont liés de façon covalente.

4. L'ARNsi de la revendication 1, où l'ARNsi comprend en outre du matériau non nucléotidique.

5. L'ARNsi de la revendication 1, où les brins d'ARN sens et antisens sont stabilisés pour empêcher la dégradation par nucléase.

6. L'ARNsi de la revendication 1, où le brin d'ARN sens comprend un premier débordement en 3' et / ou le brin d'ARN antisens comprend un deuxième débordement en 3'.

7. L'ARNsi de la revendication 6, où le premier et / ou le deuxième débordement en 3' comprend de 1 à environ 6 nucléotides.

8. L'ARNsi de la revendication 6, où le premier et / ou le deuxième débordement en 3' comprend environ 2 nucléotides.

9. L'ARNsi de la revendication 6, où le dinucléotide comprenant les premier et / ou deuxième débordements en 3' est de la dithymidine (TT) ou de la diuridine (UU).

10. L'ARNsi de la revendication 1, où le brin d'ARN sens a une séquence correspondant à une séquence sélectionnée parmi SEQ ID NO : 43, SEQ ID NO : 45, SEQ ID NO : 48, SEQ ID NO : 49, SEQ ID NO : 51, SEQ ID NO : 53, et SEQ ID NO : 56.

11. Une composition pharmaceutique comprenant un ARNsi de n'importe laquelle des revendications 1 à 10, et
un excipient acceptable d'un point de vue pharmaceutique.

12. La composition pharmaceutique de la revendication 11, comprenant en outre un réactif de délivrance.

13. La composition pharmaceutique de la revendication 11, où l'agent de délivrance est sélectionné parmi : lipofectine, lipofectamine, cellfectin, polycations, liposomes, et combinaisons de ceux-ci.

14. La composition pharmaceutique de n'importe laquelle des revendications 11 à 13 où ledit ARNsi est de l'ARNsi nu.

15. La composition pharmaceutique de la revendication 13, où le liposome comprend en outre un ligand de ciblage qui amène le liposome à cibler des cellules au ou près du site d'un druse.

16. La composition pharmaceutique de la revendication 15, où le ligand comprend un anticorps.

17. La composition pharmaceutique de la revendication 13, où le liposome est modifié avec une fraction d'inhibition de l'opsonisation.

18. La composition pharmaceutique de la revendication 16, où la fraction d'inhibition de l'opsonisation comprend un PEG, un PPG, ou des dérivés de ceux-ci.

19. L'ARNsi de n'importe laquelle des revendications 1 à 10 destiné à inhiber l'expression d'ARNm du système du complément humain ou d'ARNm régulateur du système du complément humain chez un sujet qui en a besoin.

20. L'ARNsi de la revendication 19, où le sujet qui en a besoin est un être humain.

21. L'ARNsi de la revendication 19, où la quantité efficace de l'ARNsi est d'environ 1 nM à environ 100 nM.

22. L'ARNsi de la revendication 19, où l'ARNsi est exprimé à partir d'un plasmide recombiné ou d'un virus recombiné.

23. L'ARNsi de la revendication 22, où le virus recombiné comprend un vecteur adénoviral, un vecteur viral adéno-associé, un vecteur lentiviral, un vecteur rétroviral, ou un vecteur du virus de l'herpès.

24. L'ARNsi de la revendication 22, où le virus recombiné exprime des protéines de surface provenant d'un virus sélectionné parmi : virus de la stomatite vésiculaire, virus de la rage, virus Ebola, ou virus Mokola.

25. L'ARNsi de la revendication 19, où l'ARNsi est administré par voie entérale ou parentérale.

26. L'ARNsi de la revendication 19, où l'ARNsi est administré au moyen de gouttes ophtalmiques ou par injection intra-orbitale.

27. L'ARNsi de n'importe laquelle des revendications 1 à 10 destiné au traitement d'une maladie chez un sujet.

28. L'ARNsi de la revendication 27, où la maladie est sélectionnée parmi : trouble apparenté à la dégénérescence maculaire, trouble maculaire lié à l'âge, dystrophie maculaire de la Caroline du Nord, dystrophie du fond de Sorsby, maladie de Stargardt, dystrophie maculaire réticulée, maladie de Best, drusen dominants, rétinopathie diabétique et malattia leventinese.

29. L'ARNsi de la revendication 28, où le trouble apparenté à la dégénérescence maculaire est sélectionné parmi : décollement de la rétine, dégénérescences choriorétiniennes, dégénérescences rétiniennes, dégénérescences des photorécepteurs, dégénérescences RPE, mucopolysaccharidoses, dystrophies bâtonnets-cônes, dystrophies cônes-bâtonnets, et dégénérescences des cônes.

30. L'ARNsi de la revendication 27, où la maladie est la dégénérescence maculaire liée à l'âge.

31. L'ARNsi de la revendication 27, où la maladie est la rétinopathie diabétique.

32. L'ARNsi de la revendication 27, où le niveau d'expression de l'ARNm du système du complément humain ou de l'ARNm régulateur du système du complément humain est détecté dans l'urine, le plasma sanguin, le sérum, le sang entier, ou le liquide oculaire du sujet.

33. L'ARNsi de n'importe laquelle des revendications 27 à 32, où la maladie est un trouble oculaire associé aux drusen.

34. L'ARNsi de la revendication 32, où le traitement comprend une administration oculaire sélectionnée parmi : injection topique, intra-rétinienne, injection sub-rétinienne, injection intra-vitréenne, et injection intra-orbitale ou gouttes ophtalmiques.

35. L'ARNsi de n'importe laquelle des revendications 1, 19 ou 27, où ledit ARNsi est de l'ARNsi nu.


### SEQUENCE LISTING

<110> Reich, Samuel Jotham
   Dejneka, Nadine
<120> COMPOSITIONS AND METHODS FOR REGULATING COMPLEMENT SYSTEM
<130> 129402.01001
<140> Not Yet Assigned
   <141> 2006-12-22
<150> 60/753,041
   <151> 2005-12-22
<160> 57
<170> PatentIn version 3.3
<210> 1
   <211> 2526
   <212> DNA
   <213> Homo sapiens
<400> 1
   gcacacagtg cacgaagacg ctgtcgggag agcccaggat tcaacacggg ccttgagaaa 60
   tgtggctctt gtacctcctg gtgccggccc tgttctgcag ggcaggaggc tccattccca 120
   tccctcagaa gttatttggg gaggtgactt cccctctgtt ccccaagcct taccccaaca 180
   actttgaaac aaccactgtg atcacagtcc ccacgggata cagggtgaag ctcgtcttcc 240
   agcagtttga cctggagcct tctgaaggct gcttctatga ttatgtcaag atctctgctg 300
   ataagaaaag cctggggagg ttctgtgggc aactgggttc tccactgggc aaccccccgg 360
   gaaagaagga atttatgtcc caagggaaca agatgctgct gaccttccac acagacttct 420
   ccaacgagga gaatgggacc atcatgttct acaagggctt cctggcctac taccaagctg 480
   tggaccttga tgaatgtgct tcccggagca aattagggga ggaggatccc cagccccagt 540
   gccagcacct gtgtcacaac tacgttggag gctacttctg ttcctgccgt ccaggctatg 600
   agcttcagga agacaggcat tcctgccagg ctgagtgcag cagcgagctg tacacggagg 660
   catcaggcta catctccagc ctggagtacc ctcggtccta cccccctgac ctgcgctgca 720
   actacagcat ccgggtggag cggggcctca ccctgcacct caagttcctg gagccttttg 780
   atattgatga ccaccagcaa gtacactgcc cctatgacca gctacagatc tatgccaacg 840
   ggaagaacat tggcgagttc tgtgggaagc aaaggccccc cgacctcgac accagcagca 900
   atgctgtgga tctgctgttc ttcacagatg agtcggggga cagccggggc tggaagctgc 960
   gctacaccac cgagatcatc aagtgccccc agcccaagac cctagacgag ttcaccatca 1020
   tccagaacct gcagcctcag taccagttcc gtgactactt cattgctacc tgcaagcaag 1080
   gctaccagct catagagggg aaccaggtgc tgcattcctt cacagctgtc tgccaggatg 1140
   atggcacgtg gcatcgtgcc atgcccagat gcaagatcaa ggactgtggg cagccccgaa 1200
   acctgcctaa tggtgacttc cgttacacca ccacaatggg agtgaacacc tacaaggccc 1260
   gtatccagta ctactgccat gagccatatt acaagatgca gaccagagct ggcagcaggg 1320
   agtctgagca aggggtgtac acctgcacag cacagggcat ttggaagaat gaacagaagg 1380
   gagagaagat tcctcggtgc ttgccagtgt gtgggaagcc cgtgaacccc gtggaacaga 1440
   ggcagcgcat catcggaggg caaaaagcca agatgggcaa cttcccctgg caggtgttca 1500
   ccaacatcca cgggcgcggg ggcggggccc tgctgggcga ccgctggatc ctcacagctg 1560
   cccacaccct gtatcccaag gaacacgaag cgcaaagcaa cgcctctttg gatgtgttcc 1620
   tgggccacac aaatgtggaa gagctcatga agctaggaaa tcaccccatc cgcagggtca 1680
   gcgtccaccc ggactaccgt caggatgagt cctacaattt tgagggggac atcgccctgc 1740
   tggagctgga aaatagtgtc accctgggtc ccaacctcct ccccatctgc ctccctgaca 1800
   acgatacctt ctacgacctg ggcttgatgg gctatgtcag tggcttcggg gtcatggagg 1860
   agaagattgc tcatgacctc aggtttgtcc gtctgcccgt agctaatcca caggcctgtg 1920
   agaactggct ccggggaaag aataggatgg atgtgttctc tcaaaacatg ttctgtgctg 1980
   gacacccatc tctaaagcag gacgcctgcc agggggatag tgggggcgtt tttgcagtaa 2040
   gggacccgaa cactgatcgc tgggtggcca cgggcatcgt gtcctggggc atcgggtgca 2100
   gcaggggcta tggcttctac accaaagtgc tcaactacgt ggactggatc aagaaagaga 2160
   tggaggagga ggactgagcc cagaattcac taggttcgaa tccagagagc agtgtggaaa 2220
   aaaaaaaaca aaaaacaact gaccagttgt tgataaccac taagagtctc tattaaaatt 2280
   actgatgcag aaagaccgtg tgtgaaattc tctttcctgt agtcccattg atgtacttta 2340
   cctgaaacaa cccaaagggc ccctttcttt cttctgagga ttgcagagga tatagttatc 2400
   aatctctagt tgtcactttc ctcttccact ttgataccat tgggtcattg aatataactt 2460
   tttccaaata aagttttatg agaaatgcca gtgtgcaaaa aaaaaaaaaa aaaaaaaaaa 2520
   aaaaaa 2526
<210> 2
   <211> 2714
   <212> DNA
   <213> Homo sapiens
<400> 2
   aatcttatga acagaaccag gacagggagg ctggccggag gttcctgcag agggagcgtc    60
   aaggccctgt gctgctgtcc ctgggggcca gaggggttgc ccagcatgcc cactggcagg   120
   agagagggaa ctgacccact tgctcctacc agcttctgaa ggctccaaag tccggaggtg   180
   cagaaagcca ggaccaagag acaggcagct caccagggtg gacaaatcgc cagagatgtg   240
   gtgcattgtc ctgttttcac ttttggcatg ggtttatgct gagcctacca tgtatgggga   300
   gatcctgtcc cctaactatc ctcaggcata tcccagtgag gtagagaaat cttgggacat   360
   agaagttcct gaagggtatg ggattcacct ctacttcacc catctggaca ttgagctgtc   420
   agagaactgt gcgtatgact cagtgcagat aatctcagga gacactgaag aagggaggct   480
   ctgtggacag aggagcagta acaatcccca ctctccaatt gtggaagagt tccaagtccc   540
   atacaacaaa ctccaggtga tctttaagtc agacttttcc aatgaagagc gttttacggg   600
   gtttgctgca tactatgttg ccacagacat aaatgaatgc acagattttg tagatgtccc 660
   ttgtagccac ttctgcaaca atttcattgg tggttacttc tgctcctgcc ccccggaata 720
   tttcctccat gatgacatga agaattgcgg agttaattgc agtggggatg tattcactgc 780
   actgattggg gagattgcaa gtcccaatta tcccaaacca tatccagaga actcaaggtg 840
   tgaataccag atccggttgg agaaagggtt ccaagtggtg gtgaccttgc ggagagaaga 900
   ttttgatgtg gaagcagctg actcagcggg aaactgcctt gacagtttag tttttgttgc 960
   aggagatcgg caatttggtc cttactgtgg tcatggattc cctgggcctc taaatattga 1020
   aaccaagagt aatgctcttg atatcatctt ccaaactgat ctaacagggc aaaaaaaggg 1080
   ctggaaactt cgctatcatg gagatccaat gccctgccct aaggaagaca ctcccaattc 1140
   tgtttgggag cctgcgaagg caaaatatgt ctttagagat gtggtgcaga taacctgtct 1200
   ggatgggttt gaagttgtgg agggacgtgt tggtgcaaca tctttctatt cgacttgtca 1260
   aagcaatgga aagtggagta attccaaact gaaatgtcaa cctgtggact gtggcattcc 1320
   tgaatccatt gagaatggta aagttgaaga cccagagagc actttgtttg gttctgtcat 1380
   ccgctacact tgtgaggagc catattacta catggaaaat ggaggaggtg gggagtatca 1440
   ctgtgctggt aacgggagct gggtgaatga ggtgctgggc ccggagctgc cgaaatgtgt 1500
   tccagtctgt ggagtcccca gagaaccctt tgaagaaaaa cagaggataa ttggaggatc 1560
   cgatgcagat attaaaaact tcccctggca agtcttcttt gacaacccat gggctggtgg 1620
   agcgctcatt aatgagtact gggtgctgac ggctgctcat gttgtggagg gaaacaggga 1680
   gccaacaatg tatgttgggt ccacctcagt gcagacctca cggctggcaa aatccaagat 1740
   gctcactcct gagcatgtgt ttattcatcc gggatggaag ctgctggaag tcccagaagg 1800
   acgaaccaat tttgataatg acattgcact ggtgcggctg aaagacccag tgaaaatggg 1860
   acccaccgtc tctcccatct gcctaccagg cacctcttcc gactacaacc tcatggatgg 1920
   ggacctggga ctgatctcag gctggggccg aacagagaag agagatcgtg ctgttcgcct 1980
   caaggcggca aggttacctg tagctccttt aagaaaatgc aaagaagtga aagtggagaa 2040
   acccacagca gatgcagagg cctatgtttt cactcctaac atgatctgtg ctggaggaga 2100
   gaagggcatg gatagctgta aaggggacag tggtggggcc tttgctgtac aggatcccaa 2160
   tgacaagacc aaattctacg cagctggcct ggtgtcctgg gggccccagt gtgggaccta 2220
   tgggctctac acacgggtaa agaactatgt tgactggata atgaagacta tgcaggaaaa 2280
   tagcaccccc cgtgaggact aatccagata catcccacca gcctctccaa gggtggtgac 2340
   caatgcatta ccttctgttc cttatgatat tctcattatt tcatcatgac tgaaagaaga 2400
   cacgagcgaa tgatttaaat agaacttgat tgttgagacg ccttgctaga ggtagagttt 2460
   gatcatagaa ttgtgctggt catacatttg tggtctgact ccttggggtc ctttccccgg 2520
   agtacctatt gtagataaca ctatgggtgg ggcactcctt tcttgcacta ttccacaggg 2580
   ataccttaat tctttgtttc ctctttacct gttcaaaatt ccatttactt gatcattctc 2640
   agtatccact gtctatgtac aataaaggat gtttataagc aaaaaaaaaa aaaaaaaaaa 2700
   aaaaaaaaaa aaaa 2714
<210> 3
   <211> 2609
   <212> DNA
   <213> Homo sapiens
<400> 3
   ggctctctac ctctcgccgc ccctagggag gacaccatgg gcccactgat ggttcttttt 60
   tgcctgctgt tcctgtaccc aggtctggca gactcggctc cctcctgccc tcagaacgtg 120
   aatatctcgg gtggcacctt caccctcagc catggctggg ctcctgggag ccttctcacc 180
   tactcctgcc cccagggcct gtacccatcc ccagcatcac ggctgtgcaa gagcagcgga 240
   cagtggcaga ccccaggagc cacccggtct ctgtctaagg cggtctgcaa acctgtgcgc 300
   tgtccagccc ctgtctcctt tgagaatggc atttataccc cacggctggg gtcctatccc 360
   gtgggtggca atgtgagctt cgagtgtgag gatggcttca tattgcgggg ctcgcctgtg 420
   cgtcagtgtc gccccaacgg catgtgggat ggagaaacag ctgtgtgtga taatggggct 480
   ggccactgcc ccaacccagg catttcactg ggcgcagtgc ggacaggctt ccgctttggt 540
   catggggaca aggtccgcta tcgctgctcc tcgaatcttg tgctcacggg gtcttcggag 600
   cgggagtgcc agggcaacgg ggtctggagt ggaacggagc ccatctgccg ccaaccctac 660
   tcttatgact tccctgagga cgtggcccct gccctgggca cttccttctc ccacatgctt 720
   ggggccacca atcccaccca gaagacaaag gaaagcctgg gccgtaaaat ccaaatccag 780
   cgctctggtc atctgaacct ctacctgctc ctggactgtt cgcagagtgt gtcggaaaat 840
   gactttctca tcttcaagga gagcgcctcc ctcatggtgg acaggatctt cagctttgag 900
   atcaatgtga gcgttgccat tatcaccttt gcctcagagc ccaaagtcct catgtctgtc 960
   ctgaacgaca actcccggga tatgactgag gtgatcagca gcctggaaaa tgccaactat 1020
   aaagatcatg aaaatggaac tgggactaac acctatgcag ccttaaacag tgtctatctc 1080
   atgatgaaca accaaatgcg actcctcggc atggaaacga tggcctggca ggaaatccga 1140
   catgccatca tccttctgac agatggaaag tccaatatgg gtggctctcc caagacagct 1200
   gttgaccata tcagagagat cctgaacatc aaccagaaga ggaatgacta tctggacatc 1260
   tatgccatcg gggtgggcaa gctggatgtg gactggagag aactgaatga gctagggtcc 1320
   aagaaggatg gtgagaggca tgccttcatt ctgcaggaca caaaggctct gcaccaggtc 1380
   tttgaacata tgctggatgt ctccaagctc acagacacca tctgcggggt ggggaacatg 1440
   tcagcaaacg cctctgacca ggagaggaca ccctggcatg tcactattaa gcccaagagc 1500
   caagagacct gccggggggc cctcatctcc gaccaatggg tcctgacagc agctcattgc 1560
   ttccgcgatg gcaacgacca ctccctgtgg agggtcaatg tgggagaccc caaatcccag 1620
   tggggcaaag aattccttat tgagaaggcg gtgatctccc cagggtttga tgtctttgcc 1680
   aaaaagaacc agggaatcct ggagttctat ggtgatgaca tagctctgct gaagctggcc 1740
   cagaaagtaa agatgtccac ccatgccagg cccatctgcc ttccctgcac gatggaggcc 1800
   aatctggctc tgcggagacc tcaaggcagc acctgtaggg accatgagaa tgaactgctg 1860
   aacaaacaga gtgttcctgc tcattttgtc gccttgaatg ggagcaaact gaacattaac 1920
   cttaagatgg gagtggagtg gacaagctgt gccgaggttg tctcccaaga aaaaaccatg 1980
   ttccccaact tgacagatgt cagggaggtg gtgacagacc agttcctatg cagtgggacc 2040
   caggaggatg agagtccctg caagggagaa tctgggggag cagttttcct tgagcggaga 2100
   ttcaggtttt ttcaggtggg tctggtgagc tggggtcttt acaacccctg ccttggctct 2160
   gctgacaaaa actcccgcaa aagggcccct cgtagcaagg tcccgccgcc acgagacttt 2220
   cacatcaatc tcttccgcat gcagccctgg ctgaggcagc acctggggga tgtcctgaat 2280
   tttttacccc tctagccatg gccactgagc cctctgctgc cctgccagaa tctgccgccc 2340
   ctccatcttc tacctctgaa tggccaccct tagaccctgt gatccatcct ctctcctagc 2400
   tgagtaaatc cgggtctcta ggatgccaga ggcagcgcac acaagctggg aaatcctcag 2460
   ggctcctacc agcaggactg cctcgctgcc ccacctcccg ctccttggcc tgtccccaga 2520
   ttccttccct ggttgacttg actcatgctt gtttcacttt cacatggaat ttcccagtta 2580
   tgaaattaat aaaaatcaat ggtttccac 2609
<210> 4
   <211> 5101
   <212> DNA
   <213> Homo sapiens
<400> 4
   cactcctccc catcctctcc ctctgtccct ctgtccctct gaccctgcac tgtcccagca     60
   ccatgggacc cacctcaggt cccagcctgc tgctcctgct actaacccac ctccccctgg    120
   ctctggggag tcccatgtac tctatcatca cccccaacat cttgcggctg gagagcgagg    180
   agaccatggt gctggaggcc cacgacgcgc aaggggatgt tccagtcact gttactgtcc    240
   acgacttccc aggcaaaaaa ctagtgctgt ccagtgagaa gactgtgctg acccctgcca    300
   ccaaccacat gggcaacgtc accttcacga tcccagccaa cagggagttc aagtcagaaa    360
   aggggcgcaa caagttcgtg accgtgcagg ccaccttcgg gacccaagtg gtggagaagg    420
   tggtgctggt cagcctgcag agcgggtacc tcttcatcca gacagacaag accatctaca    480
   cccctggctc cacagttctc tatcggatct tcaccgtcaa ccacaagctg ctacccgtgg    540
   gccggacggt catggtcaac attgagaacc cggaaggcat cccggtcaag caggactcct    600
   tgtcttctca gaaccagctt ggcgtcttgc ccttgtcttg ggacattccg gaactcgtca    660
   acatgggcca gtggaagatc cgagcctact atgaaaactc accacagcag gtcttctcca    720
   ctgagtttga ggtgaaggag tacgtgctgc ccagtttcga ggtcatagtg gagcctacag    780
   agaaattcta ctacatctat aacgagaagg gcctggaggt caccatcacc gccaggttcc    840
   tctacgggaa gaaagtggag ggaactgcct ttgtcatctt cgggatccag gatggcgaac    900
   agaggatttc cctgcctgaa tccctcaagc gcattccgat tgaggatggc tcgggggagg    960
   ttgtgctgag ccggaaggta ctgctggacg gggtgcagaa cccccgagca gaagacctgg   1020
   tggggaagtc tttgtacgtg tctgccaccg tcatcttgca ctcaggcagt gacatggtgc 1080
   aggcagagcg cagcgggatc cccatcgtga cctctcccta ccagatccac ttcaccaaga 1140
   cacccaagta cttcaaacca ggaatgccct ttgacctcat ggtgttcgtg acgaaccctg 1200
   atggctctcc agcctaccga gtccccgtgg cagtccaggg cgaggacact gtgcagtctc 1260
   taacccaggg agatggcgtg gccaaactca gcatcaacac acaccccagc cagaagccct 1320
   tgagcatcac ggtgcgcacg aagaagcagg agctctcgga ggcagagcag gctaccagga 1380
   ccatgcaggc tctgccctac agcaccgtgg gcaactccaa caattacctg catctctcag 1440
   tgctacgtac agagctcaga cccggggaga ccctcaacgt caacttcctc ctgcgaatgg 1500
   accgcgccca cgaggccaag atccgctact acacctacct gatcatgaac aagggcaggc 1560
   tgttgaaggc gggacgccag gtgcgagagc ccggccagga cctggtggtg ctgcccctgt 1620
   ccatcaccac cgacttcatc ccttccttcc gcctggtggc gtactacacg ctgatcggtg 1680
   ccagcggcca gagggaggtg gtggccgact ccgtgtgggt ggacgtcaag gactcctgcg 1740
   tgggctcgct ggtggtaaaa agcggccagt cagaagaccg gcagcctgta cctgggcagc 1800
   agatgaccct gaagatagag ggtgaccacg gggcccgggt ggtactggtg gccgtggaca 1860
   agggcgtgtt cgtgctgaat aagaagaaca aactgacgca gagtaagatc tgggacgtgg 1920
   tggagaaggc agacatcggc tgcaccccgg gcagtgggaa ggattacgcc ggtgtcttct 1980
   ccgacgcagg gctgaccttc acgagcagca gtggccagca gaccgcccag agggcagaac 2040
   ttcagtgccc gcagccagcc gcccgccgac gccgttccgt gcagctcacg gagaagcgaa 2100
   tggacaaagt cggcaagtac cccaaggagc tgcgcaagtg ctgcgaggac ggcatgcggg 2160
   agaaccccat gaggttctcg tgccagcgcc ggacccgttt catctccctg ggcgaggcgt 2220
   gcaagaaggt cttcctggac tgctgcaact acatcacaga gctgcggcgg cagcacgcgc 2280
   gggccagcca cctgggcctg gccaggagta acctggatga ggacatcatt gcagaagaga 2340
   acatcgtttc ccgaagtgag ttcccagaga gctggctgtg gaacgttgag gacttgaaag 2400
   agccaccgaa aaatggaatc tctacgaagc tcatgaatat atttttgaaa gactccatca 2460
   ccacgtggga gattctggct gtgagcatgt cggacaagaa agggatctgt gtggcagacc 2520
   ccttcgaggt cacagtaatg caggacttct tcatcgacct gcggctaccc tactctgttg 2580
   ttcgaaacga gcaggtggaa atccgagccg ttctctacaa ttaccggcag aaccaagagc 2640
   tcaaggtgag ggtggaacta ctccacaatc cagccttctg cagcctggcc accaccaaga 2700
   ggcgtcacca gcagaccgta accatccccc ccaagtcctc gttgtccgtt ccatatgtca 2760
   tcgtgccgct aaagaccggc ctgcaggaag tggaagtcaa ggctgctgtc taccatcatt 2820
   tcatcagtga cggtgtcagg aagtccctga aggtcgtgcc ggaaggaatc agaatgaaca 2880
   aaactgtggc tgttcgcacc ctggatccag aacgcctggg ccgtgaagga gtgcagaaag 2940
   aggacatccc acctgcagac ctcagtgacc aagtcccgga caccgagtct gagaccagaa 3000
   ttctcctgca agggacccca gtggcccaga tgacagagga tgccgtcgac gcggaacggc 3060
   tgaagcacct cattgtgacc ccctcgggct gcggggaaca gaacatgatc ggcatgacgc 3120
   ccacggtcat cgctgtgcat tacctggatg aaacggagca gtgggagaag ttcggcctag 3180
   agaagcggca gggggccttg gagctcatca agaaggggta cacccagcag ctggccttca 3240
   gacaacccag ctctgccttt gcggccttcg tgaaacgggc acccagcacc tggctgaccg 3300
   cctacgtggt caaggtcttc tctctggctg tcaacctcat cgccatcgac tcccaagtcc 3360
   tctgcggggc tgttaaatgg ctgatcctgg agaagcagaa gcccgacggg gtcttccagg 3420
   aggatgcgcc cgtgatacac caagaaatga ttggtggatt acggaacaac aacgagaaag 3480
   acatggccct cacggccttt gttctcatct cgctgcagga ggctaaagat atttgcgagg 3540
   agcaggtcaa cagcctgcca ggcagcatca ctaaagcagg agacttcctt gaagccaact 3600
   acatgaacct acagagatcc tacactgtgg ccattgctgg ctatgctctg gcccagatgg 3660
   gcaggctgaa ggggcctctt cttaacaaat ttctgaccac agccaaagat aagaaccgct 3720
   gggaggaccc tggtaagcag ctctacaacg tggaggccac atcctatgcc ctcttggccc 3780
   tactgcagct aaaagacttt gactttgtgc ctcccgtcgt gcgttggctc aatgaacaga 3840
   gatactacgg tggtggctat ggctctaccc aggccacctt catggtgttc caagccttgg 3900
   ctcaatacca aaaggacgcc cctgaccacc aggaactgaa ccttgatgtg tccctccaac 3960
   tgcccagccg cagctccaag atcacccacc gtatccactg ggaatctgcc agcctcctgc 4020
   gatcagaaga gaccaaggaa aatgagggtt tcacagtcac agctgaagga aaaggccaag 4080
   gcaccttgtc ggtggtgaca atgtaccatg ctaaggccaa agatcaactc acctgtaata 4140
   aattcgacct caaggtcacc ataaaaccag caccggaaac agaaaagagg cctcaggatg 4200
   ccaagaacac tatgatcctt gagatctgta ccaggtaccg gggagaccag gatgccacta 4260
   tgtctatatt ggacatatcc atgatgactg gctttgctcc agacacagat gacctgaagc 4320
   agctggccaa tggtgttgac agatacatct ccaagtatga gctggacaaa gccttctccg 4380
   ataggaacac cctcatcatc tacctggaca aggtctcaca ctctgaggat gactgtctag 4440
   ctttcaaagt tcaccaatac tttaatgtag agcttatcca gcctggagca gtcaaggtct 4500
   acgcctatta caacctggag gaaagctgta cccggttcta ccatccggaa aaggaggatg 4560
   gaaagctgaa caagctctgc cgtgatgaac tgtgccgctg tgctgaggag aattgcttca 4620
   tacaaaagtc ggatgacaag gtcaccctgg aagaacggct ggacaaggcc tgtgagccag 4680
   gagtggacta tgtgtacaag acccgactgg tcaaggttca gctgtccaat gactttgacg 4740
   agtacatcat ggccattgag cagaccatca agtcaggctc ggatgaggtg caggttggac 4800
   agcagcgcac gttcatcagc cccatcaagt gcagagaagc cctgaagctg gaggagaaga 4860
   aacactacct catgtggggt ctctcctccg atttctgggg agagaagccc aacctcagct 4920
   acatcatcgg gaaggacact tgggtggagc actggcccga ggaggacgaa tgccaagacg 4980
   aagagaacca gaaacaatgc caggacctcg gcgccttcac cgagagcatg gttgtctttg 5040
   ggtgccccaa ctgaccacac ccccattccc ccactccaga taaagcttca gttatatctc 5100
   a 5101
<210> 5
   <211> 5426
   <212> DNA
   <213> Homo sapiens
<400> 5
   agaaggtagc agacagacag acggatctaa cctctcttgg atcctccagc catgaggctg 60
   ctctgggggc tgatctgggc atccagcttc ttcaccttat ctctgcagaa gcccaggttg 120
   ctcttgttct ctccttctgt ggttcatctg ggggtccccc tatcggtggg ggtgcagctc 180
   caggatgtgc cccgaggaca ggtagtgaaa ggatcagtgt tcctgagaaa cccatctcgt 240
   aataatgtcc cctgctcccc aaaggtggac ttcaccctta gctcagaaag agacttcgca 300
   ctcctcagtc tccaggtgcc cttgaaagat gcgaagagct gtggcctcca tcaactcctc 360
   agaggccctg aggtccagct ggtggcccat tcgccatggc taaaggactc tctgtccaga 420
   acgacaaaca tccagggtat caacctgctc ttctcctctc gccgggggca cctctttttg 480
   cagacggacc agcccattta caaccctggc cagcgggttc ggtaccgggt ctttgctctg 540
   gatcagaaga tgcgcccgag cactgacacc atcacagtca tggtggagaa ctctcacggc 600
   ctccgcgtgc ggaagaagga ggtgtacatg ccctcgtcca tcttccagga tgactttgtg 660
   atcccagaca tctcagagcc agggacctgg aagatctcag cccgattctc agatggcctg 720
   gaatccaaca gcagcaccca gtttgaggtg aagaaatatg tccttcccaa ctttgaggtg 780
   aagatcaccc ctggaaagcc ctacatcctg acggtgccag gccatcttga tgaaatgcag 840
   ttagacatcc aggccaggta catctatggg aagccagtgc agggggtggc atatgtgcgc 900
   tttgggctcc tagatgagga tggtaagaag actttctttc gggggctgga gagtcagacc 960
   aagctggtga atggacagag ccacatttcc ctctcaaagg cagagttcca ggacgccctg 1020
   gagaagctga atatgggcat tactgacctc caggggctgc gcctctacgt tgctgcagcc 1080
   atcattgagt ctccaggtgg ggagatggag gaggcagagc tcacatcctg gtattttgtg 1140
   tcatctccct tctccttgga tcttagcaag accaagcgac accttgtgcc tggggccccc 1200
   ttcctgctgc aggccttggt ccgtgagatg tcaggctccc cagcttctgg cattcctgtc 1260
   aaagtttctg ccacggtgtc ttctcctggg tctgttcctg aagtccagga cattcagcaa 1320
   aacacagacg ggagcggcca agtcagcatt ccaataatta tccctcagac catctcagag 1380
   ctgcagctct cagtatctgc aggctcccca catccagcga tagccaggct cactgtggca 1440
   gccccacctt caggaggccc cgggtttctg tctattgagc ggccggattc tcgacctcct 1500
   cgtgttgggg acactctgaa cctgaacttg cgagccgtgg gcagtggggc caccttttct 1560
   cattactact acatgatcct atcccgaggg cagatcgtgt tcatgaatcg agagcccaag 1620
   aggaccctga cctcggtctc ggtgtttgtg gaccatcacc tggcaccctc cttctacttt 1680
   gtggccttct actaccatgg agaccaccca gtggccaact ccctgcgagt ggatgtccag 1740
   gctggggcct gcgagggcaa gctggagctc agcgtggacg gtgccaagca gtaccggaac 1800
   ggggagtccg tgaagctcca cttagaaacc gactccctag ccctggtggc gctgggagcc 1860
   ttggacacag ctctgtatgc tgcaggcagc aagtcccaca agcccctcaa catgggcaag 1920
   gtctttgaag ctatgaacag ctatgacctc ggctgtggtc ctgggggtgg ggacagtgcc 1980
   cttcaggtgt tccaggcagc gggcctggcc ttttctgatg gagaccagtg gaccttatcc 2040
   agaaagagac taagctgtcc caaggagaag acaacccgga aaaagagaaa cgtgaacttc 2100
   caaaaggcga ttaatgagaa attgggtcag tatgcttccc cgacagccaa gcgctgctgc 2160
   caggatgggg tgacacgtct gcccatgatg cgttcctgcg agcagcgggc agcccgcgtg 2220
   cagcagccgg actgccggga gcccttcctg tcctgctgcc aatttgctga gagtctgcgc 2280
   aagaagagca gggacaaggg ccaggcgggc ctccaacgag ccctggagat cctgcaggag 2340
   gaggacctga ttgatgagga tgacattccc gtgcgcagct tcttcccaga gaactggctc 2400
   tggagagtgg aaacagtgga ccgctttcaa atattgacac tgtggctccc cgactctctg 2460
   accacgtggg agatccatgg cctgagcctg tccaaaacca aaggcctatg tgtggccacc 2520
   ccagtccagc tccgggtgtt ccgcgagttc cacctgcacc tccgcctgcc catgtctgtc 2580
   cgccgctttg agcagctgga gctgcggcct gtcctctata actacctgga taaaaacctg 2640
   actgtgagcg tccacgtgtc cccagtggag gggctgtgcc tggctggggg cggagggctg 2700
   gcccagcagg tgctggtgcc tgcgggctct gcccggcctg ttgccttctc tgtggtgccc 2760
   acggcagccg ccgctgtgtc tctgaaggtg gtggctcgag ggtccttcga attccctgtg 2820
   ggagatgcgg tgtccaaggt tctgcagatt gagaaggaag gggccatcca tagagaggag 2880
   ctggtctatg aactcaaccc cttggaccac cgaggccgga ccttggaaat acctggcaac 2940
   tctgatccca atatgatccc tgatggggac tttaacagct acgtcagggt tacagcctca 3000
   gatccattgg acactttagg ctctgagggg gccttgtcac caggaggcgt ggcctccctc 3060
   ttgaggcttc ctcgaggctg tggggagcaa accatgatct acttggctcc gacactggct 3120
   gcttcccgct acctggacaa gacagagcag tggagcacac tgcctcccga gaccaaggac 3180
   cacgccgtgg atctgatcca gaaaggctac atgcggatcc agcagtttcg gaaggcggat 3240
   ggttcctatg cggcttggtt gtcacgggac agcagcacct ggctcacagc ctttgtgttg 3300
   aaggtcctga gtttggccca ggagcaggta ggaggctcgc ctgagaaact gcaggagaca 3360
   tctaactggc ttctgtccca gcagcaggct gacggctcgt tccaggaccc ctgtccagtg 3420
   ttagacagga gcatgcaggg gggtttggtg ggcaatgatg agactgtggc actcacagcc 3480
   tttgtgacca tcgcccttca tcatgggctg gccgtcttcc aggatgaggg tgcagagcca 3540
   ttgaagcaga gagtggaagc ctccatctca aaggcaaact catttttggg ggagaaagca 3600
   agtgctgggc tcctgggtgc ccacgcagct gccatcacgg cctatgccct gacactgacc 3660
   aaggcgcctg tggacctgct cggtgttgcc cacaacaacc tcatggcaat ggcccaggag 3720
   actggagata acctgtactg gggctcagtc actggttctc agagcaatgc cgtgtcgccc 3780
   accccggctc ctcgcaaccc atccgacccc atgccccagg ccccagccct gtggattgaa 3840
   accacagcct acgccctgct gcacctcctg cttcacgagg gcaaagcaga gatggcagac 3900
   caggcttcgg cctggctcac ccgtcagggc agcttccaag ggggattccg cagtacccaa 3960
   gacacggtga ttgccctgga tgccctgtct gcctactgga ttgcctccca caccactgag 4020
   gagaggggtc tcaatgtgac tctcagctcc acaggccgga atgggttcaa gtcccacgcg 4080
   ctgcagctga acaaccgcca gattcgcggc ctggaggagg agctgcagtt ttccttgggc 4140
   agcaagatca atgtgaaggt gggaggaaac agcaaaggaa ccctgaaggt ccttcgtacc 4200
   tacaatgtcc tggacatgaa gaacacgacc tgccaggacc tacagataga agtgacagtc 4260
   aaaggccacg tcgagtacac gatggaagca aacgaggact atgaggacta tgagtacgat 4320
   gagcttccag ccaaggatga cccagatgcc cctctgcagc ccgtgacacc cctgcagctg 4380
   tttgagggtc ggaggaaccg ccgcaggagg gaggcgccca aggtggtgga ggagcaggag 4440
   tccagggtgc actacaccgt gtgcatctgg cggaacggca aggtggggct gtctggcatg 4500
   gccatcgcgg acgtcaccct cctgagtgga ttccacgccc tgcgtgctga cctggagaag 4560
   ctgacctccc tctctgaccg ttacgtgagt cactttgaga ccgaggggcc ccacgtcctg 4620
   ctgtattttg actcggtccc cacctcccgg gagtgcgtgg gctttgaggc tgtgcaggaa 4680
   gtgccggtgg ggctggtgca gccggccagc gcaaccctgt acgactacta caaccccgag 4740
   cgcagatgtt ctgtgtttta cggggcacca agtaagagca gactcttggc caccttgtgt 4800
   tctgctgaag tctgccagtg tgctgagggg aagtgccctc gccagcgtcg cgccctggag 4860
   cggggtctgc aggacgagga tggctacagg atgaagtttg cctgctacta cccccgtgtg 4920
   gagtacggct tccaggttaa ggttctccga gaagacagca gagctgcttt ccgcctcttt 4980
   gagaccaaga tcacccaagt cctgcacttc accaaggatg tcaaggccgc tgctaatcag 5040
   atgcgcaact tcctggttcg agcctcctgc cgccttcgct tggaacctgg gaaagaatat 5100
   ttgatcatgg gtctggatgg ggccacctat gacctcgagg gacaccccca gtacctgctg 5160
   gactcgaata gctggatcga ggagatgccc tctgaacgcc tgtgccggag cacccgccag 5220
   cgggcagcct gtgcccagct caacgacttc ctccaggagt atggcactca ggggtgccag 5280
   gtgtgagggc tgccctccca cctccgctgg gaggaacctg aacctgggaa ccatgaagct 5340
   ggaagcactg ctgtgtccgc tttcatgaac acagcctggg accagggcat attaaaggct 5400
   tttggcagca aagtgtcagt gttggc 5426
<210> 6
   <211> 5426
   <212> DNA
   <213> Homo sapiens
<400> 6
   agaaggtagc agacagacag acggatctaa cctctcttgg atcctccagc catgaggctg 60
   ctctgggggc tgatctgggc atccagcttc ttcaccttat ctctgcagaa gcccaggttg 120
   ctcttgttct ctccttctgt ggttcatctg ggggtccccc tatcggtggg ggtgcagctc 180
   caggatgtgc cccgaggaca ggtagtgaaa ggatcagtgt tcctgagaaa cccatctcgt 240
   aataatgtcc cctgctcccc aaaggtggac ttcaccctta gctcagaaag agacttcgca 300
   ctcctcagtc tccaggtgcc cttgaaagat gcgaagagct gtggcctcca tcaactcctc 360
   agaggccctg aggtccagct ggtggcccat tcgccatggc taaaggactc tctgtccaga 420
   acgacaaaca tccagggtat caacctgctc ttctcctctc gccgggggca cctctttttg 480
   cagacggacc agcccattta caaccctggc cagcgggttc ggtaccgggt ctttgctctg 540
   gatcagaaga tgcgcccgag cactgacacc atcacagtca tggtggagaa ctctcacggc 600
   ctccgcgtgc ggaagaagga ggtgtacatg ccctcgtcca tcttccagga tgactttgtg 660
   atcccagaca tctcagagcc agggacctgg aagatctcag cccgattctc agatggcctg 720
   gaatccaaca gcagcaccca gtttgaggtg aagaaatatg tccttcccaa ctttgaggtg 780
   aagatcaccc ctggaaagcc ctacatcctg acggtgccag gccatcttga tgaaatgcag 840
   ttagacatcc aggccaggta catctatggg aagccagtgc agggggtggc atatgtgcgc 900
   tttgggctcc tagatgagga tggtaagaag actttctttc gggggctgga gagtcagacc 960
   aagctggtga atggacagag ccacatttcc ctctcaaagg cagagttcca ggacgccctg 1020
   gagaagctga atatgggcat tactgacctc caggggctgc gcctctacgt tgctgcagcc 1080
   atcattgagt ctccaggtgg ggagatggag gaggcagagc tcacatcctg gtattttgtg 1140
   tcatctccct tctccttgga tcttagcaag accaagcgac accttgtgcc tggggccccc 1200
   ttcctgctgc aggccttggt ccgtgagatg tcaggctccc cagcttctgg cattcctgtc 1260
   aaagtttctg ccacggtgtc ttctcctggg tctgttcctg aagtccagga cattcagcaa 1320
   aacacagacg ggagcggcca agtcagcatt ccaataatta tccctcagac catctcagag 1380
   ctgcagctct cagtatctgc aggctcccca catccagcga tagccaggct cactgtggca 1440
   gccccacctt caggaggccc cgggtttctg tctattgagc ggccggattc tcgacctcct 1500
   cgtgttgggg acactctgaa cctgaacttg cgagccgtgg gcagtggggc caccttttct 1560
   cattactact acatgatcct atcccgaggg cagatcgtgt tcatgaatcg agagcccaag 1620
   aggaccctga cctcggtctc ggtgtttgtg gaccatcacc tggcaccctc cttctacttt 1680
   gtggccttct actaccatgg agaccaccca gtggccaact ccctgcgagt ggatgtccag 1740
   gctggggcct gcgagggcaa gctggagctc agcgtggacg gtgccaagca gtaccggaac 1800
   ggggagtccg tgaagctcca cttagaaacc gactccctag ccctggtggc gctgggagcc 1860
   ttggacacag ctctgtatgc tgcaggcagc aagtcccaca agcccctcaa catgggcaag 1920
   gtctttgaag ctatgaacag ctatgacctc ggctgtggtc ctgggggtgg ggacagtgcc 1980
   cttcaggtgt tccaggcagc gggcctggcc ttttctgatg gagaccagtg gaccttatcc 2040
   agaaagagac taagctgtcc caaggagaag acaacccgga aaaagagaaa cgtgaacttc 2100
   caaaaggcga ttaatgagaa attgggtcag tatgcttccc cgacagccaa gcgctgctgc 2160
   caggatgggg tgacacgtct gcccatgatg cgttcctgcg agcagcgggc agcccgcgtg 2220
   cagcagccgg actgccggga gcccttcctg tcctgctgcc aatttgctga gagtctgcgc 2280
   aagaagagca gggacaaggg ccaggcgggc ctccaacgag ccctggagat cctgcaggag 2340
   gaggacctga ttgatgagga tgacattccc gtgcgcagct tcttcccaga gaactggctc 2400
   tggagagtgg aaacagtgga ccgctttcaa atattgacac tgtggctccc cgactctctg 2460
   accacgtggg agatccatgg cctgagcctg tccaaaacca aaggcctatg tgtggccacc 2520
   ccagtccagc tccgggtgtt ccgcgagttc cacctgcacc tccgcctgcc catgtctgtc 2580
   cgccgctttg agcagctgga gctgcggcct gtcctctata actacctgga taaaaacctg 2640
   actgtgagcg tccacgtgtc cccagtggag gggctgtgcc tggctggggg cggagggctg 2700
   gcccagcagg tgctggtgcc tgcgggctct gcccggcctg ttgccttctc tgtggtgccc 2760
   acggcagcca ccgctgtgtc tctgaaggtg gtggctcgag ggtccttcga attccctgtg 2820
   ggagatgcgg tgtccaaggt tctgcagatt gagaaggaag gggccatcca tagagaggag 2880
   ctggtctatg aactcaaccc cttggaccac cgaggccgga ccttggaaat acctggcaac 2940
   tctgatccca atatgatccc tgatggggac tttaacagct acgtcagggt tacagcctca 3000
   gatccattgg acactttagg ctctgagggg gccttgtcac caggaggcgt ggcctccctc 3060
   ttgaggcttc ctcgaggctg tggggagcaa accatgatct acttggctcc gacactggct 3120
   gcttcccgct acctggacaa gacagagcag tggagcacac tgcctcccga gaccaaggac 3180
   cacgccgtgg atctgatcca gaaaggctac atgcggatcc agcagtttcg gaaggcggat 3240
   ggttcctatg cggcttggtt gtcacggggc agcagcacct ggctcacagc ctttgtgttg 3300
   aaggtcctga gtttggccca ggagcaggta ggaggctcgc ctgagaaact gcaggagaca 3360
   tctaactggc ttctgtccca gcagcaggct gacggctcgt tccaggacct ctctccagtg 3420
   atacatagga gcatgcaggg gggtttggtg ggcaatgatg agactgtggc actcacagcc 3480
   tttgtgacca tcgcccttca tcatgggctg gccgtcttcc aggatgaggg tgcagagcca 3540
   ttgaagcaga gagtggaagc ctccatctca aaggcaagct catttttggg ggagaaagca 3600
   agtgctgggc tcctgggtgc ccacgcagct gccatcacgg cctatgccct gacactgacc 3660
   aaggcccctg cggacctgcg gggtgttgcc cacaacaacc tcatggcaat ggcccaggag 3720
   actggagata acctgtactg gggctcagtc actggttctc agagcaatgc cgtgtcgccc 3780
   accccggctc ctcgcaaccc atccgacccc atgccccagg ccccagccct gtggattgaa 3840
   accacagcct acgccctgct gcacctcctg cttcacgagg gcaaagcaga gatggcagac 3900
   caggctgcgg cctggctcac ccgtcagggc agcttccaag gggattccgg cagtacccaa 3960
   gacacggtga ttgccctgga tgccctgtct gcctactgga ttgcctccca caccactgag 4020
   gagaggggtc tcaatgtgac tctcagctcc acaggccgga atgggttcaa gtcccacgcg 4080
   ctgcagctga acaaccgcca gattcgcggc ctggaggagg agctgcagtt ttccttgggc 4140
   agcaagatca atgtgaaggt gggaggaaac agcaaaggaa ccctgaaggt ccttcgtacc 4200
   tacaatgtcc tggacatgaa gaacacgacc tgccaggacc tacagataga agtgacagtc 4260
   aaaggccacg tcgagtacac gatggaagca aacgaggact atgaggacta tgagtacgat 4320
   gagcttccag ccaaggatga cccagatgcc cctctgcagc ccgtgacacc cctgcagctg 4380
   tttgagggtc ggaggaaccg ccgcaggagg gaggcgccca aggtggtgga ggagcaggag 4440
   tccagggtgc actacaccgt gtgcatctgg cggaacggca aggtggggct gtctggcatg   4500
   gccatcgcgg acgtcaccct cctgagtgga ttccacgccc tgcgtgctga cctggagaag   4560
   ctgacctccc tctctgaccg ttacgtgagt cactttgaga ccgaggggcc ccacgtcctg   4620
   ctgtattttg actcggtccc cacctcccgg gagtgcgtgg gctttgaggc tgtgcaggaa   4680
   gtgccggtgg ggctggtgca gccggccagc gcaaccctgt acgactacta caaccccgag   4740
   cgcagatgtt ctgtgtttta cggggcacca agtaagagca gactcttggc caccttgtgt   4800
   tctgctgaag tctgccagtg tgctgagggg aagtgccctc gccagcgtcg cgccctggag   4860
   cggggtctgc aggacgagga tggctacagg atgaagtttg cctgctacta cccccgtgtg   4920
   gagtacggct tccaggttaa ggttctccga gaagacagca gagctgcttt ccgcctcttt   4980
   gagaccaaga tcacccaagt cctgcacttc accaaggatg tcaaggccgc tgctaatcag   5040
   atgcgcaact tcctggttcg agcctcctgc cgccttcgct tggaacctgg gaaagaatat   5100
   ttgatcatgg gtctggatgg ggccacctat gacctcgagg gacaccccca gtacctgctg   5160
   gactcgaata gctggatcga ggagatgccc tctgaacgcc tgtgccggag cacccgccag   5220
   cgggcagcct gtgcccagct caacgacttc ctccaggagt atggcactca ggggtgccag   5280
   gtgtgagggc tgccctccca cctccgctgg gaggaacctg aacctgggaa ccatgaagct   5340
   ggaagcactg ctgtgtccgc tttcatgaac acagcctggg accagggcat attaaaggct   5400
   tttggcagca aagtgtcagt gttggc                                        5426
<210> 7
   <211> 5480
   <212> DNA
   <213> Homo sapiens
<400> 7
<210> 8
   <211> 3551
   <212> DNA
   <213> Homo sapiens
<400> 8
   ttgccttgtg ttagctagca ataagaaaag aagctttgtt tggattaaca tatataccct 60
   cttcattctg catacctatt ttttccccaa taatttgcag cttaggtccg aggacaccac 120
   aaactctgct taaagggcct ggaggctctc aaggcatggc cagacgctct gtcttgtact 180
   tcatcctgct gaatgctctg atcaacaagg gccaagcctg cttctgtgat cactatgcat 240
   ggactcagtg gaccagctgc tcaaaaactt gcaattctgg aacccagagc agacacagac 300
   aaatagtagt agataagtac taccaggaaa acttttgtga acagatttgc agcaagcagg 360
   agactagaga atgtaactgg caaagatgcc ccatcaactg cctcctggga gattttggac 420
   catggtcaga ctgtgaccct tgtattgaaa aacagtctaa agttagatct gtcttgcgtc 480
   ccagtcagtt tgggggacag ccatgcactg agcctctggt agcctttcaa ccatgcattc 540
   catctaagct ctgcaaaatt gaagaggctg actgcaagaa taaatttcgc tgtgacagtg 600
   gccgctgcat tgccagaaag ttagaatgca atggagaaaa tgactgtgga gacaattcag 660
   atgaaaggga ctgtgggagg acaaaggcag tatgcacacg gaagtataat cccatcccta 720
   gtgtacagtt gatgggcaat gggtttcatt ttctggcagg agagcccaga ggagaagtcc 780
   ttgataactc tttcactgga ggaatatgta aaactgtcaa aagcagtagg acaagtaatc 840
   cataccgtgt tccggccaat ctggaaaatg tcggctttga ggtacaaact gcagaagatg 900
   acttgaaaac agatttctac aaggatttaa cttctcttgg acacaatgaa aatcaacaag 960
   gctcattctc aagtcagggg gggagctctt tcagtgtacc aattttttat tcctcaaaga 1020
   gaagtgaaaa tatcaaccat aattctgcct tcaaacaagc cattcaagcc tctcacaaaa 1080
   aggattctag ttttattagg atccataaag tgatgaaagt cttaaacttc acaacgaaag 1140
   ctaaagatct gcacctttct gatgtctttt tgaaagcact taaccatctg cctctagaat 1200
   acaactctgc tttgtacagc cgaatattcg atgactttgg gactcattac ttcacctctg 1260
   gctccctggg aggcgtgtat gaccttctct atcagtttag cagtgaggaa ctaaagaact 1320
   caggtttaac cgaggaagaa gccaaacact gtgtcaggat tgaaacaaag aaacgcgttt 1380
   tatttgctaa gaaaacaaaa gtggaacata ggtgcaccac caacaagctg tcagagaaac 1440
   atgaaggttc atttatacag ggagcagaga aatccatatc cctgattcga ggtggaagga 1500
   gtgaatatgg agcagctttg gcatgggaga aagggagctc tggtctggag gagaagacat 1560
   tttctgagtg gttagaatca gtgaaggaaa atcctgctgt gattgacttt gagcttgccc 1620
   ccatcgtgga cttggtaaga aacatcccct gtgcagtgac aaaacggaac aacctcagga 1680
   aagctttgca agagtatgca gccaagttcg atccttgcca gtgtgctcca tgccctaata 1740
   atggccgacc caccctctca gggactgaat gtctgtgtgt gtgtcagagt ggcacctatg 1800
   gtgagaactg tgagaaacag tctccagatt ataaatccaa tgcagtagac ggacagtggg 1860
   gttgttggtc ttcctggagt acctgtgatg ctacttataa gagatcgaga acccgagaat 1920
   gcaataatcc tgccccccaa cgaggaggga aacgctgtga gggggagaag cgacaagagg 1980
   aagactgcac attttcaatc atggaaaaca atggacaacc atgtatcaat gatgatgaag 2040
   aaatgaaaga ggtcgatctt cctgagatag aagcagattc cgggtgtcct cagccagttc 2100
   ctccagaaaa tggatttatc cggaatgaaa agcaactata cttggttgga gaagatgttg 2160
   aaatttcatg ccttactggc tttgaaactg ttggatacca gtacttcaga tgcttaccag 2220
   acgggacctg gagacaaggg gatgtggaat gccaacggac ggagtgcatc aagccagttg 2280
   tgcaggaagt cctgacaatt acaccatttc agagattgta tagaattggt gaatccattg 2340
   agctaacttg ccccaaaggc tttgttgttg ctgggccatc aaggtacaca tgccagggga 2400
   attcctggac accacccatt tcaaactctc tcacctgtga aaaagatact ctaacaaaat 2460
   taaaaggcca ttgtcagctg ggacagaaac aatcaggatc tgaatgcatt tgtatgtctc 2520
   cagaagaaga ctgtagccat cattcagaag atctctgtgt gtttgacaca gactccaacg 2580
   attactttac ttcacccgct tgtaagtttt tggctgagaa atgtttaaat aatcagcaac 2640
   tccattttct acatattggt tcctgccaag acggccgcca gttagaatgg ggtcttgaaa 2700
   ggacaagact ttcatccaac agcacaaaga aagaatcctg tggctatgac acctgctatg 2760
   actgggaaaa atgttcagcc tccacttcca aatgtgtctg cctattgccc ccacagtgct 2820
   tcaagggtgg aaaccaactc tactgtgtca aaatgggatc atcaacaagt gagaaaacat 2880
   tgaacatctg tgaagtggga actataagat gtgcaaacag gaagatggaa atactgcatc 2940
   ctggaaagtg tttggcctag cacaattact gctaggccca gcacaatgaa cagatttacc 3000
   atcccgaaga accaactcct acaaatgaga attcttgcac aaacagcaga ctggcatgct 3060
   caaagttact gacaaaaatt attttctgtt agtttgagat cattattctc ccctgactct 3120
   cctgtttggg catgtcttat tcagttccag ctcatgacgc cctgtagcat acccctaggt 3180
   accaacttcc acagcagtct cgtaaattct cctgttcaca ttgtacaaaa ataatgtgac 3240
   ttctgaggcc cttatgtagc ctgtgacatt aagcattctc acaattagaa ataagaataa 3300
   aacccataat tttcttcaat gagttaataa acagaaatct ccagaacctc tgaaacacat 3360
   tcttgaagcc cagctttcat atcttcattc aacaaataat ttctgagtgt gtatacagga 3420
   tgtcaagtac tgaccaaagt cctgagaact cggcagataa taaaacagac aaaagccttt 3480
   gccttcatga agcatacatt cattcagggg tagacacaca aaaaatgaaa taaacaggta 3540
   aaatatgtag c 3551
<210> 9
   <211> 4034
   <212> DNA
   <213> Homo sapiens
<400> 9
   agggagaggc agagaggcag gcagcctgct gggctcttcc tgctgttgaa aacttacccg 60
   gcccttacag aggaaatctt cctcctctct tctgccctga atgttttccc aaacatgaag 120
   gtgataagct tattcatttt ggtgggattt ataggagagt tccaaagttt ttcaagtgcc 180
   tcctctccag tcaactgcca gtgggacttc tatgcccctt ggtcagaatg caatggctgt 240
   accaagactc agactcgcag gcggtcagtt gctgtgtatg ggcagtatgg aggccagcct 300
   tgtgttggaa atgcttttga aacacagtcc tgtgaaccta caagaggatg tccaacagag 360
   gagggatgtg gagagcgttt caggtgcttt tcaggtcagt gcatcagcaa atcattggtt 420
   tgcaatgggg attctgactg tgatgaagac agtgctgatg aagacagatg tgaggactca 480
   gaaaggagac cttcctgtga tatcgataaa cctcctccta acatagaact tactggaaat 540
   ggttacaatg aactcactgg ccagtttagg aacagagtca tcaataccaa aagttttggt 600
   ggtcaatgta gaaaggtgtt tagtggggat ggaaaagatt tctacaggct gagtggaaat 660
   gtcctgtcct atacattcca ggtgaaaata aataatgatt ttaattatga attttacaat 720
   agtacttggt cttatgtaaa acatacgtcg acagaacaca catcatctag tcggaagcgc 780
   tcctttttta gatcttcatc atcttcttca cgcagttata cttcacatac caatgaaatc 840
   cataaaggaa agagttacca actgctggtt gttgagaaca ctgttgaagt ggctcagttc 900
   attaataaca atccagaatt tttacaactt gctgagccat tctggaagga gctttcccac 960
   ctcccctctc tgtatgacta cagtgcctac cgaagattaa tcgaccagta cgggacacat 1020
   tatctgcaat ctgggtcgtt aggaggagaa tacagagttc tattttatgt ggactcagaa 1080
   aaattaaaac aaaatgattt taattcagtc gaagaaaaga aatgtaaatc ctcaggttgg 1140
   cattttgtcg ttaaattttc aagtcatgga tgcaaggaac tggaaaacgc tttaaaagct 1200
   gcttcaggaa cccagaacaa tgtattgcga ggagaaccgt tcatcagagg gggaggtgca 1260
   ggcttcatat ctggccttag ttacctagag ctggacaatc ctgctggaaa caaaaggcga 1320
   tattctgcct gggcagaatc tgtgactaat cttcctcaag tcataaaaca aaagctgaca 1380
   cctttatatg agctggtaaa ggaagtacct tgtgcctctg tgaaaaaact atacctgaaa 1440
   tgggctcttg aagagtatct ggatgaattt gacccctgtc attgccggcc ttgtcaaaat 1500
   ggtggtttgg ctactgttga ggggacccat tgtctgtgcc attgcaaacc gtacacattt 1560
   ggtgcggcgt gtgagcaagg agtcctcgta gggaatcaag caggaggggt tgatggaggt 1620
   tggagttgct ggtcctcttg gagcccctgt gtccaaggga agaaaacaag aagccgtgaa 1680
   tgcaataacc cacctcccag tgggggtggg agatcctgcg ttggagaaac gacagaaagc 1740
   acacaatgcg aagatgagga gctggagcac ttgaggttgc ttgaaccaca ttgctttcct 1800
   ttgtctttgg ttccaacaga attctgtcca tcacctcctg ccttgaaaga tggatttgtt 1860
   caagatgaag gtacaatgtt tcctgtgggg aaaaatgtag tgtacacttg caatgaagga 1920
   tactctctta ttggaaaccc agtggccaga tgtggagaag atttacggtg gcttgttggg 1980
   gaaatgcatt gtcagaaaat tgcctgtgtt ctacctgtac tgatggatgg catacagagt 2040
   cacccccaaa aacctttcta cacagttggt gagaaggtga ctgtttcctg ttcaggtggc 2100
   atgtccttag aaggtccttc agcatttctc tgtggctcca gccttaagtg gagtcctgag 2160
   atgaagaatg cccgctgtgt acaaaaagaa aatccgttaa cacaggcagt gcctaaatgt 2220
   cagcgctggg agaaactgca gaattcaaga tgtgtttgta aaatgcccta cgaatgtgga 2280
   ccttccttgg atgtatgtgc tcaagatgag agaagcaaaa ggatactgcc tctgacagtt 2340
   tgcaagatgc atgttctcca ctgtcagggt agaaattaca cccttactgg tagggacagc 2400
   tgtactctgc ctgcctcagc tgagaaagct tgtggtgcct gcccactgtg gggaaaatgt 2460
   gatgctgaga gcagcaaatg tgtctgccga gaagcatcgg agtgcgagga agaagggttt 2520
   agcatttgtg tggaagtgaa cggcaaggag cagacgatgt ctgagtgtga ggcgggcgct 2580
   ctgagatgca gagggcagag catctctgtc accagcataa ggccttgtgc tgcggaaacc 2640
   cagtaggctc ctggaggccc tggtcagctt gcttggaatc cagcaggcag ctggggctga 2700
   gtgaaaacat ctgcacaact gggcactgga cagcttttcc ttcttctcca gtgtctacct 2760
   tcctcctcaa ctcccagcca tctgtataaa cacaatcctt tgttctccca aatctgaatc 2820
   gaattactct tttgcctcct ttttaatgtc agtaaggata tgagcctttg cacaggctgg 2880
   ctgcgtgttc ttgaaatagg tgttaccttc tctgggcctt ggttttttaa aatctgtaaa 2940
   attagaggat tgcactagag aaacttgaat gctccattca ggcctatcat tttattaagt 3000
   atgattgaca cagcccatgg gccagaacac actctacaaa atgactagga taacagaaag 3060
   aacgtgatct cctgattaga gagggtggtt ttcctcaatg gaaccaaata taaagaggac 3120
   ttgaacaaaa atgacagata caaactattt ctatcctgag tagtaatctc acacttcatc 3180
   ctatagagtc aaccaccaca gataggaatt ccttattctt tttttaattt ttttaagaca 3240
   gagtctcact ttgttgccca ggctggagcg cagtggggtg atctcatctc cctgcaacct 3300
   ccgcctcctg ggttcaagcg attcttgtgc ctcagcttcc caagcagctg ggattacagg 3360
   tgcccgccac cacgcccagc taatttttgc atttttagta gagatggggt ttcaccatgt 3420
   tggccacgct cgtctccaac tcctgacctc aggtaatccg cctgccttgg cctcccaaag 3480
   tgctgggatt acagacatga accaccacgc ctggctggaa tacttactct tgtcgggaga 3540
   ttgaaccact aaaatgttag agcagaattc attatgctgt ggtcacaggg gtgtcttgtc 3600
   tgagaacaaa tacaattcag tcttctcttt ggggttttag tatgtgtcaa acataggact 3660
   ggaagtttgc ccctgttctt ttttcttttg aaagaacatc agttcatgcc tgaggcatga 3720
   gtgactgtgc atttgagaat agttttccct attctgtgga tacagtccca gagttttcag   3780
   ggagtacaca ggtagattag tttgaagcat tgacctttta tttattcctt atttctcttt   3840
   catcaaaaca aaacagcagc tgtgggagga gaaatgagag ggcttaaatg aaatttaaaa   3900
   taagctatat tatacaaata ctatctctgt attgttctga ccctggtaaa tatatttcaa   3960
   aacttcagat gacaaggatt agaacactca ttaaagatgc tattcttcag aaaaaaaaaa   4020
   aaaaaaaaaa aaaa                                                      4034
<210> 10
   <211> 2397
   <212> DNA
   <213> Homo sapiens
<400> 10
   tttttttttt catcctactt tgttttattg ggcgttgatt gttacaggtc ccagcctgta      60
   gacatctttt actccaattt cctgaataga tagctttatt ccttcaaggt aatatagtgc    120
   ggtggcttct ggctgagatg tttgctgttg ttttcttcat cttgtctttg atgacttgtc    180
   agcctggggt aactgcacag gagaaggtga accagagagt aagacgggca gctacacccg    240
   cagcagttac ctgccagctg agcaactggt cagagtggac agattgcttt ccgtgccagg    300
   acaaaaagta ccgacaccgg agcctcttgc agccaaacaa gtttggggga accatctgca    360
   gtggtgacat ctgggatcaa gccagctgct ccagttctac aacttgtgta aggcaagcac    420
   agtgtggaca ggatttccag tgtaaggaga caggtcgctg cctgaaacgc caccttgtgt    480
   gtaatggaga ccaggactgc cttgatggct ctgatgagga cgactgtgaa gatgtcaggg    540
   ccattgacga agactgcagc cagtatgaac caattccagg atcacagaag gcagccttgg    600
   ggtacaatat cctgacccag gaagatgctc agagtgtgta cgatgccagt tattatgggg    660
   gccagtgtga gacggtatac aatggggaat ggagggagct tcgatatgac tccacctgtg    720
   aacgtctcta ctatggagat gatgagaaat actttcggaa accctacaac tttctgaagt    780
   accactttga agccctggca gatactggaa tctcctcaga gttttatgat aatgcaaatg    840
   accttctttc caaagttaaa aaagacaagt ctgactcatt tggagtgacc atcggcatag    900
   gcccagccgg cagcccttta ttggtgggtg taggtgtatc ccactcacaa gacacttcat    960
   tcttgaacga attaaacaag tataatgaga agaaattcat tttcacaaga atcttcacaa   1020
   aggtgcagac tgcacatttt aagatgagga aggatgacat tatgctggat gaaggaatgc   1080
   tgcagtcatt aatggagctt ccagatcagt acaattatgg catgtatgcc aagttcatca   1140
   atgactatgg cacccattac atcacatctg gatccatggg tggcatttat gaatatatcc   1200
   tggtgattga caaagcaaaa atggaatccc ttggtattac cagcagagat atcacgacat   1260
   gttttggagg ctccttgggc attcaatatg aagacaaaat aaatgttggt ggaggtttat   1320
   caggagacca ttgtaaaaaa tttggaggtg gcaaaactga aagggccagg aaggccatgg   1380
   ctgtggaaga cattatttct cgggtgcgag gtggcagttc tggctggagc ggtggcttgg   1440
   cacagaacag gagcaccatt acataccgtt cctgggggag gtcattaaag tataatcctg   1500
   ttgttatcga ttttgagatg cagcctatcc acgaggtgct gcggcacaca agcctggggc 1560
   ctctggaggc caagcgccag aacctgcgcc gcgccttgga ccagtatctg atggaattca 1620
   atgcctgccg atgtgggcct tgcttcaaca atggggtgcc catcctcgag ggcaccagct 1680
   gcaggtgcca gtgccgcctg ggtagcttgg gtgctgcctg tgagcaaaca cagacagaag 1740
   gagccaaagc agatgggagc tggagttgct ggagctcctg gtctgtatgc agagcaggca 1800
   tccaggaaag gagaagagag tgtgacaatc cagcacctca gaatggaggg gcctcgtgtc 1860
   cagggcggaa agtacagacg caggcttgct gagggcctct ggacacaggc tggaccagat 1920
   gctgtggatg tcgacccctg cactgactat tggataaaga cttctttcaa ctaagagaag 1980
   atgcaaatca gcacactttt ttctttgttc tgccagcttc caggcctaag actaggtttt 2040
   gctgtctaca gccaactatt ctattagtta caaaactcaa tcattttatt cagcaactgg 2100
   atgttgactg ttaactagaa gctctgtcct acttacagca ctttggatca tcaaaaaaat 2160
   aaagtaaaat agaaaactga gaaaactcaa tccatgacca gggagaactt acaggatgtt 2220
   agagacaaaa caagcagaca cctgaaacaa tcaacgccca ataaaacaaa gtaggatgaa 2280
   aattctctta gttctttgat aacaatttgt tcactcatag aaacattatt aattggtagg 2340
   gtaagcagac actctgaaac aatgagaaaa atactaaaaa ttgacttgag ttatttc 2397
<210> 11
   <211> 1173
   <212> DNA
   <213> Homo sapiens
<400> 11
   gtgtctcagc cacagcggct tcaccatgca cagctgggag cgcctggcag ttctggtcct     60
   cctaggagcg gccgcctgcg cggcgccgcc ccgtggtcgg atcctgggcg gcagagaggc    120
   cgaggcgcac gcgcggccct acatggcgtc ggtgcagctg aacggcgcgc acctgtgcgg    180
   cggcgtcctg gtggcggagc agtgggtgct gagcgcggcg cactgcctgg aggacgcggc    240
   cgacgggaag gtgcaggttc tcctgggcgc gcactccctg tcgcagccgg agccctccaa    300
   gcgcctgtac gacgtgctcc gcgcagtgcc ccacccggac agccagcccg acaccatcga    360
   ccacgacctc ctgctgctac agctgtcgga gaaggccaca ctgggccctg ctgtgcgccc    420
   cctgccctgg cagcgcgtgg accgcgacgt ggcaccggga actctctgcg acgtggccgg    480
   ctggggcata gtcaaccacg cgggccgccg cccggacagc ctgcagcacg tgctcttgcc    540
   agtgctggac cgcgccacct gcaaccggcg cacgcaccac gacggcgcca tcaccgagcg    600
   cttgatgtgc gcggagagca atcgccggga cagctgcaag ggtgactccg ggggcccgct     660
   ggtgtgcggg ggcgtgctcg agggcgtggt cacctcgggc tcgcgcgttt gcggcaaccg    720
   caagaagccc gggatctaca cccgcgtggc gagctatgcg gcctggatcg acagcgtcct    780
   ggcctagggt gccggggcct gaaggtcagg gtcacccaag caacaaagtc ccgagcaatg    840
   aagtcatcca ctcctgcatc tggttggtct ttattgagca cctactatat gcagaagggg    900
   aggccgaggt gggaggatca ttggatctca ggagttcgag atcagcatgg gccacgtagc    960
   gcgactccat ctctacaaat aaataaaaaa ttagctgggc aattggcggg catggaggtg   1020
   ggtgcttgta gttccagcta ctcaggaggc tgaggtggga ggatgacttg aacgcaggag   1080
   gctgaggctg cagtgagttg tgattgcacc actgccctcc agcctgggca acagagtgaa   1140
   accttgtctc tctctacaaa aaaaaaaaaa aaa                                1173
<210> 12
   <211> 857
   <212> DNA
   <213> Homo sapiens
<400> 12
   ctgggacttt ggtggtgcta cccttggcct cccagagtcc tgccaccctg ctgccgccac    60
   catgctgccc cctgggactg cgaccctctt gactctgctc ctggcagctg gctcgctggg   120
   ccagaagcct cagaggccac gccggcccgc atcccccatc agcaccatcc agcccaaggc   180
   caattttgat gctcagcagt ttgcagggac ctggctcctt gtggctgtgg gctccgcttg   240
   ccgtttcctg caggagcagg gccaccgggc cgaggccacc acactgcatg tggctcccca   300
   gggcacagcc atggctgtca gtaccttccg aaagctggat gggatctgct ggcaggtgcg   360
   ccagctctat ggagacacag gggtcctcgg ccgcttcctg cttcaagccc gaggcgcccg   420
   aggggctgtg cacgtggttg tcgctgagac cgactaccag agtttcgctg tcctgtacct   480
   ggagcgggcg gggcagctgt cagtgaagct ctacgcccgc tcgctccctg tgagcgactc   540
   ggtcctgagt gggtttgagc agcgggtcca ggaggcccac ctgactgagg accagatctt   600
   ctacttcccc aagtacggct tctgcgaggc tgcagaccag ttccacgtcc tggacgaagt   660
   gaggaggtga ggccggcaca cagctccagt gctgagaagt cagtgccccg agagacgacc   720
   ccaccagtgg ggtgcccgct gcctgtcctc cgtgaaacca gcctcagatc agggccctgc   780
   cacccagggc aggggatctt ctgccggctg ccccagagga cagtgggtgg agtggtacct   840
   acttattaaa tgtctcc                                                  857
<210> 13
   <211> 2094
   <212> DNA
   <213> Homo sapiens
<400> 13
   gcttgttccc tgtcctctgg ccctttgcaa ataaatgcct taccagacct gccctgccac    60
   cccactcgca gccacccagc aagagcagca tgtcagcctg ccggagcttt gcagttgcaa   120
   tctgcatttt agaaataagc atcctcacag cacagtacac gaccagttat gacccagagc   180
   taacagaaag cagtggctct gcatcacaca tagactgcag aatgagcccc tggagtgaat   240
   ggtcacaatg cgatccttgt ctcagacaaa tgtttcgttc aagaagcatt gaggtctttg   300
   gacaatttaa tgggaaaaga tgcaccgacg ctgtgggaga cagacgacag tgtgtgccca   360
   cagagccctg tgaggatgct gaggatgact gcggaaatga ctttcaatgc agtacaggca   420
   gatgcataaa gatgcgactt cggtgtaatg gtgacaatga ctgcggagac ttttcagatg   480
   aggatgattg tgaaagtgag ccccgtcccc cctgcagaga cagagtggta gaagagtctg   540
   agctggcacg aacagcaggc tatgggatca acattttagg gatggatccc ctaagcacac 600
   cttttgacaa tgagttctac aatggactct gtaaccggga tcgggatgga aacactctga 660
   catactaccg aagaccttgg aacgtggctt ctttgatcta tgaaaccaaa ggcgagaaaa 720
   atttcagaac cgaacattac gaagaacaaa ttgaagcatt taaaagtatc atccaagaga 780
   agacatcaaa ttttaatgca gctatatctc taaaatttac acccactgaa acaaataaag 840
   ctgaacaatg ttgtgaggaa acagcctcct caatttcttt acatggcaag ggtagttttc 900
   ggttttcata ttccaaaaat gaaacttacc aactattttt gtcatattct tcaaagaagg 960
   aaaaaatgtt tctgcatgtg aaaggagaaa ttcatctggg aagatttgta atgagaaatc 1020
   gcgatgttgt gctcacaaca acttttgtgg atgatataaa agctttgcca actacctatg 1080
   aaaagggaga atattttgcc tttttggaaa cctatggaac tcactacagt agctctgggt 1140
   ctctaggagg actctatgaa ctaatatatg ttttggataa agcttccatg aagcggaaag 1200
   gtgttgaact aaaagacata aagagatgcc ttgggtatca tctggatgta tctctggctt 1260
   tctctgaaat ctctgttgga gctgaattta ataaagatga ttgtgtaaag aggggagagg 1320
   gtagagctgt aaacatcacc agtgaaaacc tcatagatga tgttgtttca ctcataagag 1380
   gtggaaccag aaaatatgca tttgaactga aagaaaagct tctccgagga accgtgattg 1440
   atgtgactga ctttgtcaac tgggcctctt ccataaatga tgctcctgtt ctcattagtc 1500
   aaaaactgtc tcctatatat aatctggttc cagtgaaaat gaaaaatgca cacctaaaga 1560
   aacaaaactt ggaaagagcc attgaagact atatcaatga atttagtgta agaaaatgcc 1620
   acacatgcca aaatggaggt acagtgattc taatggatgg aaagtgtttg tgtgcctgcc 1680
   cattcaaatt tgagggaatt gcctgtgaaa tcagtaaaca aaaaatttct gaaggattgc 1740
   cagccctaga gttccccaat gaaaaataga gctgttggct tctctgagct ccagtggaag 1800
   aagaaaacac tagtaccttc agatcctacc cctgaagata atcttagctg ccaagtaaat 1860
   agcaacatgc ttcatgaaaa tcctaccaac ctctgaagtc tcttctctct taggtctata 1920
   attttttttt taatttttct tccttaaact cctgtgatgt ttccattttt tgttccctaa 1980
   tgagaagtca acagtgaaat acgccagaac tgctttatcc cacggaaaat gccaatctct 2040
   tctaaaaaaa aacaaaatta aactaaaaaa aaaaaaaaaa aaaaaaaaaa aaaa 2094
<210> 14
   <211> 2574
   <212> DNA
   <213> Homo sapiens
<400> 14
   ttggatgttc cgggaaagtg atgtgggtag gacaggcggg gcgagccgca ggtgccagaa 60
   cacagattgt ataaaaggct gggggctggt ggggagcagg ggaagggaat gtgaccaggt 120
   ctaggtctgg agtttcagct tggacactga gccaagcaga caagcaaagc aagccaggac 180
   acaccatcct gccccaggcc cagcttctct cctgccttcc aacgccatgg ggagcaatct 240
   cagcccccaa ctctgcctga tgccctttat cttgggcctc ttgtctggag gtgtgaccac 300
   cactccatgg tctttggccc ggccccaggg atcctgctct ctggaggggg tagagatcaa 360
   aggcggctcc ttccgacttc tccaagaggg ccaggcactg gagtacgtgt gtccttctgg 420
   cttctacccg taccctgtgc agacacgtac ctgcagatct acggggtcct ggagcaccct 480
   gaagactcaa gaccaaaaga ctgtcaggaa ggcagagtgc agagcaatcc actgtccaag 540
   accacacgac ttcgagaacg gggaatactg gccccggtct ccctactaca atgtgagtga 600
   tgagatctct ttccactgct atgacggtta cactctccgg ggctctgcca atcgcacctg 660
   ccaagtgaat ggccgatgga gtgggcagac agcgatctgt gacaacggag cggggtactg 720
   ctccaacccg ggcatcccca ttggcacaag gaaggtgggc agccagtacc gccttgaaga 780
   cagcgtcacc taccactgca gccgggggct taccctgcgt ggctcccagc ggcgaacgtg 840
   tcaggaaggt ggctcttgga gcgggacgga gccttcctgc caagactcct tcatgtacga 900
   cacccctcaa gaggtggccg aagctttcct gtcttccctg acagagacca tagaaggagt 960
   cgatgctgag gatgggcacg gcccagggga acaacagaag cggaagatcg tcctggaccc 1020
   ttcaggctcc atgaacatct acctggtgct agatggatca gacagcattg gggccagcaa 1080
   cttcacagga gccaaaaagt gtctagtcaa cttaattgag aaggtggcaa gttatggtgt 1140
   gaagccaaga tatggtctag tgacatatgc cacatacccc aaaatttggg tcaaagtgtc 1200
   tgaagcagac agcagtaatg cagactgggt cacgaagcag ctcaatgaaa tcaattatga 1260
   agaccacaag ttgaagtcag ggactaacac caagaaggcc ctccaggcag tgtacagcat 1320
   gatgagctgg ccagatgacg tccctcctga aggctggaac cgcacccgcc atgtcatcat 1380
   cctcatgact gatggattgc acaacatggg cggggaccca attactgtca ttgatgagat 1440
   ccgggacttg ctatacattg gcaaggatcg caaaaaccca agggaggatt atctggatgt 1500
   ctatgtgttt ggggtcgggc ctttggtgaa ccaagtgaac atcaatgctt tggcttccaa 1560
   gaaagacaat gagcaacatg tgttcaaagt caaggatatg gaaaacctgg aagatgtttt 1620
   ctaccaaatg atcgatgaaa gccagtctct gagtctctgt ggcatggttt gggaacacag 1680
   gaagggtacc gattaccaca agcaaccatg gcaggccaag atctcagtca ttcgcccttc 1740
   aaagggacac gagagctgta tgggggctgt ggtgtctgag tactttgtgc tgacagcagc 1800
   acattgtttc actgtggatg acaaggaaca ctcaatcaag gtcagcgtag gaggggagaa 1860
   gcgggacctg gagatagaag tagtcctatt tcaccccaac tacaacatta atgggaaaaa 1920
   agaagcagga attcctgaat tttatgacta tgacgttgcc ctgatcaagc tcaagaataa 1980
   gctgaaatat ggccagacta tcaggcccat ttgtctcccc tgcaccgagg gaacaactcg 2040
   agctttgagg cttcctccaa ctaccacttg ccagcaacaa aaggaagagc tgctccctgc 2100
   acaggatatc aaagctctgt ttgtgtctga ggaggagaaa aagctgactc ggaaggaggt 2160
   ctacatcaag aatggggata agaaaggcag ctgtgagaga gatgctcaat atgccccagg 2220
   ctatgacaaa gtcaaggaca tctcagaggt ggtcacccct cggttccttt gtactggagg 2280
   agtgagtccc tatgctgacc ccaatacttg cagaggtgat tctggcggcc ccttgatagt 2340
   tcacaagaga agtcgtttca ttcaagttgg tgtaatcagc tggggagtag tggatgtctg   2400
   caaaaaccag aagcggcaaa agcaggtacc tgctcacgcc cgagactttc acatcaacct   2460
   ctttcaagtg ctgccctggc tgaaggagaa actccaagat gaggatttgg gttttctata   2520
   aggggtttcc tgctggacag gggcgtggga ttgaattaaa acagctgcga caac         2574
<210> 15
   <211> 1173
   <212> DNA
   <213> Homo sapiens
<400> 15
   gtgtctcagc cacagcggct tcaccatgca cagctgggag cgcctggcag ttctggtcct     60
   cctaggagcg gccgcctgcg cggcgccgcc ccgtggtcgg atcctgggcg gcagagaggc    120
   cgaggcgcac gcgcggccct acatggcgtc ggtgcagctg aacggcgcgc acctgtgcgg    180
   cggcgtcctg gtggcggagc agtgggtgct gagcgcggcg cactgcctgg aggacgcggc    240
   cgacgggaag gtgcaggttc tcctgggcgc gcactccctg tcgcagccgg agccctccaa    300
   gcgcctgtac gacgtgctcc gcgcagtgcc ccacccggac agccagcccg acaccatcga    360
   ccacgacctc ctgctgctac agctgtcgga gaaggccaca ctgggccctg ctgtgcgccc    420
   cctgccctgg cagcgcgtgg accgcgacgt ggcaccggga actctctgcg acgtggccgg    480
   ctggggcata gtcaaccacg cgggccgccg cccggacagc ctgcagcacg tgctcttgcc    540
   agtgctggac cgcgccacct gcaaccggcg cacgcaccac gacggcgcca tcaccgagcg    600
   cttgatgtgc gcggagagca atcgccggga cagctgcaag ggtgactccg ggggcccgct     660
   ggtgtgcggg ggcgtgctcg agggcgtggt cacctcgggc tcgcgcgttt gcggcaaccg    720
   caagaagccc gggatctaca cccgcgtggc gagctatgcg gcctggatcg acagcgtcct    780
   ggcctagggt gccggggcct gaaggtcagg gtcacccaag caacaaagtc ccgagcaatg    840
   aagtcatcca ctcctgcatc tggttggtct ttattgagca cctactatat gcagaagggg    900
   aggccgaggt gggaggatca ttggatctca ggagttcgag atcagcatgg gccacgtagc    960
   gcgactccat ctctacaaat aaataaaaaa ttagctgggc aattggcggg catggaggtg   1020
   ggtgcttgta gttccagcta ctcaggaggc tgaggtggga ggatgacttg aacgcaggag   1080
   gctgaggctg cagtgagttg tgattgcacc actgccctcc agcctgggca acagagtgaa   1140
   accttgtctc tctctacaaa aaaaaaaaaa aaa                                1173
<210> 16
   <211> 1043
   <212> DNA
   <213> Homo sapiens
<400> 16
   tgttaatgaa agcagattca aagcaacacc accaccactg aagtattttt agttatataa    60
   gattggaact accaagcatg tggctcctgg tcagtgtaat tctaatctca cggatatcct   120
   ctgttggggg agaagcaatg ttctgtgatt ttccaaaaat aaaccatgga attctatatg   180
   atgaagaaaa atataagcca ttttcccaag ttcctacagg ggaagttttc tattactcct   240
   gtgaatataa ttttgtgtct ccttcaaaat ccttttggac tcgcataacg tgcgcagaag   300
   aaggatggtc accaacacca aagtgtctca gactgtgttt ctttcctttt gtggaaaatg   360
   gtcattctga atcttcagga caaacacatc tggaaggtga tactgtacaa attatttgca   420
   acacaggata cagacttcaa aacaatgaga acaacatttc atgtgtagaa cggggctggt   480
   ccactcctcc caaatgcagg tccactattt ctgcagaaaa atgtgggccc cctccaccta   540
   ttgacaatgg agacattact tcattcctgt tgtcagtata tgctccaggt tcatcagttg   600
   agtaccagtg ccagaacttg tatcaacttg agggtaacaa tcaaataaca tgtagaaacg   660
   gacaatggtc agaaccacca aaatgcttag atccatgtgt aatatcacaa gaaattatgg   720
   aaaaatataa cataaaatta aagtggacaa accaacaaaa gctttattca agaacaggtg   780
   acatagttga atttgtttgt aaatctggat atcatccaac aaaatctcat tcatttcgag   840
   caatgtgtca gaatgggaaa ctggtatatc ccagttgtga agaaaaatag aatcaatggc   900
   attactatta gtaaaatgca cacctttttc tgaatttact attatatttg ttttcaattt    960
   catttttcaa gtactgtttt actcattttt attcataaat aaagttttgt gttgatttgt  1020
   gaaaatgcaa ttacaagagc caa                                          1043
<210> 17
   <211> 1292
   <212> DNA
   <213> Homo sapiens
<400> 17
   tgaaagattt caaaccccaa acagtgcaac tgaaactttt gcattactat actactgaga      60
   atatctaaca tgttgttact aatcaatgtc attctgacct tgtgggtttc ctgtgctaat     120
   ggacaagaag tgaaaccttg tgattttcca gaaattcaac atggaggtct atattataag     180
   agtttgcgta gactatactt tccagcagct gcaggacaat cttattccta ttactgtgat     240
   caaaattttg tgactccttc aggaagttac tgggattaca ttcattgcac acaagatggt     300
   tggtcaccaa cggtcccatg cctcagaaca tgctcaaaat cagatataga aattgaaaat     360
   ggattcattt ctgaatcttc ctctatttat attttaaata aagaaataca atataaatgt     420
   aaaccaggat atgcaacagc agatggaaat tcttcaggtt caattacatg tttgcaaaat     480
   ggatggtcag cacaaccaat ttgcattaaa ttttgtgata tgcctgtttt tgagaattcc     540
   agagccaaga gtaatggcat gcggtttaag ctccatgaca cattggacta cgaatgctac     600
   gatggatatg aaatcagtta tggaaacacc acaggttcca tagtgtgtgg tgaagatggg     660
   tggtcccatt tcccaacatg ttataattct tcagaaaagt gtgggcctcc tccacctatt     720
   agcaatggtg ataccacctc ctttctacta aaagtgtatg tgccacagtc aagagtcgag     780
   taccaatgcc agtcctacta tgaacttcag ggttctaatt atgtaacatg tagtaatgga     840
   gagtggtcgg aaccaccaag atgcatacat ccatgtataa taactgaaga aaacatgaat     900
   aaaaataaca tacagttaaa aggaaaaagt gacataaaat attatgcaaa aacaggggat     960
   accattgaat ttatgtgtaa attgggatat aatgcgaata catcagttct atcatttcaa    1020
   gcagtgtgta gggaaggcat agtggaatac cccagatgcg aataaggcag cattgttacc    1080
   ctaaatgtat gtccaacttc cacttctcac tcttatggtc tcaaagcttg caaagatagc    1140
   ttctgatatt gttgtaattt ctactttatt tcaaagaaaa ttaatataat agtttcaatt    1200
   tgcaacttaa tatgttctca aaaatatgtt aaaacaaact aaattattgc ttatgcttgt    1260
   actaaaataa taaaaactac ccttatattg ga                                  1292
<210> 18
   <211> 2823
   <212> DNA
   <213> Homo sapiens
<400> 18
   ggcaggtgct tgttactgtt aatgaaagca gatttaaagc aacaccacca tcactggagt     60
   atttttagtt atatacgatt gagactacca agcatgttgc tcttattcag tgtaatccta    120
   atctcatggg tatccactgt tgggggagaa ggaacacttt gtgattttcc aaaaatacac    180
   catggatttc tgtatgatga agaagattat aacccttttt cccaagttcc tacaggggaa    240
   gttttctatt actcctgtga atataatttt gtgtctcctt caaaatcctt ttggactcgc    300
   ataacatgca cagaagaagg atggtcacca acaccgaagt gtctcagaat gtgttccttt    360
   ccttttgtga aaaatggtca ttctgaatct tcaggactaa tacatctgga aggtgatact    420
   gtacaaatta tttgcaacac aggatacagc cttcaaaaca atgagaaaaa catttcgtgt    480
   gtagaacggg gctggtccac tcctcccata tgcagcttca ctaaaggaga atgtcatgtt    540
   ccaattttag aagccaatgt agatgctcag ccaaaaaaag aaagctacaa agttggagac    600
   gtgttgaaat tctcctgcag aaaaaatctt ataagagttg gatcagactc agttcaatgt    660
   taccaatttg ggtggtcacc taactttcca acatgcaaag gacaagtacg atcatgtggt    720
   ccacctcctc aactctccaa tggtgaagtt aaggagataa gaaaagagga atatggacac    780
   aatgaagtag tggaatatga ttgcaatcct aattttataa taaacgggcc taagaaaata    840
   caatgtgtgg atggagaatg gacaacttta cccacttgtg ttgaacaagt gaaaacatgt    900
   ggatacatac ctgaactcga gtacggttat gttcagccgt ctgtccctcc ctatcaacat    960
   ggagtttcag tcgaggtgaa ttgcagaaat gaatatgcaa tgattggaaa taacatgatt   1020
   acctgtatta atggaatatg gacagagctt cctatgtgtg ttgcaacaca ccaacttaag   1080
   aggtgcaaaa tagcaggagt taatataaaa acattactca agctatctgg gaaagaattt   1140
   aatcataatt ctagaatacg ttacagatgt tcagacatct tcagatacag gcactcagtc   1200
   tgtataaacg ggaaatggaa tcctgaagta gactgcacag aaaaaaggga acaattctgc   1260
   ccaccgccac ctcagatacc taatgctcag aatatgacaa ccacagtgaa ttatcaggat   1320
   ggagaaaaag tagctgttct ctgtaaagaa aactatctac ttccagaagc aaaagaaatt   1380
   gtatgtaaag atggacgatg gcaatcatta ccacgctgtg ttgagtctac tgcatattgt   1440
   gggccccctc catctattaa caatggagat accacctcat tcccattatc agtatatcct   1500
   ccagggtcaa cagtgacgta ccgttgccag tccttctata aactccaggg ctctgtaact   1560
   gtaacatgca gaaataaaca gtggtcagaa ccaccaagat gcctagatcc atgtgtggta 1620
   tctgaagaaa acatgaacaa aaataacata cagttaaaat ggagaaacga tggaaaactc 1680
   tatgcaaaaa caggggatgc tgttgaattc cagtgtaaat tcccacataa agcgatgata 1740
   tcatcaccac catttcgagc aatctgtcag gaagggaaat ttgaatatcc tatatgtgaa 1800
   tgaagcaagc ataattttcc tgaatatatt cttcaaacat ccatctacgc taaaagtagc 1860
   cattatgtag ccaattctgt agttacttct tttattcttt caggtgttgt ttaactcagt 1920
   tttatttaga actctggatt tttagagctt tagaaatttg taagctgaga gaacaatgtt 1980
   tcacttaata ggagggtgtc ttagtccata ttacattgtt ataacagagt atcacagact 2040
   ggataacttc taaccaatag tttatttgtt tcataaatct aaaagctgag aagtccaaga 2100
   tggtggggct gcctctggtg agggtcttct cgaagcatca taatatgctg gaaggcatca 2160
   caacatggtg gaagggatca cgtggcaaaa gagcatgtac atgggagtga gagaaaaaga 2220
   gagagagaga cagagtggcg ggggccgggg aggagcgcaa actcatcctt tataaagaca 2280
   ccactcctga gataacaatc caatcccatg ataatgacat taatccattc aagaagatag 2340
   agctctcgtg acttaatcac cttctaaaga tctcacctga caacactgtt gcattggcag 2400
   ttaagtttcc acgtaaactt tcggggacac attcaaacca caggagaaac tcaaattgtt 2460
   cctgggcaaa tcacaacatg gggaatttta ttcataaatg tccacagaaa cagtaaatgt 2520
   tctcgcttca gaacttaatt catctaatcc ctcctgtttg tctcaaatta taggataact 2580
   ttgaaacttt ctgaattaac gttatttaaa aggaaatgta gatgttattt tagtctctat 2640
   cttcaggtta ttatcactta aaaacctgcg aaagctgtca acttttgtgg ttgtagcaag 2700
   tattaataaa tatttataaa tcctctaatg taagtctagc tacctatcca atactaaata 2760
   ccccttaaag tattaaatgc actatctgct gtaaacggaa aaaaaaaaaa aaaaaaaaaa 2820
   aaa 2823
<210> 19
   <211> 2160
   <212> DNA
   <213> Homo sapiens
<400> 19
   ctttaatggg aaggccattt tttggtttca gttacggtaa atctctctgg atttcagcca    60
   aattctttca gagttcaaaa gtacaaagct ctttaggagg tttgttcttt tgaaacattt   120
   tttcagaggt taaaaattaa atttcaaaag aatacctgga gtggaaaaga gttctcagca   180
   gagacaaaga ccccgaacac ctccaacatg aagcttcttc atgttttcct gttatttctg   240
   tgcttccact taaggttttg caaggtcact tatacatctc aagaggatct ggtggagaaa   300
   aagtgcttag caaaaaaata tactcacctc tcctgcgata aagtcttctg ccagccatgg   360
   cagagatgca ttgagggcac ctgtgtttgt aaactaccgt atcagtgccc aaagaatggc   420
   actgcagtgt gtgcaactaa caggagaagc ttcccaacat actgtcaaca aaagagtttg   480
   gaatgtcttc atccagggac aaagttttta aataacggaa catgcacagc cgaaggaaag   540
   tttagtgttt ccttgaagca tggaaataca gattcagagg gaatagttga agtaaaactt   600
   gtggaccaag ataagacaat gttcatatgc aaaagcagct ggagcatgag ggaagccaac   660
   gtggcctgcc ttgaccttgg gtttcaacaa ggtgctgata ctcaaagaag gtttaagttg   720
   tctgatctct ctataaattc cactgaatgt ctacatgtgc attgccgagg attagagacc   780
   agtttggctg aatgtacttt tactaagaga agaactatgg gttaccagga tttcgctgat   840
   gtggtttgtt atacacagaa agcagattct ccaatggatg acttctttca gtgtgtgaat   900
   gggaaataca tttctcagat gaaagcctgt gatggtatca atgattgtgg agaccaaagt   960
   gatgaactgt gttgtaaagc atgccaaggc aaaggcttcc attgcaaatc gggtgtttgc  1020
   attccaagcc agtatcaatg caatggtgag gtggactgca ttacagggga agatgaagtt  1080
   ggctgtgcag gctttgcatc tgtggctcaa gaagaaacag aaattttgac tgctgacatg  1140
   gatgcagaaa gaagacggat aaaatcatta ttacctaaac tatcttgtgg agttaaaaac  1200
   agaatgcaca ttcgaaggaa acgaattgtg ggaggaaagc gagcacaact gggagacctc  1260
   ccatggcagg tggcaattaa ggatgccagt ggaatcacct gtgggggaat ttatattggt  1320
   ggctgttgga ttctgactgc tgcacattgt ctcagagcca gtaaaactca tcgttaccaa  1380
   atatggacaa cagtagtaga ctggatacac cccgacctta aacgtatagt aattgaatac  1440
   gtggatagaa ttattttcca tgaaaactac aatgcaggca cttaccaaaa tgacatcgct  1500
   ttgattgaaa tgaaaaaaga cggaaacaaa aaagattgtg agctgcctcg ttccatccct  1560
   gcctgtgtcc cctggtctcc ttacctattc caacctaatg atacatgcat cgtttctggc  1620
   tggggacgag aaaaagataa cgaaagagtc ttttcacttc agtggggtga agttaaacta  1680
   ataagcaact gctctaagtt ttacggaaat cgtttctatg aaaaagaaat ggaatgtgca  1740
   ggtacatatg atggttccat cgatgcctgt aaaggggact ctggaggccc cttagtctgt  1800
   atggatgcca acaatgtgac ttatgtctgg ggtgttgtga gttgggggga aaactgtgga  1860
   aaaccagagt tcccaggtgt ttacaccaaa gtggccaatt attttgactg gattagctac  1920
   catgtaggaa ggccttttat ttctcagtac aatgtataaa attgtgatct ctctcttcat  1980
   tctattcttt ttctctcaag agttccattt aatggaaata aaacggtata attaataatt  2040
   ctctaggggg gaaaaatgaa gcaaatctca ctggatattt ttaaaggtct ccacagagtt  2100
   tatgccatat tggaattttg ttgtataatt ctcaaataaa tattttggtg aagcatacaa  2160
<210> 20
   <211> 1678
   <212> DNA
   <213> Homo sapiens
<400> 20
   gagcaaacag agcagcagaa aaggcagttc ctcttctcca gtgccctcct tccctgtctc    60
   tgcctctccc tcccttcctc aggcatcaga gcggagactt cagggagacc agagcccagc   120
   ttgccaggca ctgagctaga agccctgcca tggcacccct gagacccctt ctcatactgg   180
   ccctgctggc atgggttgct ctggctgacc aagagtcatg caagggccgc tgcactgagg   240
   gcttcaacgt ggacaagaag tgccagtgtg acgagctctg ctcttactac cagagctgct    300
   gcacagacta tacggctgag tgcaagcccc aagtgactcg cggggatgtg ttcactatgc    360
   cggaggatga gtacacggtc tatgacgatg gcgaggagaa aaacaatgcc actgtccatg    420
   aacaggtggg gggcccctcc ctgacctctg acctccaggc ccagtccaaa gggaatcctg    480
   agcagacacc tgttctgaaa cctgaggaag aggcccctgc gcctgaggtg ggcgcctcta    540
   agcctgaggg gatagactca aggcctgaga cccttcatcc agggagacct cagcccccag    600
   cagaggagga gctgtgcagt gggaagccct tcgacgcctt caccgacctc aagaacggtt    660
   ccctctttgc cttccgaggg cagtactgct atgaactgga cgaaaaggca gtgaggcctg    720
   ggtaccccaa gctcatccga gatgtctggg gcatcgaggg ccccatcgat gccgccttca    780
   cccgcatcaa ctgtcagggg aagacctacc tcttcaaggg tagtcagtac tggcgctttg    840
   aggatggtgt cctggaccct gattaccccc gaaatatctc tgacggcttc gatggcatcc    900
   cggacaacgt ggatgcagcc ttggccctcc ctgcccatag ctacagtggc cgggagcggg    960
   tctacttctt caaggggaaa cagtactggg agtaccagtt ccagcaccag cccagtcagg   1020
   aggagtgtga aggcagctcc ctgtcggctg tgtttgaaca ctttgccatg atgcagcggg   1080
   acagctggga ggacatcttc gagcttctct tctggggcag aacctctgct ggtaccagac   1140
   agccccagtt cattagccgg gactggcacg gtgtgccagg gcaagtggac gcagccatgg   1200
   ctggccgcat ctacatctca ggcatggcac cccgcccctc cttggccaag aaacaaaggt   1260
   ttaggcatcg caaccgcaaa ggctaccgtt cacaacgagg ccacagccgt ggccgcaacc   1320
   agaactcccg ccggccatcc cgcgccacgt ggctgtcctt gttctccagt gaggagagca   1380
   acttgggagc caacaactat gatgactaca ggatggactg gcttgtgcct gccacctgtg   1440
   aacccatcca gagtgtcttc ttcttctctg gagacaagta ctaccgagtc aatcttcgca   1500
   cacggcgagt ggacactgtg gaccctccct acccacgctc catcgctcag tactggctgg   1560
   gctgcccagc tcctggccat ctgtaggagt cagagcccac atggccgggc cctctgtagc   1620
   tccctcctcc catctccttc ccccagccca ataaaggtcc cttagccccg agtttaaa     1678
<210> 21
   <211> 1681
   <212> DNA
   <213> Homo sapiens
<400> 21
   gccgtccaag ggtccattgg ttgccataga gatcgtcgag cgctgggcct gtgatcgctg 60
   aggggcgagc agttgcgacc ctgggctcct ggggacctga gcgttatgtc tttccgcgac 120
   ctccgcaatt tcacagagat gatgagagcc ctgggatacc ctcgacatat ttctatggaa 180
   aatttccgta cacccaattt tggacttgta tctgaagtgc ttctctggct tgtgaaaaga 240
   tatgagcccc agactgacat cccgcctgac gtggatactg aacaggaccg agttttcttc 300
   attaaggcaa ttgcccagtt catggccacc aaggcacata taaaactcaa cactaagaag 360
   ctttatcaag cagatgggta tgcggtaaaa gagctgctga agatcacatc tgtcctttat 420
   aatgctatga agaccaaggg gatggagggc tctgaaatag tagaggaaga tgtcaacaag 480
   ttcaagtttg atcttggctc aaagattgca gatttgaagg cagccaggca gcttgcgtct 540
   gaaatcacct ccaaaggagc atctctgtat gacttgctcg gcatggaagt agagttgagg 600
   gaaatgagaa cagaagccat tgccagacct ctggaaataa acgagactga aaaagtgatg 660
   agaattgcaa taaaagagat tttgacacag gttcagaaga ctaaagacct gctcaataat 720
   gtggcctctg atgaagctaa tttagaagcc aaaatcgaaa agagaaaatt agaactggaa 780
   agaaatcgga agcgactaga gactctgcag agtgtcaggc catgttttat ggatgagtat 840
   gagaagactg aggaagaatt acaaaagcag tatgacactt atctggagaa atttcaaaat 900
   ctgacttatc tggaacaaca gcttgaagac catcatagga tggagcaaga aaggtttgag 960
   gaagctaaaa acactctctg cctgatacag aacaagctca aggaggaaga gaagcgcctg 1020
   ctcaagagtg gaagtaacga tgactcggac atagacatcc aggaggacga tgaatccgac 1080
   agtgagttgg aagaaaggcg gctgcccaag ccacagacag ccatggagat gctcatgcaa 1140
   ggaagacctg gcaaacgcat tgtgggcacg atgcaaggtg gagactccga tgacaatgag 1200
   gactcggagg agagtgaaat tgacatggaa gatgatgatg acgaggatga cgatttggaa 1260
   gacgagagca tttctctctc accaaccaag cccaatcgaa gggtccggaa atctgaaccc 1320
   ctggatgaga gtgacaatga cttctgaccc ttttgccaag ggaccctggc agattaaaac 1380
   cctcagactt gtaggtaaat gggaacttag aaggttagga aggtaacccc tgttttgttt 1440
   actaagctgg ctggactcat gatcactgaa gcaatactta tttctgcttt agcctcctat 1500
   gtttgcattc catgaagctt aaataagaat tgaagcaaat ccctaagatt tatttttttc 1560
   caccttattt atcttctaaa acttgaggaa tgcatgtgtt cttagtgatt cacatccacg 1620
   ggacaaaaac tcaagaagaa ataagagctg acgccacaca aaaaaaaaaa aaaaaaaaaa 1680
   a 1681
<210> 22
   <211> 2859
   <212> DNA
   <213> Homo sapiens
<400> 22
   ctttccgcgg cattctttgg gcgtgagtca tgcaggtttg cagccagccc caaagggggt     60
   gtgtgcgcga gcagagcgct ataaatacgg cgcctcccag tgcccacaac gcggcgtcgc    120
   caggaggagc gcgcgggcac agggtgccgc tgaccgaggc gtgcaaagac tccagaattg    180
   gaggcatgat gaagactctg ctgctgtttg tggggctgct gctgacctgg gagagtgggc    240
   aggtcctggg ggaccagacg gtctcagaca atgagctcca ggaaatgtcc aatcagggaa    300
   gtaagtacgt caataaggaa attcaaaatg ctgtcaacgg ggtgaaacag ataaagactc    360
   tcatagaaaa aacaaacgaa gagcgcaaga cactgctcag caacctagaa gaagccaaga    420
   agaagaaaga ggatgcccta aatgagacca gggaatcaga gacaaagctg aaggagctcc    480
   caggagtgtg caatgagacc atgatggccc tctgggaaga gtgtaagccc tgcctgaaac    540
   agacctgcat gaagttctac gcacgcgtct gcagaagtgg ctcaggcctg gttggccgcc 600
   agcttgagga gttcctgaac cagagctcgc ccttctactt ctggatgaat ggtgaccgca 660
   tcgactccct gctggagaac gaccggcagc agacgcacat gctggatgtc atgcaggacc 720
   acttcagccg cgcgtccagc atcatagacg agctcttcca ggacaggttc ttcacccggg 780
   agccccagga tacctaccac tacctgccct tcagcctgcc ccaccggagg cctcacttct 840
   tctttcccaa gtcccgcatc gtccgcagct tgatgccctt ctctccgtac gagcccctga 900
   acttccacgc catgttccag cccttccttg agatgataca cgaggctcag caggccatgg 960
   acatccactt ccatagcccg gccttccagc acccgccaac agaattcata cgagaaggcg 1020
   acgatgaccg gactgtgtgc cgggagatcc gccacaactc cacgggctgc ctgcggatga 1080
   aggaccagtg tgacaagtgc cgggagatct tgtctgtgga ctgttccacc aacaacccct 1140
   cccaggctaa gctgcggcgg gagctcgacg aatccctcca ggtcgctgag aggttgacca 1200
   ggaaatacaa cgagctgcta aagtcctacc agtggaagat gctcaacacc tcctccttgc 1260
   tggagcagct gaacgagcag tttaactggg tgtcccggct ggcaaacctc acgcaaggcg 1320
   aagaccagta ctatctgcgg gtcaccacgg tggcttccca cacttctgac tcggacgttc 1380
   cttccggtgt cactgaggtg gtcgtgaagc tctttgactc tgatcccatc actgtgacgg 1440
   tccctgtaga agtctccagg aagaacccta aatttatgga gaccgtggcg gagaaagcgc 1500
   tgcaggaata ccgcaaaaag caccgggagg agtgagatgt ggatgttgct tttgcaccta 1560
   cgggggcatc tgagtccagc tccccccaag atgagctgca gccccccaga gagagctctg 1620
   cacgtcacca agtaaccagg ccccagcctc caggccccca actccgccca gcctctcccc 1680
   gctctggatc ctgcactcta acactcgact ctgctgctca tgggaagaac agaattgctc 1740
   ctgcatgcaa ctaattcaat aaaactgtct tgtgagctga tcgcttggag ggtcctcttt 1800
   ttatgttgag ttgctgcttc ccggcatgcc ttcattttgc tatggggggc aggcaggggg 1860
   gatggaaaat aagtagaaac aaaaaagcag tggctaagat ggtataggga ctgtcatacc 1920
   agtgaagaat aaaagggtga agaataaaag ggatatgatg acaaggttga tccacttcaa 1980
   gaattgcttg ctttcaggaa gagagatgtg tttcaacaag ccaactaaaa tatattgctg 2040
   caaatggaag cttttctgtt ctattataaa actgtcgatg tattctgacc aaggtgcgac 2100
   aatctcctaa aggaatacac tgaaagttaa ggagaagaat cagtaagtgt aaggtgtact 2160
   tggtattata atgcataatt gatgttttcg ttatgaaaac atttggtgcc cagaagtcca 2220
   aattatcagt tttatttgta agagctattg cttttgcagc ggttttattt gtaaaagctg 2280
   ttgatttcga gttgtaagag ctcagcatcc caggggcatc ttcttgactg tggcatttcc 2340
   tgtccaccgc cggtttatat gatcttcata cctttccctg gaccacaggc gtttctcggc 2400
   ttttagtctg aaccatagct gggctgcagt accctacgct gccagcaggt ggccatgact 2460
   acccgtggta ccaatctcag tcttaaagct caggcttttc gttcattaac attctctgat 2520
   agaattctgg tcatcagatg tactgcaatg gaacaaaact catctggctg catcccaggt 2580
   gtgtagcaaa gtccacatgt aaatttatag cttagaatat tcttaagtca ctgtcccttg 2640
   tctctctttg aagttataaa caacaaactt aaagcttagc ttatgtccaa ggtaagtatt 2700
   ttagcatggc tgtcaaggaa attcagagta aagtcagtgt gattcactta atgatataca 2760
   ttaattagaa ttatggggtc agaggtattt gcttaagtga tcataattgt aaagtatatg 2820
   tcacattgtc acattaatgt caaaaaaaaa aaaaaaaaa 2859
<210> 23
   <211> 2979
   <212> DNA
   <213> Homo sapiens
<400> 23
   gggcagcctg ctgtcggctt agaggggatg ggcagtgtgg agggcctggc agagcaagag     60
   gactcatcct tccaaaggga ctttctctgg gaagcctgct cctcgggcca ctgcgaaccc    120
   tctctactct ccgaagggaa ttgtccttcc tggcttccac tacttccacc cctgaatgca    180
   caggcagccc ggcccaagtc tcccactagg gatgcagatg gattcggtgt gaagggctgg    240
   ctgctgttgc ctccggctct tgaaagtcaa gttcagaggc gtgcaaagac tccagaattg    300
   gaggcatgat gaagactctg ctgctgtttg tggggctgct gctgacctgg gagagtgggc    360
   aggtcctggg ggaccagacg gtctcagaca atgagctcca ggaaatgtcc aatcagggaa    420
   gtaagtacgt caataaggaa attcaaaatg ctgtcaacgg ggtgaaacag ataaagactc    480
   tcatagaaaa aacaaacgaa gagcgcaaga cactgctcag caacctagaa gaagccaaga    540
   agaagaaaga ggatgcccta aatgagacca gggaatcaga gacaaagctg aaggagctcc    600
   caggagtgtg caatgagacc atgatggccc tctgggaaga gtgtaagccc tgcctgaaac    660
   agacctgcat gaagttctac gcacgcgtct gcagaagtgg ctcaggcctg gttggccgcc    720
   agcttgagga gttcctgaac cagagctcgc ccttctactt ctggatgaat ggtgaccgca    780
   tcgactccct gctggagaac gaccggcagc agacgcacat gctggatgtc atgcaggacc    840
   acttcagccg cgcgtccagc atcatagacg agctcttcca ggacaggttc ttcacccggg    900
   agccccagga tacctaccac tacctgccct tcagcctgcc ccaccggagg cctcacttct    960
   tctttcccaa gtcccgcatc gtccgcagct tgatgccctt ctctccgtac gagcccctga   1020
   acttccacgc catgttccag cccttccttg agatgataca cgaggctcag caggccatgg   1080
   acatccactt ccatagcccg gccttccagc acccgccaac agaattcata cgagaaggcg   1140
   acgatgaccg gactgtgtgc cgggagatcc gccacaactc cacgggctgc ctgcggatga   1200
   aggaccagtg tgacaagtgc cgggagatct tgtctgtgga ctgttccacc aacaacccct   1260
   cccaggctaa gctgcggcgg gagctcgacg aatccctcca ggtcgctgag aggttgacca   1320
   ggaaatacaa cgagctgcta aagtcctacc agtggaagat gctcaacacc tcctccttgc   1380
   tggagcagct gaacgagcag tttaactggg tgtcccggct ggcaaacctc acgcaaggcg   1440
   aagaccagta ctatctgcgg gtcaccacgg tggcttccca cacttctgac tcggacgttc   1500
   cttccggtgt cactgaggtg gtcgtgaagc tctttgactc tgatcccatc actgtgacgg   1560
   tccctgtaga agtctccagg aagaacccta aatttatgga gaccgtggcg gagaaagcgc   1620
   tgcaggaata ccgcaaaaag caccgggagg agtgagatgt ggatgttgct tttgcaccta    1680
   cgggggcatc tgagtccagc tccccccaag atgagctgca gccccccaga gagagctctg    1740
   cacgtcacca agtaaccagg ccccagcctc caggccccca actccgccca gcctctcccc    1800
   gctctggatc ctgcactcta acactcgact ctgctgctca tgggaagaac agaattgctc   1860
   ctgcatgcaa ctaattcaat aaaactgtct tgtgagctga tcgcttggag ggtcctcttt   1920
   ttatgttgag ttgctgcttc ccggcatgcc ttcattttgc tatggggggc aggcaggggg   1980
   gatggaaaat aagtagaaac aaaaaagcag tggctaagat ggtataggga ctgtcatacc   2040
   agtgaagaat aaaagggtga agaataaaag ggatatgatg acaaggttga tccacttcaa   2100
   gaattgcttg ctttcaggaa gagagatgtg tttcaacaag ccaactaaaa tatattgctg   2160
   caaatggaag cttttctgtt ctattataaa actgtcgatg tattctgacc aaggtgcgac   2220
   aatctcctaa aggaatacac tgaaagttaa ggagaagaat cagtaagtgt aaggtgtact   2280
   tggtattata atgcataatt gatgttttcg ttatgaaaac atttggtgcc cagaagtcca   2340
   aattatcagt tttatttgta agagctattg cttttgcagc ggttttattt gtaaaagctg   2400
   ttgatttcga gttgtaagag ctcagcatcc caggggcatc ttcttgactg tggcatttcc   2460
   tgtccaccgc cggtttatat gatcttcata cctttccctg gaccacaggc gtttctcggc   2520
   ttttagtctg aaccatagct gggctgcagt accctacgct gccagcaggt ggccatgact   2580
   acccgtggta ccaatctcag tcttaaagct caggcttttc gttcattaac attctctgat   2640
   agaattctgg tcatcagatg tactgcaatg gaacaaaact catctggctg catcccaggt   2700
   gtgtagcaaa gtccacatgt aaatttatag cttagaatat tcttaagtca ctgtcccttg   2760
   tctctctttg aagttataaa caacaaactt aaagcttagc ttatgtccaa ggtaagtatt   2820
   ttagcatggc tgtcaaggaa attcagagta aagtcagtgt gattcactta atgatataca   2880
   ttaattagaa ttatggggtc agaggtattt gcttaagtga tcataattgt aaagtatatg   2940
   tcacattgtc acattaatgt caaaaaaaaa aaaaaaaaa                          2979
<210> 24
   <211> 2256
   <212> DNA
   <213> Homo sapiens
<400> 24
   tgaaattcag ggactctttg gtggagcaat taccagtcaa cttcagggta ttatgataaa    60
   ctctgatctg gggaggaacc aggactacat agatcaaggc agttttcttc tttgagaaac   120
   tatcccagat atcatcatag agtcttctgc tcttcctcaa ctaccaaaga aaaacatcag   180
   cgaagcagca ggccatgcac cccccaaaaa ctccatctgg ggctcttcat agaaaaagga   240
   aaatggcagc ctggcccttc tccaggctgt ggaaagtctc tgatccaatt ctcttccaaa   300
   tgaccttgat cgctgctctg ttgcctgctg ttcttggcaa ttgtggtcct ccacccactt   360
   tatcatttgc tgccccgatg gatattacgt tgactgagac acgcttcaaa actggaacta   420
   ctctgaaata cacctgcctc cctggctacg tcagatccca ttcaactcag acgcttacct 480
   gtaattctga tggcgaatgg gtgtataaca ccttctgtat ctacaaacga tgcagacacc 540
   caggagagtt acgtaatggg caagtagaga ttaagacaga tttatctttt ggatcacaaa 600
   tagaattcag ctgttcagaa ggatttttct taattggctc aaccactagt cgttgtgaag 660
   tccaagatag aggagttggc tggagtcatc ctctcccaca atgtgaaatt gtcaagtgta 720
   agcctcctcc agacatcagg aatggaaggc acagcggtga agaaaatttc tacgcatacg 780
   gcttttctgt cacctacagc tgtgaccccc gcttctcact cttgggccat gcctccattt 840
   cttgcactgt ggagaatgaa acaataggtg tttggagacc aagccctcct acctgtgaaa 900
   aaatcacctg tcgcaagcca gatgtttcac atggggaaat ggtctctgga tttggaccca 960
   tctataatta caaagacact attgtgttta agtgccaaaa aggttttgtt ctcagaggca 1020
   gcagtgtaat tcattgtgat gctgatagca aatggaatcc ttctcctcct gcttgtgagc 1080
   ccaatagttg tattaattta ccagacattc cacatgcttc ctgggaaaca tatcctaggc 1140
   cgacaaaaga ggatgtgtat gttgttggga ctgtgttaag gtaccgctgt catcctggct 1200
   acaaacccac tacagatgag cctacgactg tgatttgtca gaaaaatttg agatggaccc 1260
   cataccaagg atgtgaggcg ttatgttgcc ctgaaccaaa gctaaataat ggtgaaatca 1320
   ctcaacacag gaaaagtcgt cctgccaatc actgtgttta tttctatgga gatgagattt 1380
   cattttcatg tcatgagacc agtaggtttt cagctatatg ccaaggagat ggcacgtgga 1440
   gtccccgaac accatcatgt ggagacattt gcaattttcc tcctaaaatt gcccatgggc 1500
   attataaaca atctagttca tacagctttt tcaaagaaga gattatatat gaatgtgata 1560
   aaggctacat tctggtcgga caggcgaaac tctcctgcag ttattcacac tggtcagctc 1620
   cagcccctca atgtaaagct ctgtgtcgga aaccagaatt agtgaatgga aggttgtctg 1680
   tggataagga tcagtatgtt gagcctgaaa atgtcaccat ccaatgtgat tctggctatg 1740
   gtgtggttgg tccccaaagt atcacttgct ctgggaacag aacctggtac ccagaggtgc 1800
   ccaagtgtga gtgggagacc cccgaaggct gtgaacaagt gctcacaggc aaaagactca 1860
   tgcagtgtct cccaaaccca gaggatgtga aaatggccct ggaggtatat aagctgtctc 1920
   tggaaattga acaactggaa ctacagagag acagcgcaag acaatccact ttggataaag 1980
   aactataatt tttctcaaaa gaaggaggaa aaggtgtctt gctggcttgc ctcttgcaat 2040
   tcaatacaga tcagtttagc aaatctactg tcaatttggc agtgatattc atcataataa 2100
   atatctagaa atgataattt gctaaagttt agtgctttga gattgtgaaa ttattaatca 2160
   tcctctgtgt ggctcatgtt tttgcttttc aacacacaaa gcacaaattt tttttcgatt 2220
   aaaaatgtat gtataaaaaa ctaaaaaaaa aaaaaa 2256
<210> 25
   <211> 848
   <212> DNA
   <213> Homo sapiens
<400> 25
   agcctgggga gaggactttg atcaccagat gtttttttgg tgtgcgtgct gtcttatggt 60
   tgcgtggcga gtttctgctt cagatgcaga gcactgtcca gagcttcctc cagtggacaa 120
   tagcatattt gtcgcaaagg aggtggaagg acagattctg gggacttacg tttgtatcaa 180
   gggctaccac ctggtaggaa agaagaccct tttttgcaat gcctctaagg agtgggataa 240
   caccactact gagtgccgct tgggccactg tcctgatcct gtgctggtga atggagagtt 300
   cagttcttca gggcctgtga atgtaagtga caaaatcacg tttatgtgca atgaccacta 360
   catcctcaag ggcagcaatc ggagccagtg tctagaggac cacacctggg cacctccctt 420
   tcccatctgc aaaagtaggg actgtgaccc tcctgggaat ccagttcatg gctattttga 480
   aggaaataac ttcaccttag gatccaccat tagttattac tgtgaagaca ggtactactt 540
   agtgggcgtg caggagcagc aatgcgttga tggggagtgg agcagtgcac ttccagtctg 600
   caagttgatc caggaagctc ccaaaccaga gtgtgagaag gcacttcttg cctttcagga 660
   gagtaagaac ctctgcgaag ccatggagaa ctttatgcaa caattaaagg aaagtggcat 720
   gacaatggag gagctaaaat attctctgga gctgaagaaa gctgagttga aggcaaaatt 780
   gttgtaacac tacagctgag cagatgtaat agaaataaac ctatgaataa attttcttct 840
   tggttctg 848
<210> 26
   <211> 2017
   <212> DNA
   <213> Homo sapiens
<400> 26
   ccgagcgtgc ccgcggcgct gcccctcctc ggggagctgc cccggctgct gctgctggtg     60
   ctgttgtgcc tgccggccgt gtggggtgac tgtggccttc ccccagatgt acctaatgcc    120
   cagccagctt tggaaggccg tacaagtttt cccgaggata ctgtaataac gtacaaatgt    180
   gaagaaagct ttgtgaaaat tcctggcgag aaggactcag tgacctgcct taagggcatg    240
   caatggtcag atattgaaga gttctgcaat cgtagctgcg aggtgccaac aaggctaaat    300
   tctgcatccc tcaaacagcc ttatatcact cagaattatt ttccagtcgg tactgttgtg    360
   gaatatgagt gccgtccagg ttacagaaga gaaccttctc tatcaccaaa actaacttgc    420
   cttcagaatt taaaatggtc cacagcagtc gaattttgta aaaagaaatc atgccctaat    480
   ccgggagaaa tacgaaatgg tcagattgat gtaccaggtg gcatattatt tggtgcaacc    540
   atctccttct catgtaacac agggtacaaa ttatttggct cgacttctag tttttgtctt    600
   atttcaggca gctctgtcca gtggagtgac ccgttgccag agtgcagaga aatttattgt    660
   ccagcaccac cacaaattga caatggaata attcaagggg aacgtgacca ttatggatat    720
   agacagtctg taacgtatgc atgtaataaa ggattcacca tgattggaga gcactctatt    780
   tattgtactg tgaataatga tgaaggagag tggagtggcc caccacctga atgcagagga    840
   aaatctctaa cttccaaggt cccaccaaca gttcagaaac ctaccacagt aaatgttcca    900
   actacagaag tctcaccaac ttctcagaaa accaccacaa aaaccaccac accaaatgct    960
   caagcaacac ggagtacacc tgtttccagg acaaccaagc attttcatga aacaacccca   1020
   aataaaggaa gtggaaccac ttcaggtact acccgtcttc tatctgggca cacgtgtttc   1080
   acgttgacag gtttgcttgg gacgctagta accatgggct tgctgactta gccaaagaag   1140
   agttaagaag aaaatacaca caagtataca gactgttcct agtttcttag acttatctgc   1200
   atattggata aaataaatgc aattgtgctc ttcatttagg atgctttcat tgtctttaag   1260
   atgtgttagg aatgtcaaca gagcaaggag aaaaaaggca gtcctggaat cacattctta   1320
   gcacacctgc gcctcttgaa aatagaacaa cttgcagaat tgagagtgat tcctttccta   1380
   aaagtgtaag aaagcataga gatttgttcg tattaagaat gggatcacga ggaaaagaga   1440
   aggaaagtga tttttttcca caagatctga aatgatattt ccacttataa aggaaataaa   1500
   aaatgaaaaa cattatttgg atatcaaaag caaataaaaa cccaattcag tctcttctaa   1560
   gcaaaattgc taaagagaga tgaccacatt ataaagtaat ctttggctaa ggcattttca   1620
   tctttccttc ggttggcaaa atattttaaa ggtaaaacat gctggtgaac cagggtgttg   1680
   atggtgataa gggaggaata tagaatgaaa gactgaatct tcctttgttg cacaaataga   1740
   gtttggaaaa agcctgtgaa aggtgtcttc tttgacttaa tgtctttaaa agtatccaga   1800
   gatactacaa tattaacata agaaaagatt atatattatt tctgaatcga gatgtccata   1860
   gtcaaatttg taaatcttat tcttttgtaa tatttattta tatttattta tgacagtgaa   1920
   cattctgatt ttacatgtaa aacaagaaaa gttgaagaag atatgtgaag aaaaatgtat   1980
   ttttcctaaa tagaaataaa tgatcccatt ttttggt                             2017
<210> 27
   <211> 2017
   <212> DNA
   <213> Homo sapiens
<400> 27
   ccgagcgtgc ccgcggcgct gcccctcctc ggggagctgc cccggctgct gctgctggtg      60
   ctgttgtgcc tgccggccgt gtggggtgac tgtggccttc ccccagatgt acctaatgcc     120
   cagccagctt tggaaggccg tacaagtttt cccgaggata ctgtaataac gtacaaatgt     180
   gaagaaagct ttgtgaaaat tcctggcgag aaggactcag tgacctgcct taagggcatg     240
   caatggtcag atattgaaga gttctgcaat cgtagctgcg aggtgccaac aaggctaaat     300
   tctgcatccc tcaaacagcc ttatatcact cagaattatt ttccagtcgg tactgttgtg     360
   gaatatgagt gccgtccagg ttacagaaga gaaccttctc tatcaccaaa actaacttgc     420
   cttcagaatt taaaatggtc cacagcagtc gaattttgta aaaagaaatc atgccctaat     480
   ccgggagaaa tacgaaatgg tcagattgat gtaccaggtg gcatattatt tggtgcaacc     540
   atctccttct catgtaacac agggtacaaa ttatttggct cgacttctag tttttgtctt     600
   atttcaggca gctctgtcca gtggagtgac ccgttgccag agtgcagaga aatttattgt     660
   ccagcaccac cacaaattga caatggaata attcaagggg aacgtgacca ttatggatat     720
   agacagtctg taacgtatgc atgtaataaa ggattcacca tgattggaga gcactctatt     780
   tattgtactg tgaataatga tgaaggagag tggagtggcc caccacctga atgcagagga      840
   aaatctctaa cttccaaggt cccaccaaca gttcagaaac ctaccacagt aaatgttcca      900
   actacagaag tctcaccaac ttctcagaaa accaccacaa aaaccaccac accaaatgct      960
   caagcaacac ggagtacacc tgtttccagg acaaccaagc attttcatga aacaacccca     1020
   aataaaggaa gtggaaccac ttcaggtact acccgtcttc tatctgggca cacgtgtttc     1080
   acgttgacag gtttgcttgg gacgctagta accatgggct tgctgactta gccaaagaag     1140
   agttaagaag aaaatacaca caagtataca gactgttcct agtttcttag acttatctgc     1200
   atattggata aaataaatgc aattgtgctc ttcatttagg atgctttcat tgtctttaag     1260
   atgtgttagg aatgtcaaca gagcaaggag aaaaaaggca gtcctggaat cacattctta     1320
   gcacacctgc gcctcttgaa aatagaacaa cttgcagaat tgagagtgat tcctttccta     1380
   aaagtgtaag aaagcataga gatttgttcg tattaagaat gggatcacga ggaaaagaga     1440
   aggaaagtga tttttttcca caagatctga aatgatattt ccacttataa aggaaataaa     1500
   aaatgaaaaa cattatttgg atatcaaaag caaataaaaa cccaattcag tctcttctaa     1560
   gcaaaattgc taaagagaga tgaccacatt ataaagtaat ctttggctaa ggcattttca     1620
   tctttccttc ggttggcaaa atattttaaa ggtaaaacat gctggtgaac cagggtgttg     1680
   atggtgataa gggaggaata tagaatgaaa gactgaatct tcctttgttg cacaaataga     1740
   gtttggaaaa agcctgtgaa aggtgtcttc tttgacttaa tgtctttaaa agtatccaga     1800
   gatactacaa tattaacata agaaaagatt atatattatt tctgaatcga gatgtccata     1860
   gtcaaatttg taaatcttat tcttttgtaa tatttattta tatttattta tgacagtgaa     1920
   cattctgatt ttacatgtaa aacaagaaaa gttgaagaag atatgtgaag aaaaatgtat     1980
   ttttcctaaa tagaaataaa tgatcccatt ttttggt                              2017
<210> 28
   <211> 21
   <212> DNA
   <213> Homo sapiens
<400> 28
   aatccgagcc gttctctaca a 21
<210> 29
   <211> 21
   <212> DNA
   <213> Homo sapiens
<400> 29
   aacaagctct gccgtgatga a 21
<210> 30
   <211> 21
   <212> DNA
   <213> Homo sapiens
<400> 30
   aatggacaaa gtcggcaagt a 21
<210> 31
   <211> 21
   <212> DNA
   <213> Homo sapiens
<400> 31
   aactacatga acctacagag a 21
<210> 32
   <211> 21
   <212> DNA
   <213> Homo sapiens
<400> 32
   aaaaagcggc cagtcagaag a 21
<210> 33
   <211> 21
   <212> DNA
   <213> Homo sapiens
<400> 33
   aatgattggt ggattacgga a 21
<210> 34
   <211> 21
   <212> DNA
   <213> Homo sapiens
<400> 34
   aagtcctcgt tgtccgttcc a 21
<210> 35
   <211> 21
   <212> DNA
   <213> Homo sapiens
<400> 35
   aaatgattgg tggattacgg a 21
<210> 36
   <211> 21
   <212> DNA
   <213> Homo sapiens
<400> 36
   aattaccggc agaaccaaga g 21
<210> 37
   <211> 21
   <212> DNA
   <213> Homo sapiens
<400> 37
   aaatggaatc tctacgaagc t 21
<210> 38
   <211> 21
   <212> DNA
   <213> Homo sapiens
<400> 38
   aatgaacaga gatactacgg t 21
<210> 39
   <211> 21
   <212> DNA
   <213> Homo sapiens
<400> 39
   aagccttggc tcaataccaa a 21
<210> 40
   <211> 21
   <212> DNA
   <213> Homo sapiens
<400> 40
   aagcgcattc cgattgagga t 21
<210> 41
   <211> 21
   <212> DNA
   <213> Homo sapiens
<400> 41
   aatggaatct ctacgaagct c 21
<210> 42
   <211> 21
   <212> RNA
   <213> Homo sapiens
<400> 42
   aauccgagcc guucucuaca a 21
<210> 43
   <211> 21
   <212> RNA
   <213> Homo sapiens
<400> 43
   aacaagcucu gccgugauga a 21
<210> 44
   <211> 21
   <212> RNA
   <213> Homo sapiens
<400> 44
   aauggacaaa gucggcaagu a 21
<210> 45
   <211> 21
   <212> RNA
   <213> Homo sapiens
<400> 45
   aacuacauga accuacagag a 21
<210> 46
   <211> 21
   <212> RNA
   <213> Homo sapiens
<400> 46
   aaaaagcggc cagucagaag a 21
<210> 47
   <211> 21
   <212> RNA
   <213> Homo sapiens
<400> 47
   aaugauuggu ggauuacgga a 21
<210> 48
   <211> 21
   <212> RNA
   <213> Homo sapiens
<400> 48
   aaguccucgu uguccguucc a 21
<210> 49
   <211> 21
   <212> RNA
   <213> Homo sapiens
<400> 49
   aaaugauugg uggauuacgg a 21
<210> 50
   <211> 21
   <212> RNA
   <213> Homo sapiens
<400> 50
   aauuaccggc agaaccaaga g 21
<210> 51
   <211> 21
   <212> RNA
   <213> Homo sapiens
<400> 51
   aaauggaauc ucuacgaagc u 21
<210> 52
   <211> 21
   <212> RNA
   <213> Homo sapiens
<400> 52
   aaugaacaga gauacuacgg u 21
<210> 53
   <211> 21
   <212> RNA
   <213> Homo sapiens
<400> 53
   aagccuuggc ucaauaccaa a 21
<210> 54
   <211> 21
   <212> RNA
   <213> Homo sapiens
<400> 54
   aagcgcauuc cgauugagga u 21
<210> 55
   <211> 21
   <212> RNA
   <213> Homo sapiens
<400> 55
   aauggaaucu cuacgaagcu c 21
<210> 56
   <211> 21
   <212> RNA
   <213> Homo sapiens
<400> 56
   aaccucaucg ccaucgacuc c 21
<210> 57
   <211> 21
   <212> RNA
   <213> Homo sapiens
<400> 57
   ggagucgaug gcgaugaggu u 21